# EUROPEAN PATENT APPLICATION

(11) **EP 4 741 390 A1**
(43) Date of publication of application: **13.05.2026**
(21) Application number: 24858777.6
(22) Date of filing: 02.09.2024
(51) Int. Cl.: C07D 417/14, C07D 417/12, A61K 31/425, A61K 31/433, A61P 35/00

(54) **BIARYL RING COMPOUND, INTERMEDIATE THEREOF, PREPARATION METHOD THEREFOR AND USE THEREOF**

(30) Priority: 01.09.2023 CN 202311124367; 27.10.2023 CN 202311410407; 28.12.2023 CN 202311838459
(71) Applicant: Hangzhou Synrx Therapeutics Biomedical Technology Co., Ltd., Hangzhou, Zhejiang 311121 (CN)
(72) Inventor: HE, Hu, Hangzhou, Zhejiang 311121 (CN); GE, Chongxun, Hangzhou, Zhejiang 311121 (CN); LIU, Song, Hangzhou, Zhejiang 311121 (CN); SHI, Song, Hangzhou, Zhejiang 311121 (CN)
(74) Representative: WSL Patentanwälte Partnerschaft mbB
(86) International application number: PCT/CN2024/116342
(87) International publication number: WO 2025/045248

(57) **Abstract**

Provided are a biaryl ring compound, an intermediate thereof, a preparation method therefor and a use thereof. Specifically provided are a compound represented by Formula I, a pharmaceutically acceptable salt thereof, or an isotopic compound thereof. The ATPase activity and the inhibitory effect on proteins of the provided biaryl ring compound are greatly improved compared with the prior art.

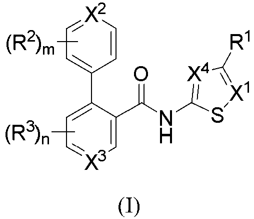

## Description

This application claims priorities to Chinese Patent Application No. 202311124367X filed on September 1, 2023, Chinese Patent Application No. 2023114104077 filed on October 27, 2023, and Chinese Patent Application No. 2023118384594 filed on December 28, 2023. The present application makes reference to the full texts of the Chinese Patent Applications above.

### FIELD OF THE INTVENTION

The present invention relates to a biaryl cyclic compound, intermediates thereof, a preparation method therefor and use thereof.

### BACKGROUND OF THE INVENTION

DNA damage is an important mechanism for many chemotherapy drugs to exert anti-tumor effects. DNA double-strand breaks (DSBs) are one of the most common types of damage in cells, which can be directly caused by ionizing radiation or induced by ultraviolet light, reactive oxygen species (ROS), or other mutagens, such as common chemotherapy drugs like cisplatin, 5-FU, and etoposide. Unrepaired DNA damage may lead to functional blockage such as cell transcription and replication, inducing apoptosis or necrosis, and clearance by immune cells. In tumor cells, there is an efficient damage repair pathway or alternative repair pathway. It is one of the important strategies for cancer treatment to target the key proteins of the DNA damage repair pathway, allowing for the constant accumulation of unrepaired DNA damage to eventually lead to cell death. The DNA DSBs are typically repaired in three ways, including non-homologous end joining (NHEJ), homologous recombination (HR), and alternative non-homologous end joining (Alt-NHEJ, also known as microhomology-mediated end joining (MMEJ) or theta-mediated end joining (TMEJ)). Here, the HR repair pathway only occurs in phases G2 and S to complete error-free repair in the presence of sister chromatids. In mammals, more than 90% of DSB damage is repaired by the NHEJ pathway. This repair is error-prone, resulting in the deletion mutation of < 30 bp, or the insertion mutation of < 5 bp, or the micro-homologous sequence of < 2 bp. Recent studies have shown that, in the case of HR and/or NHEJ deficiency, cells are highly dependent on the third way, i.e., MMEJ, to repair the broken DNAs. Inhibition of MMEJ leads to apoptosis. MMEJ, which is also an error-prone repair pathway, relies on DNA polymerase theta to link theta mediated end joining (TMEJ) sequences, thereby completing the repair of DNA double-strand breaks.

DNA polymerase theta (Pol theta or Pol θ), a key protein in the MMEJ repair pathway, is a unique multifunctional polymerase composed of an N-terminus helicase domain, a central domain, and a C-terminus polymerase domain. Basic studies have found that the polymerase domain is essential for DNA extension of DSB damage repair sites, and the helicase domain and the central domain play a key role in the recognition and binding of Pol θ to a substrate. Pol θ can eliminate the interaction between DNA and damage repair complexes (such as competition with RAD51 for binding to single-stranded DNA) and inhibit the HR repair pathway. In addition, the helicase domain of Pol θ is also involved in DNA replication blockage, and its function deficiency leads to increased replication pressure and apoptosis of tumor cells.

Pol θ is not expressed or expressed at a low level in normal tissue cells, but is highly expressed in a variety of tumors such as lung cancer, breast cancer, HR-deficient ovarian cancer, gastric cancer, and colon cancer, and it is associated with worse prognosis. Pol θ is overexpressed in more than 70% of breast cancers. These facts indicate that Pol θ may play an important role in these cancers and is a potential tumor-specific target.

Studies on knockdown or knockout of Pol θ in tumor cells have found that Pol θ deficiency can sensitize these cells to ionizing radiation, induce DSB production, enhance replication fork instability, and make tumor cells sensitive to genotoxic agents, which may enhance the effect of radiotherapy and chemotherapy, and Pol θ is a potential drug target. The studies have also found that Pol θ shows a synthetic lethality effect with HR deficiency, and its small molecule inhibitors can kill HR-deficient tumor cells in vitro and in vivo. In particular, in tumors resistant to PARP inhibitors (Olaparib, etc.) with HR deficiency reverse mutations, Pol θ inhibitors can sensitize cells to PARPi again, providing valuable therapeutic opportunities. In addition, given that Pol θ is a key protein in the MMEJ pathway, its functional impairment also leads to intensified genomic instability in cancer cells and increased somatic mutations, facilitating the production of tumor neoantigens. In addition, Pol θ has been reported to be involved in cGAS-STING-mediated immune activation. Hence, targeting Pol θ also has the potential to enhance immunotherapy.

In summary, Pol θ is a target with great potential for cancer treatment, and in the treatment of lung cancer, breast cancer, HR-deficient ovarian cancer, gastric cancer, colon cancer, prostate cancer, pancreatic cancer and other tumors, it is promising to design a Pol θ protein-targeting ATPase activity inhibitor to inhibit intracellular MMEJ, and administrate it alone or in combined trial with the additional chemotherapy, radiotherapy, antibody therapy, immunotherapy, etc., to achieve the purpose of killing tumor cells.

### SUMMARY OF THE INVENTION

The technical problem to be solved by the present invention is to overcome the defect of relatively unitary structure of polymerase theta inhibitor compounds in the prior art, and to this end, the present invention provides a class of biaryl cyclic compounds, intermediates thereof, a preparation method therefor and use thereof. The ATPase activity and protein inhibition effect of these compounds are greatly improved as compared to the prior art.

The present invention solves the above technical problems by means of the following technical solutions.

The present invention provides a compound of Formula I, a pharmaceutically acceptable salt thereof or an isotopic compound thereof, 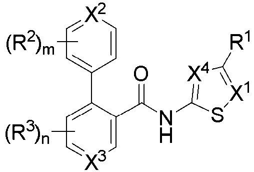 wherein,
m and n are independently 0, 1, 2, 3 or 4;
X¹ and X⁴ are independently N or CR^{a};
X² and X³ are independently N or CH;
each R^{a} is independently hydrogen, halogen, CN, OH, deuterium, cyano, -NHR^{f}, -C(O)NHR^{g}, C₁₋₆ alkyl unsubstituted or substituted by one or more R¹⁻¹ or C₁₋₆ alkoxy unsubstituted or substituted by one or more R¹⁻²;
each of R¹⁻¹ and R¹⁻² is independently halogen;
R¹ is hydrogen, deuterium, cyano, hydroxyl, halogen, amino, -NHC(O)R^{b}, -NHS(O)₂R^{c}, -S(O)₂NHR^{d}, 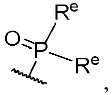 -NHR^{f}, -C(O)NHR^{g}, -OR^{h}, -SRⁱ, -C(O)Rⁱ, -S(O)₂R^{k}, -C(O)N(R^{m})₂, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸;
each of R^{b}, R^{c}, R^{d}, R^{e}, R^{f}, R^{g}, R^{h}, Rⁱ, R^{j}, R^{k} and R^{m} is independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently deuterium, halogen, C₁₋₆ alkyl, -S(O)₂C₁₋₆ alkyl or -P(O)(C₁₋₆ alkyl)₂;
each R² is independently deuterium, halogen, cyano, amino, hydroxyl, C₁₋₆ alkyl unsubstituted or substituted by one or more R²⁻¹, C₁₋₆ alkoxy unsubstituted or substituted by one or more R²⁻², C₂₋₆ alkenyl unsubstituted or substituted by one or more R²⁻³, C₂₋₆ alkynyl unsubstituted or substituted by one or more R²⁻⁴, C₃₋₁₀ cycloalkyl unsubstituted or substituted by one or more R²⁻⁵, 3-12 membered heterocycloalkyl unsubstituted or substituted by one or more R²⁻⁶, 4-12 membered heterocycloalkenyl unsubstituted or substituted by one or more R²⁻¹³, -COOR²⁻⁷, -C(O)R²⁻⁸, -C(O)N(R²⁻⁹)₂, 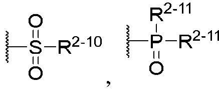 or N(R²⁻¹²)₂;
each of R²⁻¹, R²⁻², R²⁻³, R²⁻⁴, R²⁻⁵, R²⁻⁶ and R²⁻¹³ is independently deuterium, hydroxyl, cyano or halogen;
each of R²⁻², R²⁻⁸, R²⁻⁹ and R²⁻¹² is independently hydrogen or C₁₋₆ alkyl;
each of R²⁻¹⁰ and R²⁻¹¹ is independently hydroxyl or C₁₋₆ alkyl;
each R³ is independently C₁₋₆ alkyl unsubstituted or substituted by one or more R³⁻¹, C₂₋₆ alkenyl unsubstituted or substituted by one or more R³⁻², C₂₋₆ alkynyl unsubstituted or substituted by one or more R³⁻³, C₃₋₆ cycloalkyl unsubstituted or substituted by one or more R³⁻⁴, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, -0-5-12 membered heteroaryl, C₆₋₁₂ aryl unsubstituted or substituted by one or more R³⁻¹⁰, -N(R³⁻⁷)₂ or 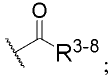
each of R³⁻¹, R³⁻² and R³⁻³ is independently 3-12 membered heterocycloalkyl unsubstituted or substituted by one or more R³⁻¹⁻¹, 5-12 membered heteroaryl unsubstituted or substituted by one or more R³⁻¹⁻² or 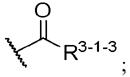
each of R³⁻⁴, R³⁻⁵ and R³⁻⁹ is independently oxo, halogen, cyano, hydroxyl, C₁₋₆ alkyl unsubstituted or substituted by one or more R³⁻⁵⁻¹, C₁₋₆ alkoxy unsubstituted or substituted by one or more R³⁻⁵⁻², C₃₋₆ cycloalkyl unsubstituted or substituted by one or more R³⁻⁵⁻⁵, 3-12 membered heterocycloalkyl unsubstituted or substituted by one or more R³⁻⁵⁻⁶, -N(R³⁻⁵⁻³)₂, 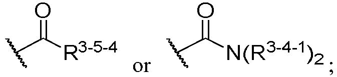
each of R³⁻⁶ and R³⁻¹⁰ is independently oxo, halogen, cyano, hydroxyl, C₁₋₆ alkyl unsubstituted or substituted by one or more R³⁻⁶⁻¹, C₁₋₆ alkoxy unsubstituted or substituted by one or more R³⁻⁵⁻², C₃₋₆ cycloalkyl unsubstituted or substituted by one or more R³⁻⁵⁻⁵, 3-12 membered heterocycloalkyl unsubstituted or substituted by one or more R³⁻⁶⁻², -N(R³⁻⁶⁻³)₂, 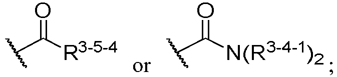
each R³⁻⁷ is independently hydrogen, C₁₋₆ alkyl unsubstituted or substituted by one or more R³⁻⁷⁻¹, 3-12 membered heterocycloalkyl unsubstituted or substituted by one or more R³⁻⁸⁻¹, 5-12 membered heteroaryl unsubstituted or substituted by one or more R³⁻⁷⁻² or 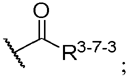
each R³⁻⁸ is independently C₁₋₆ alkyl unsubstituted or substituted by one or more R³⁻⁷⁻¹, 3-12 membered heterocycloalkyl unsubstituted or substituted by one or more R³⁻⁸⁻¹, 5-12 membered heteroaryl unsubstituted or substituted by one or more R³⁻⁷⁻² or 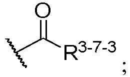
each of R³⁻¹⁻¹ and R³⁻¹⁻² is independently hydroxyl, cyano, halogen or C₁₋₆ alkyl;
each R³⁻¹⁻³ is independently 3-12 membered heterocycloalkyl unsubstituted or substituted by one or more R³⁻¹⁻³⁻¹;
each R³⁻¹⁻³⁻¹ is independently hydroxyl, cyano, halogen or C₁₋₆ alkyl;
each of R³⁻⁴⁻¹, R³⁻⁵⁻³ and R³⁻⁶⁻³ is independently hydrogen or C₁₋₆ alkyl;
each of R³⁻⁵⁻¹, R³⁻⁵⁻² and R³⁻⁷⁻¹ is independently deuterium, hydroxyl, cyano or halogen;
each of R³⁻⁵⁻⁴ and R³⁻⁷⁻³ is independently C₁₋₆ alkyl or C₃₋₆ cycloalkyl;
each of R³⁻⁵⁻⁵, R³⁻⁵⁻⁶ and R³⁻⁶⁻² is independently hydroxyl, cyano, halogen or C₁₋₆ alkyl;
each R³⁻⁶⁻¹ is independently hydroxyl, cyano, halogen or C₁₋₆ alkoxy;
each of R³⁻⁷⁻² and R³⁻⁸⁻¹ is independently hydroxyl, cyano, halogen or C₁₋₆ alkyl; and
each of 3-12 membered heterocycloalkyl and 5-12 membered heteroaryl has a heteroatom that is independently selected from one, two or three of N, O and S, and the number of heteroatom(s) is independently 1, 2 or 3;
a heteroatom in each of 3-12 membered heterocycloalkyl, 4-12 membered heterocycloalkenyl, 5-12 membered heteroaryl and -O-5-12 membered heteroaryl is independently selected from one, two or three of N, O and S, and the number of heteroatom(s) is independently 1, 2 or 3.

In a preferred embodiment, in the compound of Formula I, the pharmaceutically acceptable salt thereof or the isotopic compound thereof, some groups can be defined as follows, and other groups can be defined as in any one of the above embodiments (hereinafter referred to as "in a preferred embodiment").

In a preferred embodiment, R¹ is hydrogen, deuterium, cyano, hydroxyl, halogen, -NHC(O)R^{b}, -NHS(O)₂R^{c}, -S(O)₂NHR^{d}, 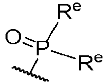 -NHR^{f}, -C(O)NHR^{g}, -OR^{h}, -SRⁱ, -C(O)R^{j}, -S(O)₂R^{k}, -C(O)N(R^{m})₂, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸; and
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, - S(O)₂C₁₋₆ alkyl or -P(O)(C₁₋₆ alkyl)₂.

In a preferred embodiment, in each R^{a}, said hydrogen is independently fluorine, chlorine, bromine or iodine.

In a preferred embodiment, in each R^{a}, C₁₋₆ alkyl in said C₁₋₆ alkyl unsubstituted or substituted by one or more R¹⁻¹ is independently methyl, ethyl, n-propyl, isopropyl, n-butyl, isobutyl, sec-butyl or tert-butyl.

In a preferred embodiment, in each R^{a}, C₁₋₆ alkoxy in said C₁₋₆ alkoxy unsubstituted or substituted by one or more R¹⁻² is independently methoxy, ethoxy, n-propoxy, isopropoxy, n-butoxy, isobutoxy, sec-butoxy, or tert-butoxy.

In a preferred embodiment, in each of R¹⁻¹ and R¹⁻², said hydrogen is independently fluorine, chlorine, bromine or iodine.

In a preferred embodiment, in R¹, said hydrogen is independently fluorine, chlorine, bromine or iodine, for example, fluorine.

In a preferred embodiment, in R¹, said halogen is independently fluorine or chlorine.

In a preferred embodiment, in R¹, C₁₋₆ alkyl in said C₁₋₆ alkyl unsubstituted or substituted by one or more R¹⁻³ is independently methyl, ethyl, n-propyl, isopropyl, n-butyl, isobutyl, sec-butyl or tert-butyl, for example, methyl, isopropyl or tert-butyl.

In a preferred embodiment, in R¹, C₁₋₆ alkyl in said C₁₋₆ alkyl unsubstituted or substituted by one or more R¹⁻³ is independently methyl, n-propyl, isopropyl or tert-butyl.

In a preferred embodiment, in R¹, C₁₋₆ alkoxy in said C₁₋₆ alkoxy unsubstituted or substituted by one or more R¹⁻⁴ is independently methoxy, ethoxy, n-propoxy, isopropoxy, n-butoxy, isobutoxy, sec-butoxy, or tert-butoxy, for example, methoxy.

In a preferred embodiment, in R¹, C₃₋₁₀ cycloalkyl in said C₃₋₁₀ cycloalkyl unsubstituted or substituted by one or more R¹⁻⁵ is independently cyclopropyl, cyclobutyl, cyclopentyl or cyclohexyl, for example, cyclopropyl. In a preferred embodiment, in R¹, C₃₋₁₀ cycloalkyl in said C₃₋₁₀ cycloalkyl unsubstituted or substituted by one or more R¹⁻⁵ is independently cyclopropyl or cyclobutyl.

In a preferred embodiment, in R¹, 3-12 membered heterocycloalkyl in said 3-12 membered heterocycloalkyl unsubstituted or substituted by one or more R¹⁻⁶ is independently 5-8 membered heterocycloalkyl.

In a preferred embodiment, in R¹, 3-12 membered heterocycloalkyl in said 3-12 membered heterocycloalkyl unsubstituted or substituted by one or more R¹⁻⁶ has a heteroatom that is independently N and/or O.

In a preferred embodiment, in R¹, 3-12 membered heterocycloalkyl in said 3-12 membered heterocycloalkyl unsubstituted or substituted by one or more R¹⁻⁶ has the number of heteroatom(s) that is independently 1 or 2, for example, 2.

In a preferred embodiment, in R¹, 3-12 membered heterocycloalkyl in said 3-12 membered heterocycloalkyl unsubstituted or substituted by one or more R¹⁻⁶ is independently a monocyclic ring, a bridged ring, a fused ring or a spiro ring;
in said spiro ring, fused ring or bridged ring, the number of rings can be 2;
said spiro ring can be 3-membered spiro 5-membered heterocyclyl, 3-membered spiro 6-membered heterocyclyl, 4-membered spiro 4-membered heterocyclyl, 4-membered spiro 5-membered heterocyclyl, 4-membered spiro 6-membered heterocyclyl, 4-membered spiro 7-membered heterocyclyl, 5-membered spiro 4-membered heterocyclyl, 5-membered spiro 5-membered heterocyclyl, 5-membered spiro 6-membered heterocyclyl, 5-membered spiro 7-membered heterocyclyl, 6-membered spiro 4-membered heterocyclyl, 6-membered spiro 5-membered heterocyclyl or 6-membered spiro 6-membered heterocyclyl, for example, 4-membered spiro 4-membered heterocyclyl;
said fused ring can be 3-membered fused 5-membered heterocyclyl, 4-membered fused 5-membered heterocyclyl, 4-membered fused 6-membered heterocyclyl, 5-membered fused 5-membered heterocyclyl, 5-membered fused 6-membered heterocyclyl or 6-membered fused 6-membered heterocyclyl, for example, 3-membered fused 5-membered heterocyclyl or 5-membered fused 5-membered heterocyclyl; preferably, said fused ring can be 4-membered fused 5-membered heterocyclyl, 4-membered fused 6-membered heterocyclyl, 5-membered fused 5-membered heterocyclyl, 5-membered fused 6-membered heterocyclyl or 6-membered fused 6-membered heterocyclyl, for example, 5-membered fused 5-membered heterocyclyl; and
said bridged ring can be 4-membered bridged 5-membered heterocyclyl, 4-membered bridged 6-membered heterocyclyl, 5-membered bridged 6-membered heterocyclyl or 6-membered bridged 6-membered heterocyclyl, for example, 4-membered bridged 6-membered heterocyclyl.

In a preferred embodiment, in R¹, 3-12 membered heterocycloalkyl in said 3-12 membered heterocycloalkyl unsubstituted or substituted by one or more R¹⁻⁶ is independently 5-8 membered heterocycloalkyl, said 5-8 membered heterocycloalkyl has a heteroatom that is N and/or O, and the number of heteroatom(s) is 1 or 2, for example,

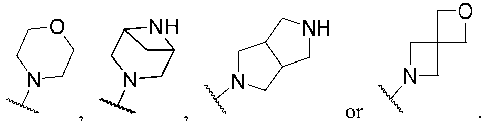

In a preferred embodiment, in R¹, 3-12 membered heterocycloalkyl in said 3-12 membered heterocycloalkyl unsubstituted or substituted by one or more R¹⁻⁶ is independently

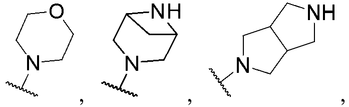

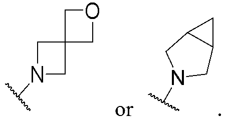

In a preferred embodiment, in R¹, C₆₋₁₂ aryl in said C₆₋₁₂ aryl unsubstituted or substituted by one or more R¹⁻⁷ is independently phenyl or naphthyl, for example, phenyl.

In a preferred embodiment, in R¹, 5-12 membered heteroaryl in said 5-12 membered heteroaryl unsubstituted or substituted by one or more R¹⁻⁸ is a monocyclic ring.

In a preferred embodiment, in R¹, 5-12 membered heteroaryl in said 5-12 membered heteroaryl unsubstituted or substituted by one or more R¹⁻⁸ is independently 5-6 membered heteroaryl.

In a preferred embodiment, in R¹, 5-12 membered heteroaryl in said 5-12 membered heteroaryl unsubstituted or substituted by one or more R¹⁻⁸ has a heteroatom that is N and/or O.

In a preferred embodiment, in R¹, 5-12 membered heteroaryl in said 5-12 membered heteroaryl unsubstituted or substituted by one or more R¹⁻⁸ has a heteroatom that is N.

In a preferred embodiment, in R¹, 5-12 membered heteroaryl in said 5-12 membered heteroaryl unsubstituted or substituted by one or more R¹⁻⁸ has the number of heteroatom(s) that is 1 or 2.

In a preferred embodiment, in R¹, 5-12 membered heteroaryl in said 5-12 membered heteroaryl unsubstituted or substituted by one or more R¹⁻⁸ is 5-6 membered heteroaryl, said 5-6 membered heteroaryl has a heteroatom that is N and/or O, and the number of heteroatom(s) is 1 or 2, for example,

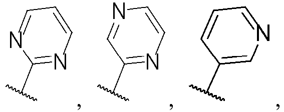

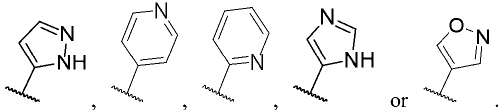

In a preferred embodiment, in R¹, 5-12 membered heteroaryl in said 5-12 membered heteroaryl unsubstituted or substituted by one or more R¹⁻⁸ is 5-6 membered heteroaryl, said 5-6 membered heteroaryl has a heteroatom that is N, and the number of heteroatom(s) is 1 or 2, for example,

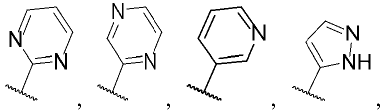

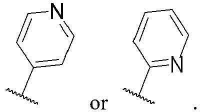

In a preferred embodiment, in R^{b}, R^{c}, R^{d}, each R^{e}, R^{f}, R^{g}, R^{h}, Rⁱ, R^{j}, R^{k} and R^{m}, C₃₋₁₀ cycloalkyl in said C₃₋₁₀ cycloalkyl unsubstituted or substituted by one or more R¹⁻⁹ is independently cyclopropyl, cyclobutyl, cyclopentyl or cyclohexyl, for example, cyclopropyl.

In a preferred embodiment, in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ is independently a monocyclic ring, a bridged ring or a fused ring.
in said fused ring or bridged ring, the number of rings can be 2;
said fused ring can be 4-membered fused 5-membered heterocyclyl, 4-membered fused 6-membered heterocyclyl, 5-membered fused 5-membered heterocyclyl, 5-membered fused 6-membered heterocyclyl or 6-membered fused 6-membered heterocyclyl, for example, 5-membered fused 5-membered heterocyclyl; and said bridged ring can be 4-membered bridged 5-membered heterocyclyl, 4-membered bridged 6-membered heterocyclyl, 5-membered bridged 6-membered heterocyclyl or 6-membered bridged 6-membered heterocyclyl, for example, 4-membered bridged 6-membered heterocyclyl.

In a preferred embodiment, in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ is independently 4-8 membered heterocycloalkyl.

In a preferred embodiment, in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ has a heteroatom that is independently N and/or O.

In a preferred embodiment, in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ has the number of heteroatom(s) that is independently 1 or 2.

In a preferred embodiment, in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ is independently 4-8 membered heterocycloalkyl, said 4-8 membered heterocycloalkyl has a heteroatom that is N and/or O, and the number of heteroatom(s) is 1 or 2, for example, 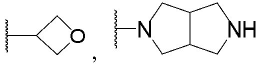 or

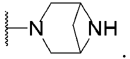

In a preferred embodiment, in R^{b}, R^{c}, R^{d}, each R^{e}, R^{f}, R^{g}, R^{h}, Rⁱ, R^{j}, R^{k} and R^{m}, C₆₋₁₂ aryl in said C₆₋₁₂ aryl unsubstituted or substituted by one or more R¹⁻¹¹ is independently phenyl or naphthyl, for example, phenyl.

In a preferred embodiment, in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² is a monocyclic ring.

In a preferred embodiment, in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² is independently 5-6 membered heteroaryl.

In a preferred embodiment, in R^{b}, R^{c}, R^{d}, each R^{e}, R^{f}, R^{g}, R^{h}, Rⁱ, R^{j} and R^{k}, 5-12 membered heteroaryl in said 5-12 membered heteroaryl unsubstituted or substituted by one or more R¹⁻¹² has a heteroatom that is N.

In a preferred embodiment, in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² has the number of heteroatom(s) that is independently 1 or 2.

In a preferred embodiment, in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² is independently 5-6 membered heteroaryl, said 5-6 membered heteroaryl has a heteroatom that is N, and the number of heteroatom(s) is 1 or 2, for example,

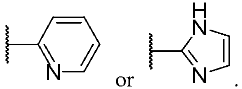

In a preferred embodiment, in R^{b}, R^{c}, R^{d}, each R^{e}, R^{f}, R^{g}, R^{h}, Rⁱ, R^{j}, R^{k} and R^{m}, C₁₋₆ alkyl in said C₁₋₆ alkyl unsubstituted or substituted by one or more R¹⁻¹³ is independently methyl, ethyl, n-propyl, isopropyl, n-butyl, isobutyl, sec-butyl or tert-butyl, for example, methyl.

In a preferred embodiment, in each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, said hydrogen is independently fluorine, chlorine, bromine or iodine, for example, fluorine or chlorine.

In a preferred embodiment, in each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, each of said C₁₋₆ alkyl, C₁₋₆ alkyl in said -S(O)₂C₁₋₆ alkyl and C₁₋₆ alkyl in said -P(O)(C₁₋₆ alkyl)₂ is independently methyl, ethyl, n-propyl, isopropyl, n-butyl, isobutyl, sec-butyl or tert-butyl, for example, methyl.

In a preferred embodiment, in each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, said - S(O)₂C₁₋₆ alkyl is

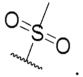

In a preferred embodiment, in each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, -P(O)(C₁₋₆ alkyl)₂ is

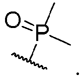

In a preferred embodiment, in R¹, "more" in each case refers to 2 or 3, for example, 2.

In a preferred embodiment, in each R², said hydrogen is independently fluorine, chlorine, bromine or iodine, fluorine or fluorine.

In a preferred embodiment, in each R², C₁₋₆ alkyl in said C₁₋₆ alkyl unsubstituted or substituted by one or more R²⁻¹ is independently methyl, ethyl, n-propyl, isopropyl, n-butyl, isobutyl, sec-butyl or tert-butyl, for example, methyl.

In a preferred embodiment, in each R², "more" in each case refers to 2 or 3, for example, 2.

In a preferred embodiment, in each R², C₁₋₆ alkoxy in said C₁₋₆ alkoxy unsubstituted or substituted by one or more R²⁻² is independently methoxy, ethoxy, n-propoxy, isopropoxy, n-butoxy, isobutoxy, sec-butoxy, or tert-butoxy, for example, methoxy.

In a preferred embodiment, in each of R²⁻¹ and R²⁻², said hydrogen is independently fluorine, chlorine, bromine or iodine, for example, fluorine.

In a preferred embodiment, in each R², said C₁₋₆ alkyl substituted by one or more R²⁻¹ is

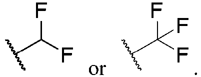

In a preferred embodiment, in each R³, C₁₋₆ alkyl in said C₁₋₆ alkyl unsubstituted or substituted by one or more R³⁻¹ is independently methyl, ethyl, n-propyl, isopropyl, n-butyl, isobutyl, sec-butyl or tert-butyl, for example, methyl.

In a preferred embodiment, in each R³, 4-12 membered heterocycloalkenyl in said 4-12 membered heterocycloalkenyl unsubstituted or substituted by one or more R³⁻⁹ is a monocyclic ring or a fused ring;
in said fused ring, the number of rings can be 2; and
said fused ring can be 4-membered fused 5 membered heterocycloalkenyl, 4-membered fused 6 membered heterocycloalkenyl, 5-membered fused 5 membered heterocycloalkenyl, 5-membered fused 6 membered heterocycloalkenyl or 6-membered fused 6 membered heterocycloalkenyl, for example, 5-membered fused 6 membered heterocycloalkenyl.

In a preferred embodiment, in each R³, 4-12 membered heterocycloalkenyl in said 4-12 membered heterocycloalkenyl unsubstituted or substituted by one or more R³⁻⁹ is independently 5-9 membered heterocycloalkenyl, for example, 5-6 membered heterocycloalkenyl.

In a preferred embodiment, in each R³, 4-12 membered heterocycloalkenyl in said 4-12 membered heterocycloalkenyl unsubstituted or substituted by one or more R³⁻⁹ has a heteroatom that is N.

In a preferred embodiment, in each R³, 4-12 membered heterocycloalkenyl in said 4-12 membered heterocycloalkenyl unsubstituted or substituted by one or more R³⁻⁹ has the number of heteroatom(s) that is 1 or 2, for example, 1.

In a preferred embodiment, in each R³, 4-12 membered heterocycloalkenyl in said 4-12 membered heterocycloalkenyl unsubstituted or substituted by one or more R³⁻⁹ has the number of double bond(s) that is 1 or 2, for example, 2.

In a preferred embodiment, in each R³, 4-12 membered heterocycloalkenylin said 4-12 membered heterocycloalkenyl unsubstituted or substituted by one or more R³⁻⁹ is independently 5-9 membered heterocycloalkenyl, said 5-9 membered heterocycloalkenyl has a heteroatom that is N, the number of heteroatom(s) is 1 or 2, and in said 5-9 membered heterocycloalkenyl, the number of double bond(s) is 1 or 2, for example,

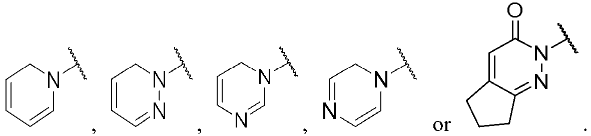

In a preferred embodiment, in each R³, C₂₋₆ alkynyl in said C₂₋₆ alkynyl unsubstituted or substituted by one or more R³⁻³ is independently ethynyl, propinyl or propargyl, for example, ethynyl.

In a preferred embodiment, in each R³, 3-12 membered heterocycloalkyl in said 3-12 membered heterocycloalkyl unsubstituted or substituted by one or more R³⁻⁵ is independently a bridged ring or a spiro ring;
in said spiro ring or bridged ring, the number of rings can be 2, and said spiro ring can be 3-membered spiro 5-membered heterocyclyl, 3-membered spiro 6-membered heterocyclyl, 4-membered spiro 4-membered heterocyclyl, 4-membered spiro 5-membered heterocyclyl, 4-membered spiro 6-membered heterocyclyl, 4-membered spiro 7-membered heterocyclyl, 5-membered spiro 4-membered heterocyclyl, 5-membered spiro 5-membered heterocyclyl, 5-membered spiro 6-membered heterocyclyl, 5-membered spiro 7-membered heterocyclyl, 6-membered spiro 4-membered heterocyclyl, 6-membered spiro 5-membered heterocyclyl or 6-membered spiro 6-membered heterocyclyl, for example, 3-membered spiro 6 membered heterocycloalkyl; and said bridged ring can be 4-membered bridged 5 membered heterocycloalkyl, 4-membered bridged 6 membered heterocycloalkyl, 5-membered bridged 6 membered heterocycloalkyl or 6-membered bridged 6 membered heterocycloalkyl, for example, 4-membered bridged 6 membered heterocycloalkyl.

In a preferred embodiment, in each R³, 3-12 membered heterocycloalkyl in said 3-12-membered heterocycloalkane unsubstituted or substituted by one or more R³⁻⁵ is independently 6-8 membered heterocycloalkyl.

In a preferred embodiment, in each R³, 3-12 membered heterocycloalkyl in said 3-12 membered heterocycloalkyl unsubstituted or substituted by one or more R³⁻⁵ has a heteroatom that is N.

In a preferred embodiment, in each R³, 3-12 membered heterocycloalkyl in said 3-12 membered heterocycloalkyl unsubstituted or substituted by one or more R³⁻⁵ has the number of heteroatom(s) that is independently 1 or 2.

In a preferred embodiment, in each R³, 3-12 membered heterocycloalkyl in said 3-12 membered heterocycloalkyl unsubstituted or substituted by one or more R³⁻⁵ is 6-8 membered heterocycloalkyl, said 6-8 membered heterocycloalkyl has a heteroatom that is N, and the number of heteroatom(s) is 1 or 2, for example,

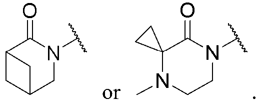

In a preferred embodiment, in each R³, C₆₋₁₂ aryl in said C₆₋₁₂ aryl unsubstituted or substituted by one or more R³⁻¹⁰ is phenyl or naphthyl, for example, phenyl.

In a preferred embodiment, in each R³, 5-12 membered heteroaryl in said 5-12 membered heteroaryl unsubstituted or substituted by one or more R³⁻⁶ is a monocyclic ring or a fused ring,
in said fused ring, the number of rings can be 2.

In a preferred embodiment, in each R³, 5-12 membered heteroaryl in said 5-12 membered heteroaryl unsubstituted or substituted by one or more R³⁻⁶is 5-9 membered heteroaryl.

In a preferred embodiment, in each R³, 5-12 membered heteroaryl in said 5-12 membered heteroaryl unsubstituted or substituted by one or more R³⁻⁶ has a heteroatom that is N and/or O.

In a preferred embodiment, in each R³, 5-12 membered heteroaryl in said 5-12 membered heteroaryl unsubstituted or substituted by one or more R³⁻⁶ is 5-9 membered heteroaryl, said 5-9 membered heteroaryl has a heteroatom that is N and/or O, and the number of heteroatom(s) is 1, 2 or 3, for example, 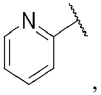 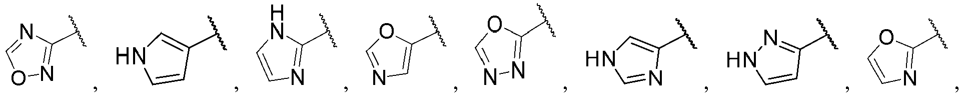 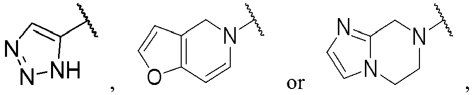 for another example,

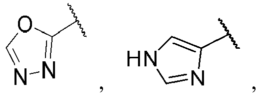

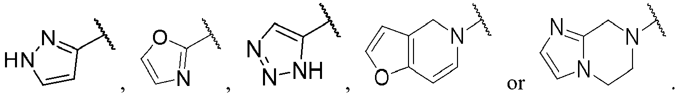

In a preferred embodiment, in each R³, "more" in each case refers to 2 or 3, for example, 2.

In a preferred embodiment, in each of R³⁻¹ and R³⁻³, 3-12 membered heterocycloalkyl in said 3-12 membered heterocycloalkyl unsubstituted or substituted by one or more R³⁻¹⁻¹ is a monocyclic ring.

In a preferred embodiment, in each of R³⁻¹ and R³⁻³, 3-12 membered heterocycloalkyl in said 3-12 membered heterocycloalkyl unsubstituted or substituted by one or more R³⁻¹⁻¹ is 5-6 membered heterocycloalkyl.

In a preferred embodiment, in each of R³⁻¹ and R³⁻³, 3-12 membered heterocycloalkyl in said 3-12 membered heterocycloalkyl unsubstituted or substituted by one or more R³⁻¹⁻¹ has a heteroatom that is N and/or O, for example, O.

In a preferred embodiment, in each of R³⁻¹ and R³⁻³, 3-12 membered heterocycloalkyl in said 3-12 membered heterocycloalkyl unsubstituted or substituted by one or more R³⁻¹⁻¹ has the number of heteroatom(s) that is 1 or 2, for example, 1.

In a preferred embodiment, in each of R³⁻¹ and R³⁻³, 3-12 membered heterocycloalkyl in said 3-12 membered heterocycloalkyl unsubstituted or substituted by one or more R³⁻¹⁻¹ is 5-6 membered heterocycloalkyl, said 5-6 membered heterocycloalkyl has a heteroatom that is N and/or O, and the number of heteroatom(s) is 1 or 2, for example,

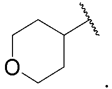

In a preferred embodiment, in each of R³⁻⁵ and R³⁻⁹, said hydrogen is independently fluorine, chlorine, bromine or iodine, for example, fluorine or fluorine.

In a preferred embodiment, in each of R³⁻⁵ and R³⁻⁹, C₁₋₆ alkyl in said C₁₋₆ alkyl unsubstituted or substituted by one or more R³⁻⁵⁻¹ is independently methyl, ethyl, n-propyl, isopropyl, n-butyl, sec-butyl, isobutyl or tert-butyl, for example, methyl.

In a preferred embodiment, in each of R³⁻⁵ and R³⁻⁹, C₁₋₆ alkyl in said C₁₋₆ alkoxy unsubstituted or substituted by one or more R³⁻⁵⁻¹ is independently methoxy, ethoxy, n-propoxy, isopropoxy, n-butoxy, sec-butoxy, isobutoxy or tert-butoxy, for example, methoxy.

In a preferred embodiment, in each of R³⁻⁶ and R³⁻¹⁰, C₁₋₆ alkyl in said C₁₋₆ alkyl unsubstituted or substituted by one or more R³⁻⁶⁻¹ is independently methyl, ethyl, n-propyl, isopropyl, n-butyl, sec-butyl, isobutyl or tert-butyl, for example, methyl.

In a preferred embodiment, in each R³⁻⁸, 3-12 membered heterocycloalkyl in said 3-12 membered heterocycloalkyl unsubstituted or substituted by one or more R³⁻⁸⁻¹ is a monocyclic ring.

In a preferred embodiment, in each R³⁻⁸, 3-12 membered heterocycloalkyl in said 3-12 membered heterocycloalkyl unsubstituted or substituted by one or more R³⁻⁸⁻¹ is 5-6 membered heterocycloalkyl.

In a preferred embodiment, in each R³⁻⁸, 3-12 membered heterocycloalkyl in said 3-12 membered heterocycloalkyl unsubstituted or substituted by one or more R³⁻⁸⁻¹ has a heteroatom that is N and/or O.

In a preferred embodiment, in each R³⁻⁸, 3-12 membered heterocycloalkyl in said 3-12 membered heterocycloalkyl unsubstituted or substituted by one or more R³⁻⁸⁻¹ has the number of heteroatom(s) that is 1 or 2, for example, 2.

In a preferred embodiment, in each R³⁻⁸, 3-12 membered heterocycloalkyl in said 3-12 membered heterocycloalkyl unsubstituted or substituted by one or more R³⁻⁸⁻¹ is 5-6 membered heterocycloalkyl, said 5-6 membered heterocycloalkyl has a heteroatom that is N and/or O, and the number of heteroatom(s) is 1 or 2, for example,

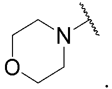

In a preferred embodiment, in each R³⁻¹⁻³, 3-12 membered heterocycloalkyl in said 3-12 membered heterocycloalkyl unsubstituted or substituted by one or more R³⁻¹⁻³⁻¹ is a monocyclic ring.

In a preferred embodiment, in each R³⁻¹⁻³, 3-12 membered heterocycloalkyl in said 3-12 membered heterocycloalkyl unsubstituted or substituted by one or more R³⁻¹⁻³⁻¹ is 5-6 membered heterocycloalkyl.

In a preferred embodiment, in each R³⁻¹⁻³, 3-12 membered heterocycloalkyl in said 3-12 membered heterocycloalkyl unsubstituted or substituted by one or more R³⁻¹⁻³⁻¹ has a heteroatom that is N and/or O.

In a preferred embodiment, in each R³⁻¹⁻³, 3-12 membered heterocycloalkyl in said 3-12 membered heterocycloalkyl unsubstituted or substituted by one or more R³⁻¹⁻³⁻¹ has the number of heteroatom(s) that is 1 or 2, for example, 2.

In a preferred embodiment, in each R³⁻¹⁻³, 3-12 membered heterocycloalkyl in said 3-12 membered heterocycloalkyl unsubstituted or substituted by one or more R³⁻¹⁻³⁻¹ is 5-6 membered heterocycloalkyl, said 5-6 membered heterocycloalkyl has a heteroatom that is N and/or O, and the number of heteroatom(s) is 1 or 2, for example,

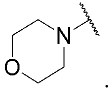

In a preferred embodiment, in each R³⁻⁵⁻¹, said hydrogen is independently fluorine, chlorine, bromine or iodine, for example, fluorine.

In a preferred embodiment, in each of R³⁻⁵ and R³⁻⁹, said C₁₋₆ alkyl substituted by one or more R³⁻⁵⁻¹ is

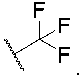

In a preferred embodiment, C₂₋₆ alkenyl described in any embodiment of the present invention is independently ethenyl, propenyl or allyl.

In a preferred embodiment, C₂₋₆ alkynyl described in any embodiment of the present invention is independently ethynyl, propinyl or propargyl.

In a preferred embodiment, C₃₋₁₀ cycloalkyl and C₃₋₆ cycloalkyl described in any embodiment of the present invention are independently cyclopropyl, cyclobutyl, cyclopentyl or cyclohexyl.

In a preferred embodiment, 3-12 membered heterocycloalkyl described in any embodiment of the present invention is a monocyclic ring, a fused ring, a bridged ring or a spiro ring;
in said spiro ring, fused ring or bridged ring, the number of rings can be 2;
said spiro ring can be 3-membered spiro 5-membered heterocyclyl, 3-membered spiro 6-membered heterocyclyl, 4-membered spiro 4-membered heterocyclyl, 4-membered spiro 5-membered heterocyclyl, 4-membered spiro 6-membered heterocyclyl, 4-membered spiro 7-membered heterocyclyl, 5-membered spiro 4-membered heterocyclyl, 5-membered spiro 5-membered heterocyclyl, 5-membered spiro 6-membered heterocyclyl, 5-membered spiro 7-membered heterocyclyl, 6-membered spiro 4-membered heterocyclyl, 6-membered spiro 5-membered heterocyclyl or 6-membered spiro 6-membered heterocyclyl;
said fused ring can be 4-membered fused 5-membered heterocyclyl, 4-membered fused 6-membered heterocyclyl, 5-membered fused 5-membered heterocyclyl, 5-membered fused 6-membered heterocyclyl or 6-membered fused 6-membered heterocyclyl;
said bridged ring can be 4-membered bridged 5-membered heterocyclyl, 4-membered bridged 6-membered heterocyclyl, 5-membered bridged 6-membered heterocyclyl or 6-membered bridged 6-membered heterocyclyl.

In a preferred embodiment, 3-12 membered heterocycloalkyl described in any embodiment of the present invention is 5-6 membered heterocycloalkyl.

In a preferred embodiment, 3-12 membered heterocycloalkyl described in any embodiment of the present invention has a heteroatom that is N and/or O.

In a preferred embodiment, 3-12 membered heterocycloalkyl described in any embodiment of the present invention has the number of heteroatom(s) that is 1 or 2, for example, 2.

In a preferred embodiment, 3-12 membered heterocycloalkyl described in any embodiment of the present invention is independently

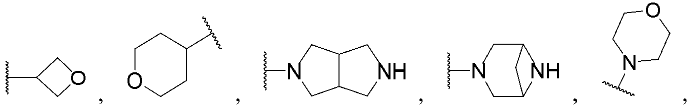

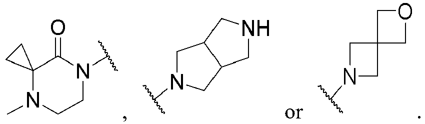

In a preferred embodiment, 4-12 membered heterocycloalkenyl described in any embodiment of the present invention is a monocyclic ring or a fused ring;
in said fused ring, the number of rings can be 2; and
said fused ring can be 4-membered fused 5 membered heterocycloalkenyl, 4-membered fused 6 membered heterocycloalkenyl, 5-membered fused 5 membered heterocycloalkenyl, 5-membered fused 6 membered heterocycloalkenyl or 6-membered fused 6 membered heterocycloalkenyl.

In a preferred embodiment, 4-12 membered heterocycloalkenyl described in any embodiment of the present invention is independently 5-9 membered heterocycloalkenyl.

In a preferred embodiment, 4-12 membered heterocycloalkenyl described in any embodiment of the present invention has a heteroatom that is N.

In a preferred embodiment, 4-12 membered heterocycloalkenyl described in any embodiment of the present invention has the number of heteroatom(s) that is 1 or 2, for example, 1.

In a preferred embodiment, 4-12 membered heterocycloalkenyl described in any embodiment of the present invention has the number of double bond(s) that is 1 or 2, for example, 2.

In a preferred embodiment, 4-12 membered heterocycloalkenyl described in any embodiment of the present invention is independently

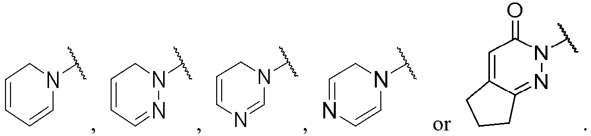

In a preferred embodiment, halogen described in any embodiment of the present invention is independently fluorine, chlorine, bromine or iodine.

In a preferred embodiment, C₁₋₆ alkyl described in any embodiment of the present invention is independently methyl, ethyl, n-propyl, isopropyl, n-butyl, isobutyl, sec-butyl or tert-butyl.

In a preferred embodiment, 5-12 membered heteroaryl described in any embodiment of the present invention is a monocyclic ring or a fused ring,
in said fused ring, the number of rings can be 2.

In a preferred embodiment, 5-12 membered heteroaryl described in any embodiment of the present invention is 5-9 membered heteroaryl.

In a preferred embodiment, described in any embodiment of the present invention 5-12 membered heteroaryl has a heteroatom that is N and/or O.

In a preferred embodiment, 5-12 membered heteroaryl described in any embodiment of the present invention is independently 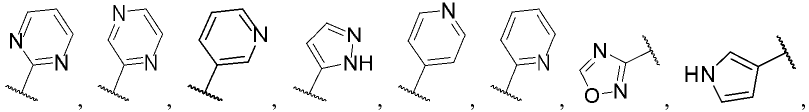 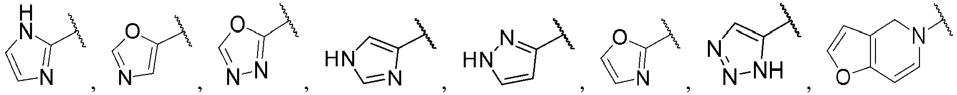 or

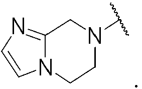

In a preferred embodiment, C₁₋₆ alkoxy described in any embodiment of the present invention is independently methoxy, ethoxy, n-propoxy, isopropoxy, n-butoxy, isobutoxy, sec-butoxy, or tert-butoxy.

In a preferred embodiment, R^{a} is hydrogen.

In a preferred embodiment, X² is N.

In a preferred embodiment, X³ is N.

In a preferred embodiment, X⁴ is N.

In a preferred embodiment, R^{b} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³.

In a preferred embodiment, R^{b} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹².

In a preferred embodiment, R^{b} is C₃₋₁₀ cycloalkyl unsubstituted or substituted by one or more R¹⁻⁹, C₆₋₁₂ aryl unsubstituted or substituted by one or more R¹⁻¹¹ or C₁₋₆ alkyl unsubstituted or substituted by one or more R¹⁻¹³.

In a preferred embodiment, R^{b} is C₃₋₁₀ cycloalkyl unsubstituted or substituted by one or more R¹⁻⁹ or C₆₋₁₂ aryl unsubstituted or substituted by one or more R¹⁻¹¹.

In a preferred embodiment, R^{b} is unsubstituted C₃₋₁₀ cycloalkyl, unsubstituted C₆₋₁₂ aryl or unsubstituted C₁₋₆ alkyl, preferably, unsubstituted C₁₋₆ alkyl.

In a preferred embodiment, R^{b} is unsubstituted C₃₋₁₀ cycloalkyl or unsubstituted C₆₋₁₂ aryl.

In a preferred embodiment, R^{c} is C₃₋₁₀ cycloalkyl unsubstituted or substituted by one or more R¹⁻⁹ or 3-12 membered heterocycloalkyl unsubstituted or substituted by one or more R¹⁻¹⁰.

In a preferred embodiment, R^{c} is C₃₋₁₀ cycloalkyl unsubstituted or substituted by one or more R¹⁻⁹.

In a preferred embodiment, R^{c} is unsubstituted C₃₋₁₀ cycloalkyl.

In a preferred embodiment, R^{d} is C₃₋₁₀ cycloalkyl unsubstituted or substituted by one or more R¹⁻⁹ or 3-12 membered heterocycloalkyl unsubstituted or substituted by one or more R¹⁻¹⁰.

In a preferred embodiment, R^{d} is C₃₋₁₀ cycloalkyl unsubstituted or substituted by one or more R¹⁻⁹.

In a preferred embodiment, R^{d} is unsubstituted C₃₋₁₀ cycloalkyl.

In a preferred embodiment, R^{e} is C₁₋₆ alkyl unsubstituted or substituted by one or more R¹⁻¹³.

In a preferred embodiment, R^{e} is unsubstituted C₁₋₆ alkyl.

In a preferred embodiment, R^{f} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹².

In a preferred embodiment, R^{f} is 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹².

In a preferred embodiment, R^{f} is unsubstituted 3-12 membered heterocycloalkyl, unsubstituted C₆₋₁₂ aryl or unsubstituted 5-12 membered heteroaryl.

In a preferred embodiment, R^{g} is C₃₋₁₀ cycloalkyl unsubstituted or substituted by one or more R¹⁻⁹ or 3-12 membered heterocycloalkyl unsubstituted or substituted by one or more R¹⁻¹⁰.

In a preferred embodiment, R^{g} is C₃₋₁₀ cycloalkyl unsubstituted or substituted by one or more R¹⁻⁹.

In a preferred embodiment, R^{g} is unsubstituted C₃₋₁₀ cycloalkyl.

In a preferred embodiment, R^{h} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹².

In a preferred embodiment, R^{h} is 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹².

In a preferred embodiment, R^{h} is unsubstituted C₃₋₁₀ cycloalkyl, unsubstituted 3-12 membered heterocycloalkyl, unsubstituted C₆₋₁₂ aryl or unsubstituted 5-12 membered heteroaryl.

In a preferred embodiment, R^{h} is unsubstituted 3-12 membered heterocycloalkyl, unsubstituted C₆₋₁₂ aryl or unsubstituted 5-12 membered heteroaryl.

In a preferred embodiment, Rⁱ is C₃₋₁₀ cycloalkyl unsubstituted or substituted by one or more R¹⁻⁹, 3-12 membered heterocycloalkyl unsubstituted or substituted by one or more R¹⁻¹⁰ or C₆₋₁₂ aryl unsubstituted or substituted by one or more R¹⁻¹¹.

In a preferred embodiment, Rⁱ is 3-12 membered heterocycloalkyl unsubstituted or substituted by one or more R¹⁻¹⁰ or C₆₋₁₂ aryl unsubstituted or substituted by one or more R¹⁻¹¹.

In a preferred embodiment, Rⁱ is unsubstituted 3-12 membered heterocycloalkyl or unsubstituted C₆₋₁₂ aryl.

In a preferred embodiment, R^{j} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹².

In a preferred embodiment, R^{j} is 3-12 membered heterocycloalkyl unsubstituted or substituted by one or more R¹⁻¹⁰ or 5-12 membered heteroaryl unsubstituted or substituted by one or more R¹⁻¹².

In a preferred embodiment, R^{j} is unsubstituted 3-12 membered heterocycloalkyl or unsubstituted 5-12 membered heteroaryl.

In a preferred embodiment, R^{k} is C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³.

In a preferred embodiment, R^{k} is 5-12 membered heteroaryl unsubstituted or substituted by one or more R¹⁻¹² or C₁₋₆ alkyl unsubstituted or substituted by one or more R¹⁻¹³.

In a preferred embodiment, R^{k} is unsubstituted 5-12 membered heteroaryl or unsubstituted C₁₋₆ alkyl.

In a preferred embodiment, each R¹⁻³ is independently halogen.

In a preferred embodiment, each R¹⁻⁴ is independently halogen.

In a preferred embodiment, each R¹⁻⁵ is independently halogen or C₁₋₆ alkyl.

In a preferred embodiment, each R¹⁻⁵ is independently C₁₋₆ alkyl.

In a preferred embodiment, each R¹⁻⁵ is independently halogen.

In a preferred embodiment, each R¹⁻⁶ is independently halogen or C₁₋₆ alkyl.

In a preferred embodiment, each R¹⁻⁶ is independently C₁₋₆ alkyl.

In a preferred embodiment, each R¹⁻⁷ is independently halogen or C₁₋₆ alkyl.

In a preferred embodiment, each R¹⁻⁷ is independently halogen.

In a preferred embodiment, each R¹⁻⁸ is independently C₁₋₆ alkyl, -S(O)₂C₁₋₆ alkyl or -P(O)(C₁₋₆ alkyl)₂.

In a preferred embodiment, each R¹⁻⁸ is independently C₁₋₆ alkyl.

In a preferred embodiment, R¹ is hydrogen, deuterium, cyano, hydroxyl, halogen, amino, -NHC(O)R^{b}, - NHS(O)₂R^{c}, -S(O)₂NHR^{d}, 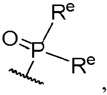 -NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸.

In a preferred embodiment, R¹ is hydrogen, deuterium, cyano, hydroxyl, halogen, -NHC(O)R^{b}, -NHS(O)₂R^{c}, -S(O)₂NHR^{d}, 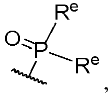 -NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸.

In a preferred embodiment, R¹ is hydrogen, halogen, amino, -NHC(O)R^{b}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸.

In a preferred embodiment, preferably, R¹ is hydrogen, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸.

In a preferred embodiment, R¹ is hydrogen, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, C₆₋₁₂ aryl unsubstituted or substituted by one or more R¹⁻⁷ or 5-12 membered heteroaryl unsubstituted or substituted by one or more R¹⁻⁸.

In a preferred embodiment, R¹ is hydrogen, halogen, amino, -NHC(O)R^{b}, unsubstituted C₁₋₆ alkyl, unsubstituted C₁₋₆ alkoxy, unsubstituted C₃₋₁₀ cycloalkyl, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸.

In a preferred embodiment, R¹ is hydrogen, unsubstituted C₁₋₆ alkyl, unsubstituted C₁₋₆ alkoxy, unsubstituted C₃₋₁₀ cycloalkyl, unsubstituted C₆₋₁₂ aryl or unsubstituted 5-12 membered heteroaryl.

In a preferred embodiment, R¹ is hydrogen, unsubstituted C₁₋₆ alkyl, unsubstituted C₁₋₆ alkoxy, unsubstituted C₃₋₁₀ cycloalkyl, unsubstituted 3-12 membered heterocycloalkyl, unsubstituted C₆₋₁₂ aryl or 5-12 membered heteroaryl unsubstituted or substituted by one or more R¹⁻⁸.

In a preferred embodiment, m is 1, 2 or 3.

In a preferred embodiment, m is 1 or 2.

In a preferred embodiment, m is 2.

In a preferred embodiment, n is 1, 2 or 3.

In a preferred embodiment, n is 1.

In a preferred embodiment, each of R²⁻¹ and R²⁻² is independently halogen.

In a preferred embodiment, each R² is independently halogen, C₁₋₆ alkyl unsubstituted or substituted by one or more R²⁻¹ or C₁₋₆ alkoxy unsubstituted or substituted by one or more R²⁻².

In a preferred embodiment, each R² is independently halogen, C₁₋₆ alkyl substituted by one or more R²⁻¹ or unsubstituted C₁₋₆ alkoxy.

In a preferred embodiment, each R³⁻¹ is independently

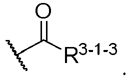

In a preferred embodiment, each R³⁻¹⁻³ is independently unsubstituted 3-12 membered heterocycloalkyl.

In a preferred embodiment, each R³⁻³ is independently 3-12 membered heterocycloalkyl unsubstituted or substituted by one or more R³⁻¹⁻¹.

In a preferred embodiment, each R³⁻³ is independently unsubstituted 3-12 membered heterocycloalkyl.

In a preferred embodiment, each R³⁻⁵ is independently oxo or C₁₋₆ alkyl unsubstituted or substituted by one or more R³⁻⁵⁻¹

In a preferred embodiment, each R³⁻⁵ is independently oxo or unsubstituted C₁₋₆ alkyl.

In a preferred embodiment, each R³⁻⁶ is independently cyano or C₁₋₆ alkyl unsubstituted or substituted by one or more R³⁻⁶⁻¹.

In a preferred embodiment, each R³⁻⁶ is independently cyano or unsubstituted C₁₋₆ alkyl.

In a preferred embodiment, each R³⁻¹⁰ is independently hydroxyl.

In a preferred embodiment, each R³⁻⁸ is independently 3-12 membered heterocycloalkyl unsubstituted or substituted by one or more R³⁻⁸⁻¹.

In a preferred embodiment, each R³⁻⁸ is independently unsubstituted 3-12 membered heterocycloalkyl.

In a preferred embodiment, each R³⁻⁹ is independently oxo, halogen, cyano, hydroxyl, C₁-C₆ alkyl unsubstituted or substituted by one or more R³⁻⁵⁻¹ or C₁-C₆ alkoxy unsubstituted or substituted by one or more R³⁻⁵⁻².

In a preferred embodiment, each R³⁻⁹ is independently oxo, halogen, C₁₋₆ alkyl unsubstituted or substituted by one or more R³⁻⁵⁻¹ or C₁₋₆ alkoxy unsubstituted or substituted by one or more R³⁻⁵⁻².

In a preferred embodiment, each R³⁻⁹ is independently oxo, halogen, C₁₋₆ alkyl substituted by one or more R³⁻⁵⁻¹ or unsubstituted C₁₋₆ alkoxy.

In a preferred embodiment, each R³⁻⁹ is independently oxo or halogen.

In a preferred embodiment, each R³⁻⁵⁻¹ is independently halogen.

In a preferred embodiment, each R³ is independently C₁₋₆ alkyl unsubstituted or substituted by one or more R³⁻¹, C₂₋₆ alkynyl unsubstituted or substituted by one or more R³⁻³, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, C₆₋₁₂ aryl unsubstituted or substituted by one or more R³⁻¹⁰ or

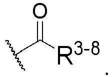

In a preferred embodiment, each R³ is independently C₁₋₆ alkyl unsubstituted or substituted by one or more R³⁻¹, C₃₋₆ cycloalkyl unsubstituted or substituted by one or more R³⁻⁴, 3-12 membered heterocycloalkyl unsubstituted or substituted by one or more R³⁻⁵ or 4-12 membered heterocycloalkenyl unsubstituted or substituted by one or more R³⁻⁹.

In a preferred embodiment, each R³ is independently C₁₋₆ alkyl unsubstituted or substituted by one or more R³⁻¹ or 4-12 membered heterocycloalkenyl unsubstituted or substituted by one or more R³⁻⁹.

In a preferred embodiment, each R³ is independently unsubstituted C₁₋₆ alkyl or 4-12 membered heterocycloalkenyl substituted by one or more R³⁻⁹.

In a preferred embodiment, each R³ is independently 4-12 membered heterocycloalkenyl substituted by one or more R³⁻⁹.

In a preferred embodiment, R¹ is hydrogen, deuterium, cyano, hydroxyl, fluorine, methyl,

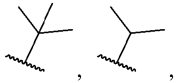

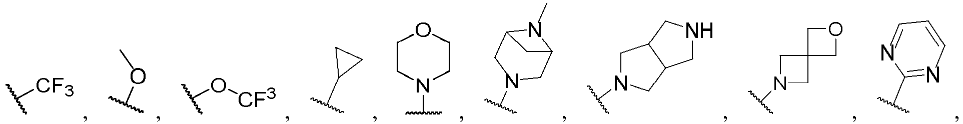

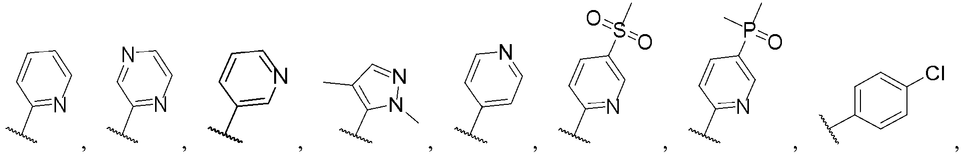

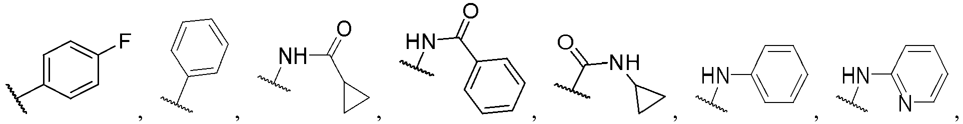

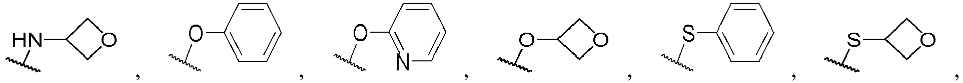

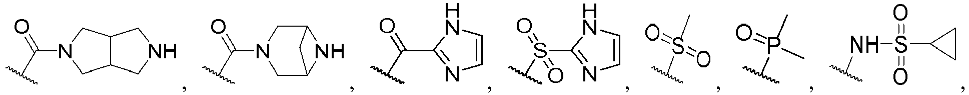

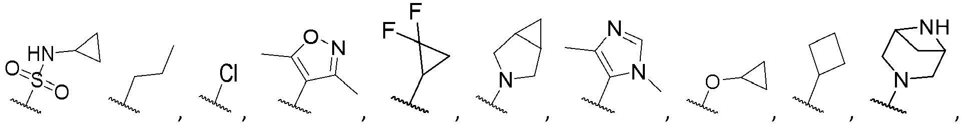

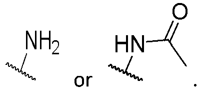

In a preferred embodiment, R¹ is hydrogen, methyl,

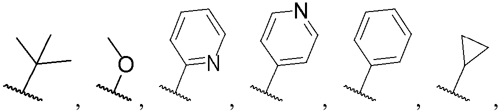

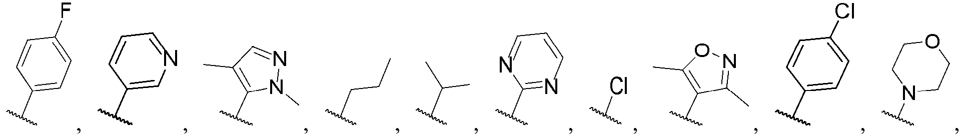

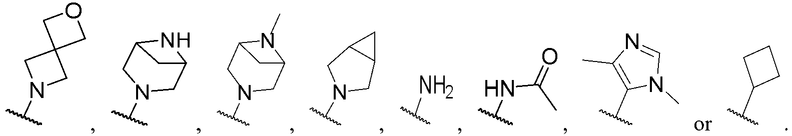

In a preferred embodiment, R¹ is hydrogen, deuterium, cyano, hydroxyl, fluorine, methyl, 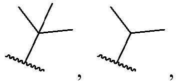 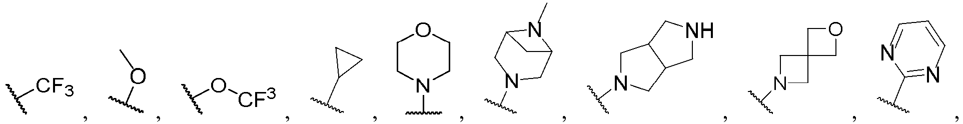 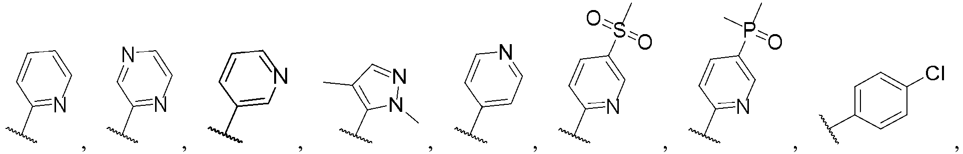 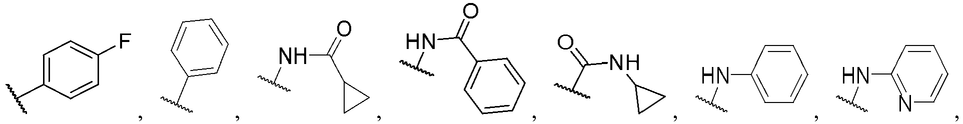 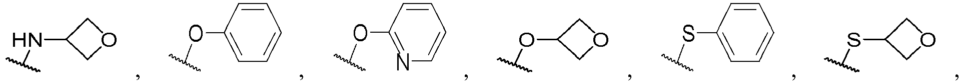 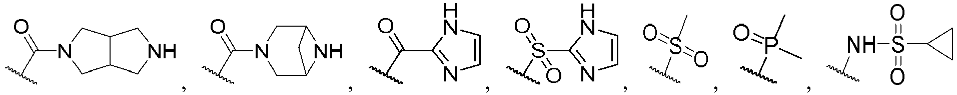 or

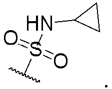

In a preferred embodiment, R¹ is hydrogen, methyl, 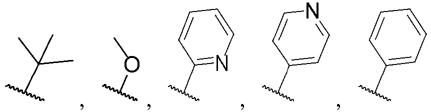 or

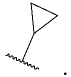

In a preferred embodiment,

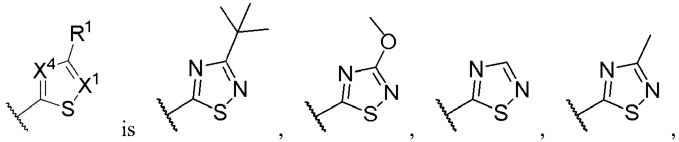

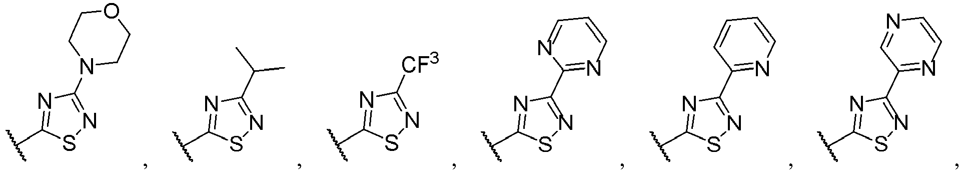

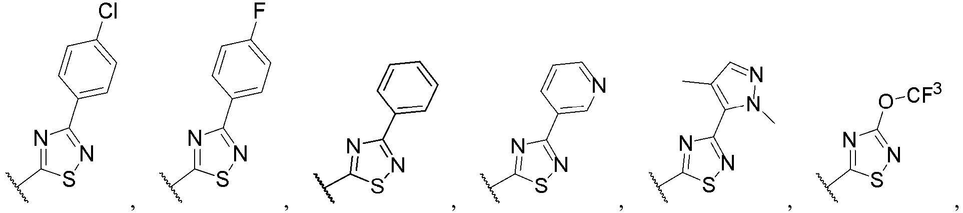

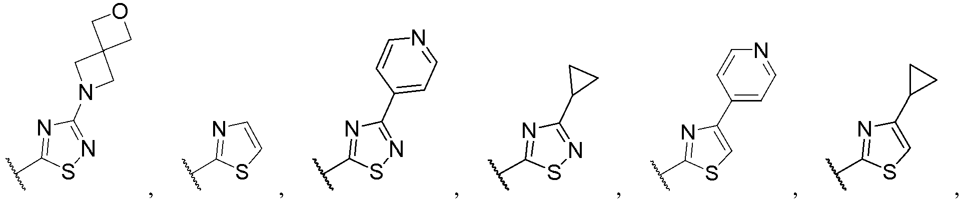

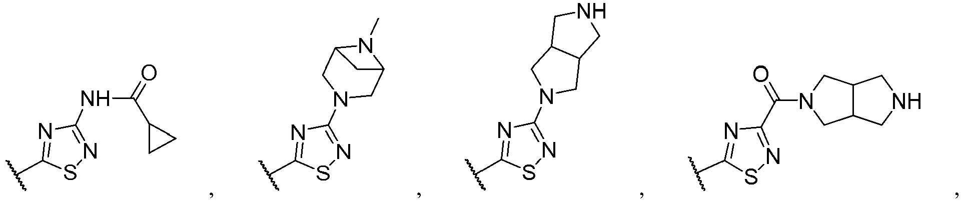

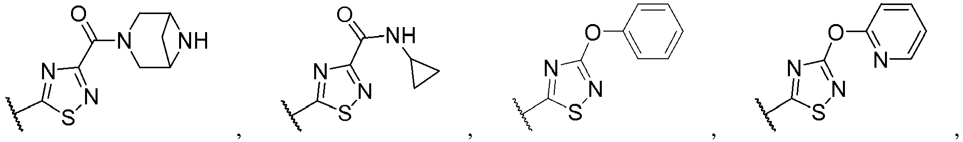

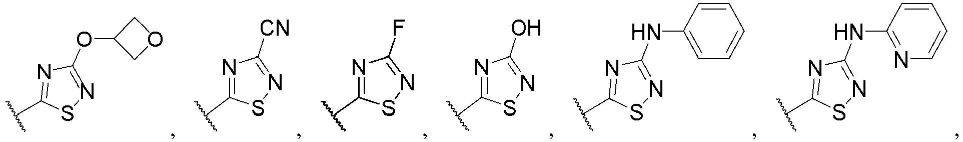

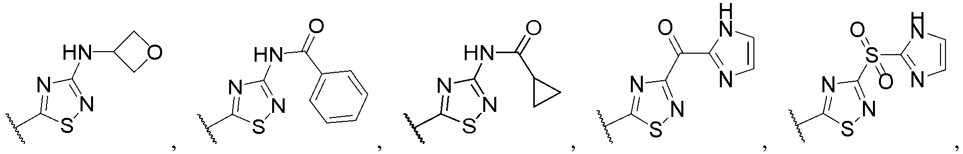

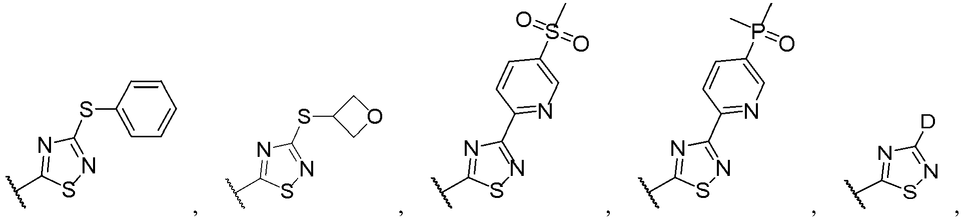

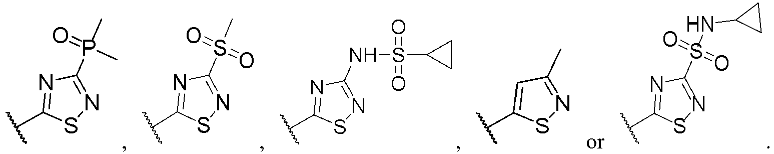

In a preferred embodiment,

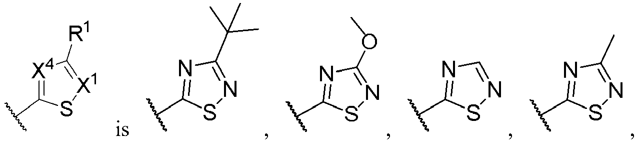

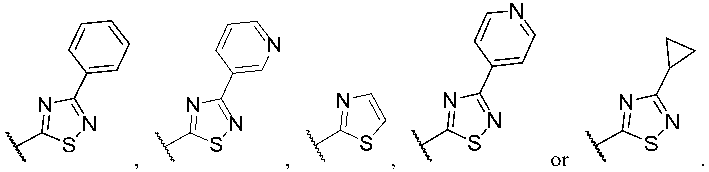

In a preferred embodiment,

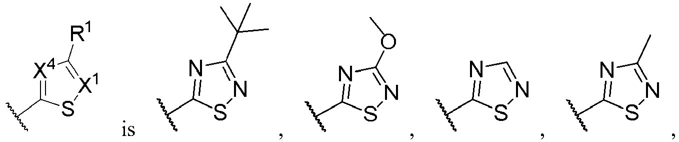

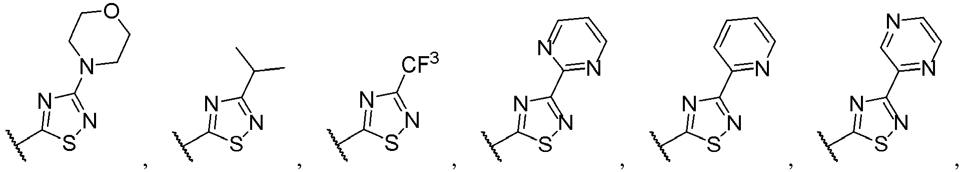

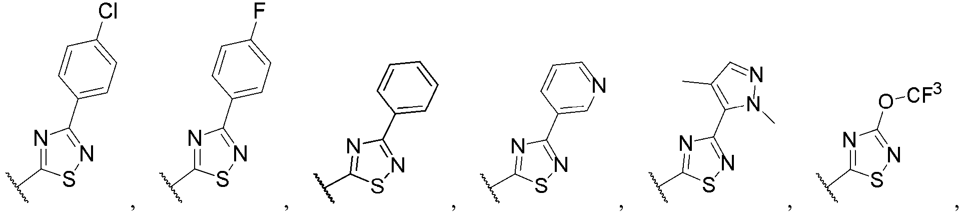

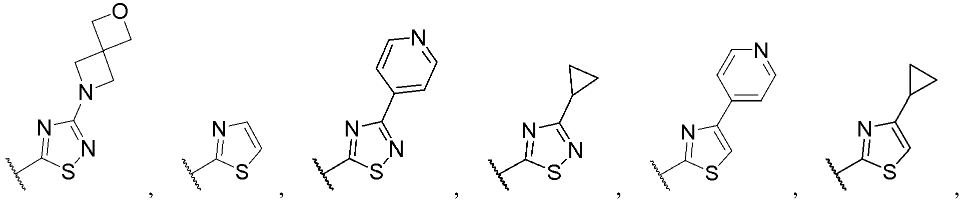

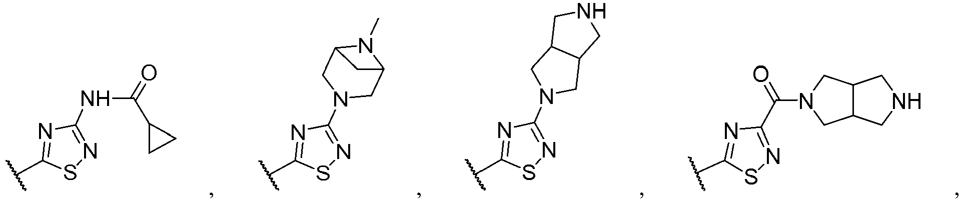

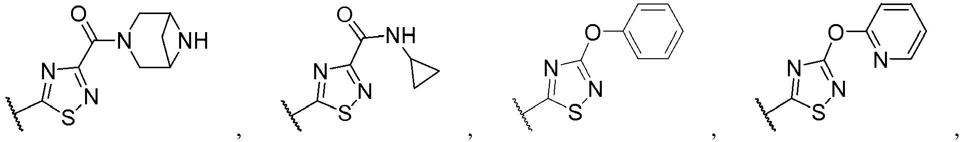

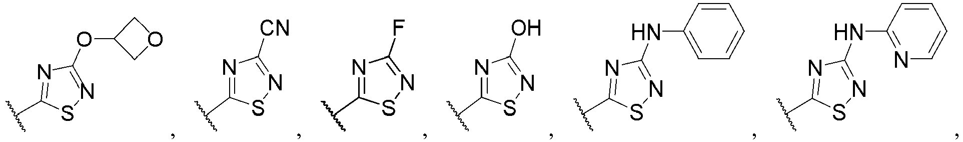

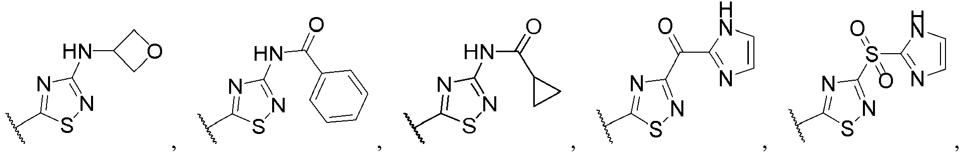

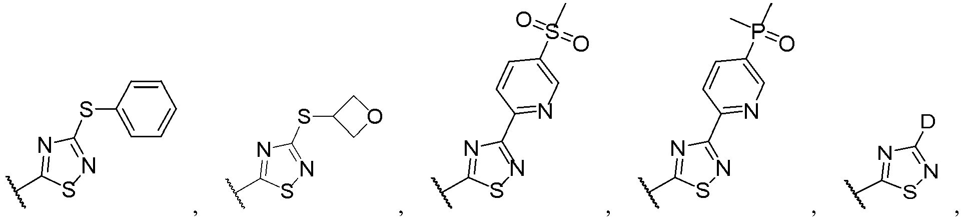

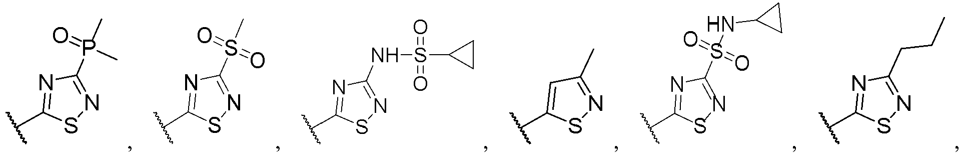

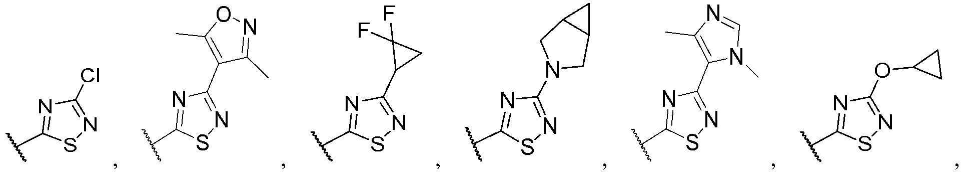

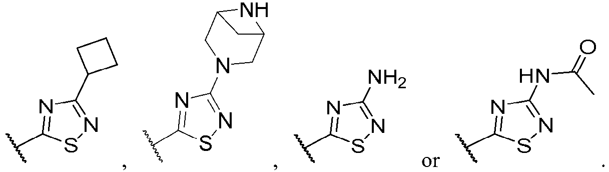

In a preferred embodiment,

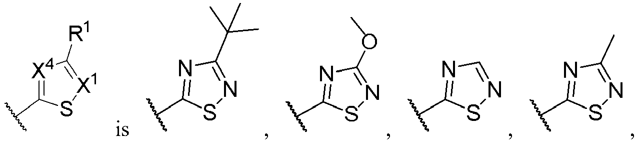

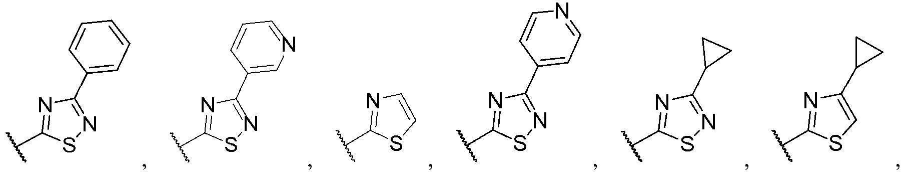

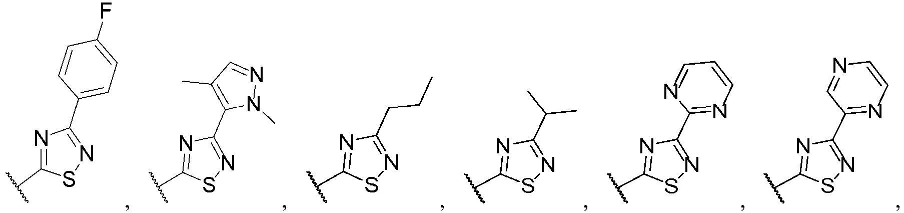

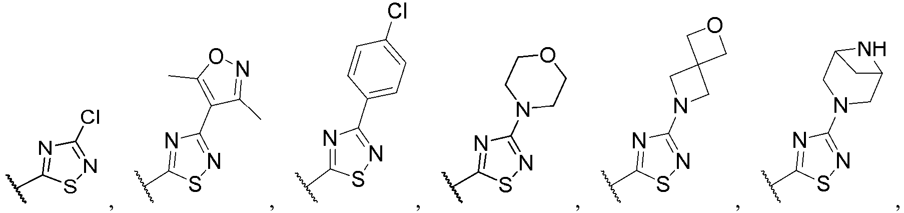

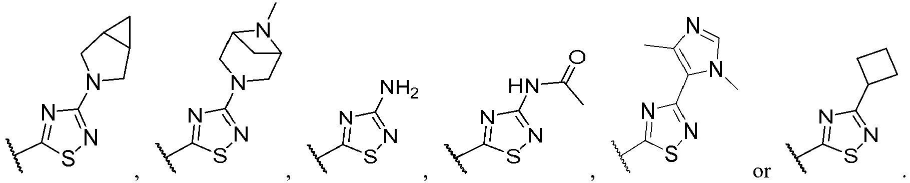

In a preferred embodiment, 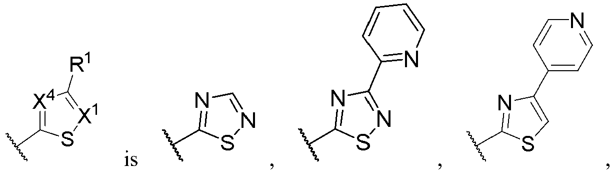 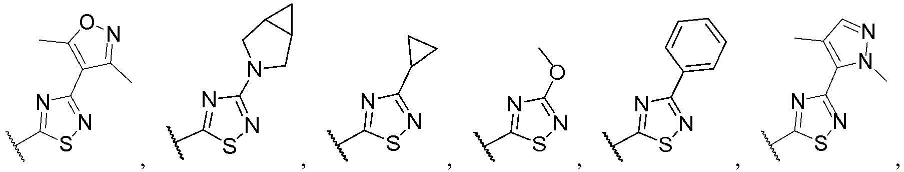 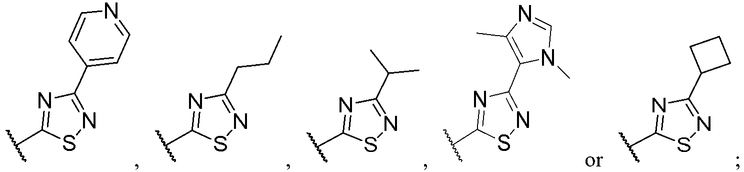 preferably,

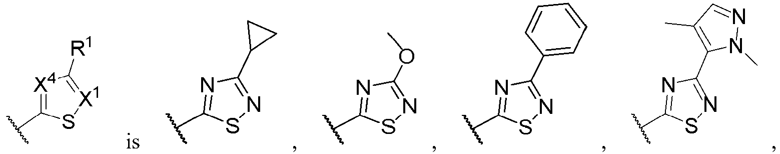

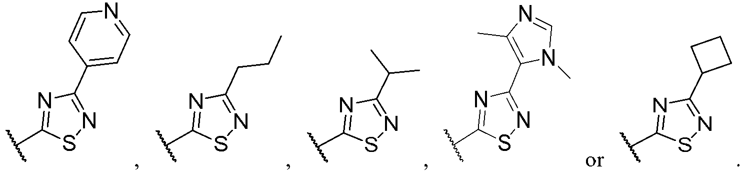

In a preferred embodiment, R² is

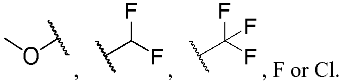

In a preferred embodiment, R² is

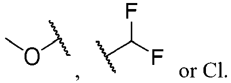

In a preferred embodiment,

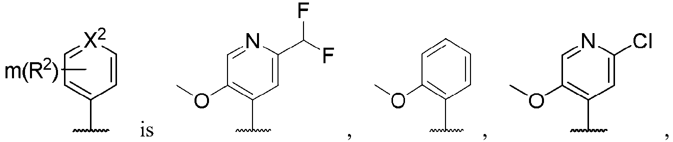

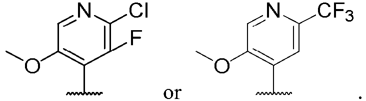

In a preferred embodiment,

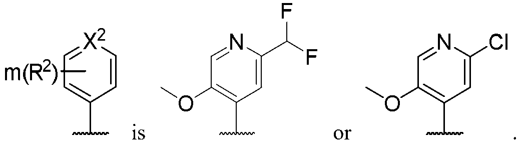

In a preferred embodiment, R³ is methyl,

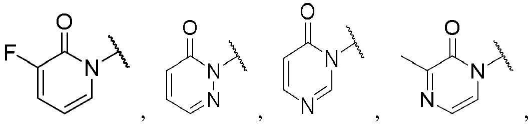

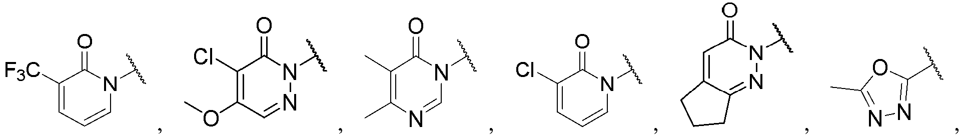

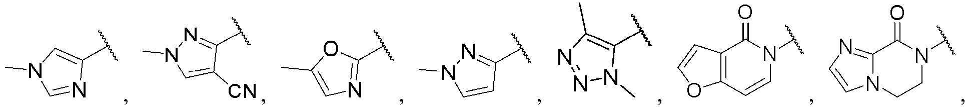

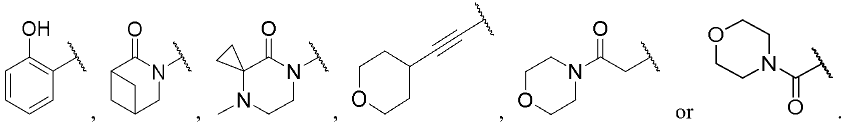

In a preferred embodiment, R³ is 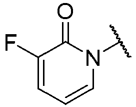 or methyl, preferably,

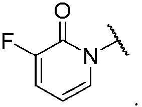

In a preferred embodiment, m and n are independently 1, 2 or 3;
X¹ is N or CH;
X² is N or CH;
X³ is N;
X⁴ is N or CH;
R¹ is hydrogen, deuterium, cyano, hydroxyl, halogen, amino, -NHC(O)R^{b}, -NHS(O)₂R^{c}, -S(O)₂NHR^{d}, 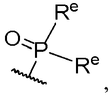 -NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸;
R^{b}, R^{c}, R^{d}, each R^{e}, R^{f}, R^{g}, R^{h}, Rⁱ, R^{j} and R^{k} are each independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, - S(O)₂C₁₋₆ alkyl or -P(O)(C₁₋₆ alkyl)₂;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently C₁₋₆ alkyl unsubstituted or substituted by one or more R³⁻¹, C₂₋₆ alkynyl unsubstituted or substituted by one or more R³⁻³, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, C₆₋₁₂ aryl unsubstituted or substituted by one or more R³⁻¹⁰ or 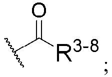
each R³⁻¹ is independently 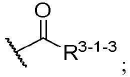
each R³⁻³ is independently 3-12 membered heterocycloalkyl unsubstituted or substituted by one or more R³⁻¹⁻¹;
each R³⁻⁵ is independently oxo or C₁₋₆ alkyl unsubstituted or substituted by one or more R³⁻⁵⁻¹;
each R³⁻⁶ is independently cyano or C₁₋₆ alkyl unsubstituted or substituted by one or more R³⁻⁶⁻¹;
each R³⁻⁹ is independently oxo, halogen, C₁₋₆ alkyl unsubstituted or substituted by one or more R³⁻⁵⁻¹ or C₁₋₆ alkoxy unsubstituted or substituted by one or more R³⁻⁵⁻²;
each R³⁻⁸ is independently 3-12 membered heterocycloalkyl unsubstituted or substituted by one or more R³⁻⁸⁻1.
each R³⁻¹⁰ is independently hydroxyl;
each R³⁻¹⁻¹ is independently hydroxyl, cyano, halogen or C₁₋₆ alkyl;
each R³⁻¹⁻³ is independently unsubstituted 3-12 membered heterocycloalkyl;
each of R³⁻⁵⁻¹ and R³⁻⁵⁻² is independently deuterium, hydroxyl, cyano or halogen;
each R³⁻⁶⁻¹ is independently hydroxyl, cyano, halogen or C₁₋₆ alkoxy;
each R³⁻⁸⁻¹ is independently hydroxyl, cyano, halogen or C₁₋₆ alkyl;
each of 3-12 membered heterocycloalkyl, 4-12 membered heterocycloalkenyl and 5-12 membered heteroaryl has a heteroatom that is independently selected from one, two or three of N, O and S, and the number of heteroatom(s) is independently 1, 2 or 3.

In a preferred embodiment, m and n are independently 1, 2 or 3;
X¹ is N or CH;
X² is N or CH;
X³ is N;
X⁴ is N or CH;
R¹ is hydrogen, deuterium, cyano, hydroxyl, halogen, -NHC(O)R^{b}, -NHS(O)₂R^{c}, -S(O)₂NHR^{d}, 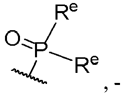 NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸;
R^{b}, R^{c}, R^{d}, each R^{e}, R^{f}, R^{g}, R^{h}, Rⁱ, R^{j} and R^{k} are each independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, - S(O)₂C₁₋₆ alkyl or -P(O)(C₁₋₆ alkyl)₂;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently C₁₋₆ alkyl unsubstituted or substituted by one or more R³⁻¹, C₂₋₆ alkynyl unsubstituted or substituted by one or more R³⁻³, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, C₆₋₁₂ aryl unsubstituted or substituted
by one or more R³⁻¹⁰ or 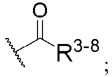
each R³⁻¹ is independently 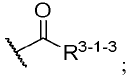
each R³⁻³ is independently 3-12 membered heterocycloalkyl unsubstituted or substituted by one or more R³⁻¹⁻¹;
each R³⁻⁵ is independently oxo or C₁₋₆ alkyl unsubstituted or substituted by one or more R³⁻⁵⁻¹;
each R³⁻⁶ is independently cyano or C₁₋₆ alkyl unsubstituted or substituted by one or more R³⁻⁶⁻¹;
each R³⁻⁹ is independently oxo, halogen, C₁₋₆ alkyl unsubstituted or substituted by one or more R³⁻⁵⁻¹ or C₁₋₆ alkoxy unsubstituted or substituted by one or more R³⁻⁵⁻²;
each R³⁻⁸ is independently 3-12 membered heterocycloalkyl unsubstituted or substituted by one or more R³⁻⁸⁻¹;
each R³⁻¹⁰ is independently hydroxyl;
each R³⁻¹⁻¹ is independently hydroxyl, cyano, halogen or C₁₋₆ alkyl;
each R³⁻¹⁻³ is independently unsubstituted 3-12 membered heterocycloalkyl;
each of R³⁻⁵⁻¹ and R³⁻⁵⁻² is independently deuterium, hydroxyl, cyano or halogen;
each R³⁻⁶⁻¹ is independently hydroxyl, cyano, halogen or C₁₋₆ alkoxy;
each R³⁻⁸⁻¹ is independently hydroxyl, cyano, halogen or C₁₋₆ alkyl; and
each of 3-12 membered heterocycloalkyl, 4-12 membered heterocycloalkenyl and 5-12 membered heteroaryl has a heteroatom that is independently selected from one, two or three of N, O and S, and the number of heteroatom(s) is independently 1, 2 or 3.

In a preferred embodiment, m is 1 or 2;
n is 1;
X¹ is N or CH;
X² is N or CH;
X³ is N;
X⁴ is N;
R¹ is hydrogen, deuterium, cyano, hydroxyl, halogen, -NHC(O)R^{b}, -NHS(O)₂R^{c}, -S(O)₂NHR^{d}, 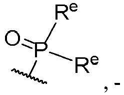 NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸;
R^{b}, R^{c}, R^{d}, each R^{e}, R^{f}, R^{g}, R^{h}, Rⁱ, R^{j} and R^{k} are each independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, - S(O)₂C₁₋₆ alkyl or -P(O)(C₁₋₆ alkyl)₂;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently unsubstituted C₁₋₆ alkyl or 4-12 membered heterocycloalkenyl unsubstituted or substituted by one or more R³⁻⁹;
each R³⁻⁹ is independently oxo or halogen; and
each of 3-12 membered heterocycloalkyl, 4-12 membered heterocycloalkenyl and 5-12 membered heteroaryl has a heteroatom that is independently selected from one, two or three of N, O and S, and the number of heteroatom(s) is independently 1, 2 or 3.
In a preferred embodiment, m is 1 or 2;
n is 1;
X¹ is N or CH;
X², X³ and X⁴ are N;
R¹ is hydrogen, halogen, amino, -NHC(O)R^{b}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸;
each of R¹⁻³ and R¹⁻⁴ is independently halogen;
each of R¹⁻⁵, R¹⁻⁶, R¹⁻⁷ and R¹⁻⁸ is independently halogen or C₁₋₆ alkyl;
R^{b} is unsubstituted C₁₋₆ alkyl;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently unsubstituted C₁₋₆ alkyl or 4-12 membered heterocycloalkenyl unsubstituted or substituted by one or more R³⁻⁹;
each R³⁻⁹ is independently oxo or halogen; and
each of 4-12 membered heterocycloalkenyl and 5-12 membered heteroaryl has a heteroatom that is independently selected from one, two or three of N, O and S, and the number of heteroatom(s) is independently 1, 2 or 3.

In a preferred embodiment, m is 1 or 2;
n is 1;
X¹ is N or CH;
X², X³ and X⁴ are N;
R¹ is hydrogen, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, C₆₋₁₂ aryl unsubstituted or substituted by one or more R¹⁻⁷ or 5-12 membered heteroaryl unsubstituted or substituted by one or more R¹⁻⁸;
each of R¹⁻³ and R¹⁻⁴ is independently halogen;
each of R¹⁻⁵, R¹⁻⁷ and R¹⁻⁸ is independently halogen or C₁₋₆ alkyl;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently unsubstituted C₁₋₆ alkyl or 4-12 membered heterocycloalkenyl unsubstituted or substituted by one or more R³⁻⁹;
each R³⁻⁹ is independently oxo or halogen; and
each of 4-12 membered heterocycloalkenyl and 5-12 membered heteroaryl has a heteroatom that is independently selected from one, two or three of N, O and S, and the number of heteroatom(s) is independently 1, 2 or 3.

In a preferred embodiment, m is 1 or 2;
n is 1;
X¹ is N or CH;
X², X³ and X⁴ are N;
R¹ is hydrogen, unsubstituted C₁₋₆ alkyl, unsubstituted C₁₋₆ alkoxy, unsubstituted C₃₋₁₀ cycloalkyl, unsubstituted 3-12 membered heterocycloalkyl, unsubstituted C₆₋₁₂ aryl or 5-12 membered heteroaryl unsubstituted or substituted by one or more R¹⁻⁸;
each R¹⁻⁸ is independently C₁₋₆ alkyl;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
R³ is 4-12 membered heterocycloalkenyl unsubstituted or substituted by one or more R³⁻⁹;
each R³⁻⁹ is independently oxo or halogen; and
each of 4-12 membered heterocycloalkenyl and 5-12 membered heteroaryl has a heteroatom that is independently selected from one, two or three of N, O and S, and the number of heteroatom(s) is independently 1, 2 or 3.

In a preferred embodiment, m is 1 or 2;
n is 1;
X² and X³ are N; 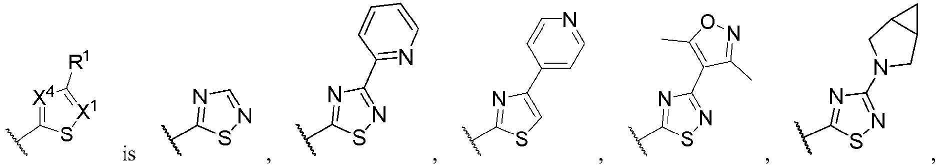 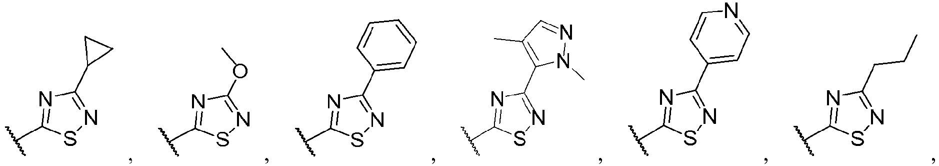 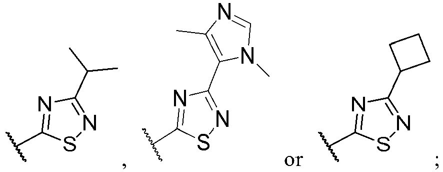
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
R³ is 4-12 membered heterocycloalkenyl unsubstituted or substituted by one or more R³⁻⁹;
each R³⁻⁹ is independently oxo or halogen; and
each 4-12 membered heterocycloalkenyl has a heteroatom that is independently selected from one, two or three of N, O and S, and the number of heteroatom(s) is independently 1, 2 or 3.

In a preferred embodiment, said compound of Formula I is any one of the following compounds:

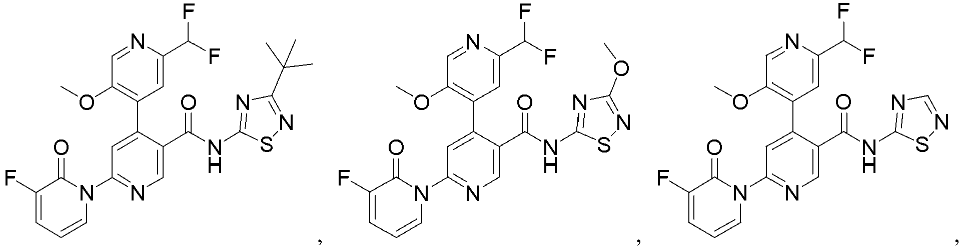

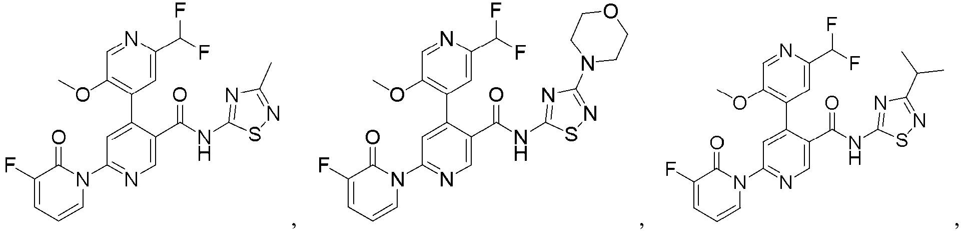

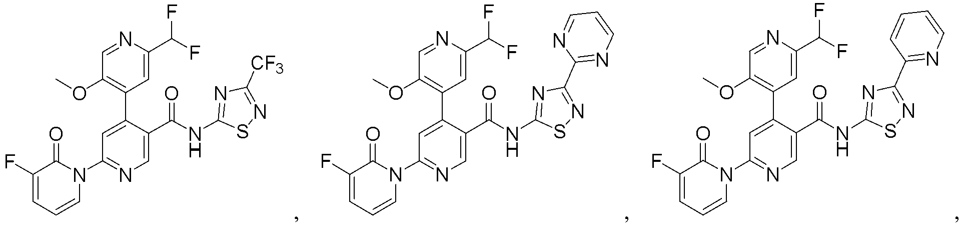

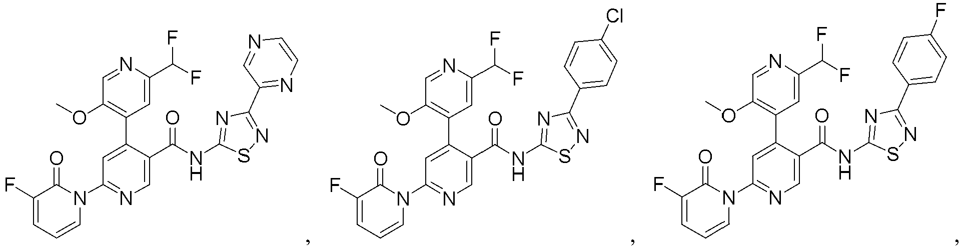

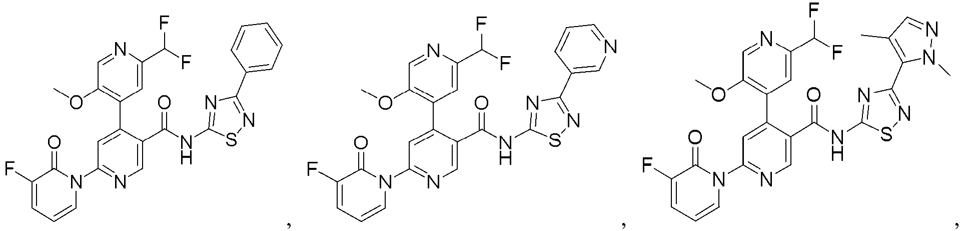

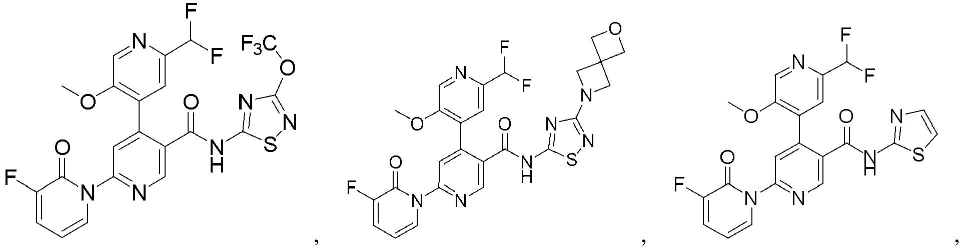

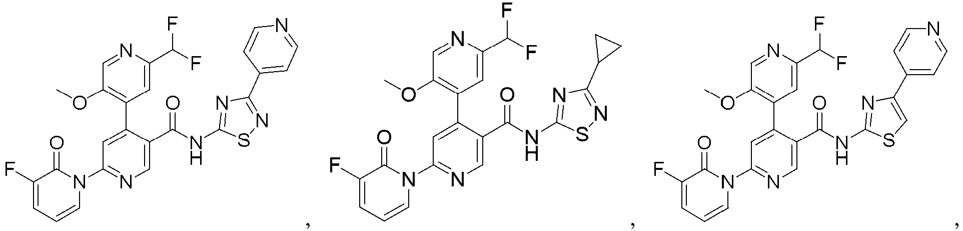

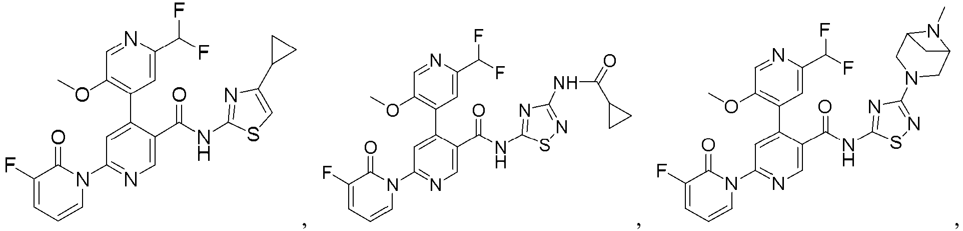

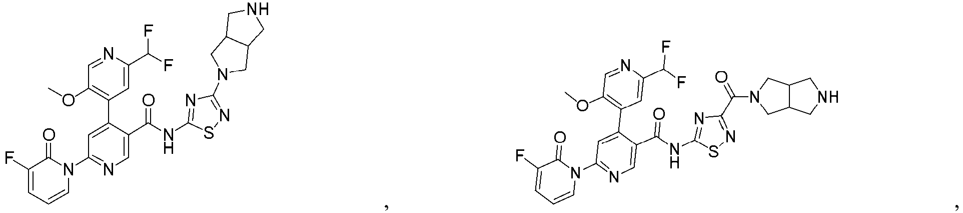

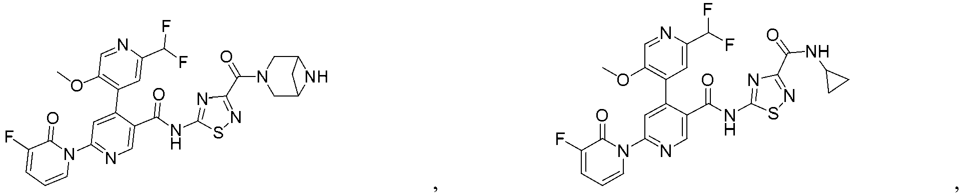

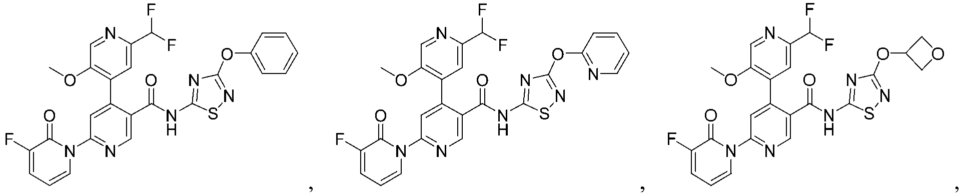

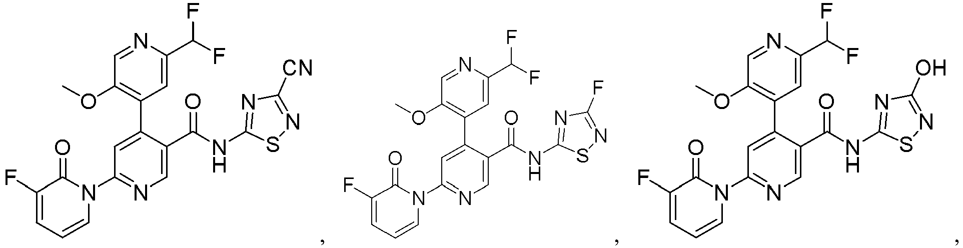

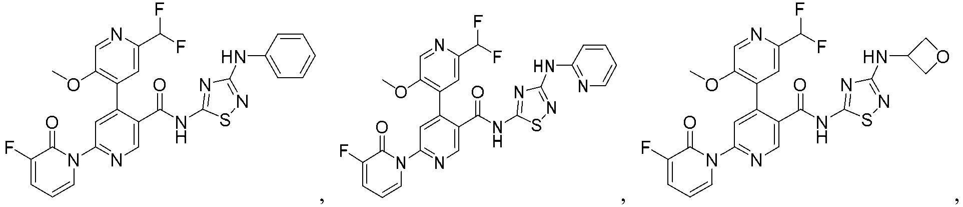

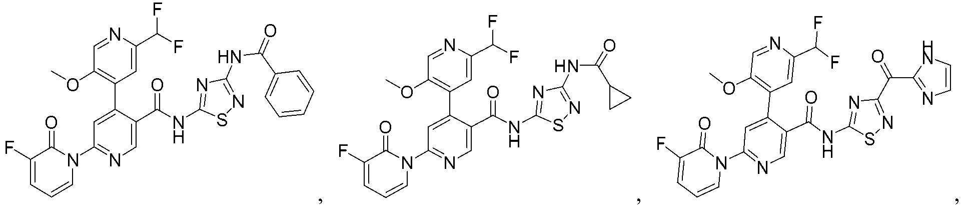

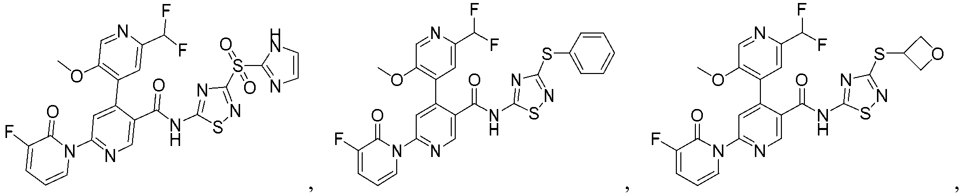

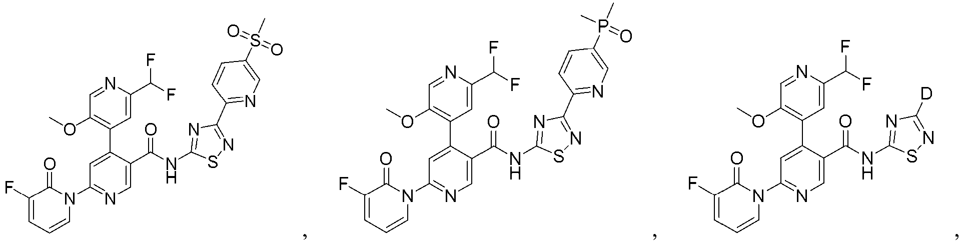

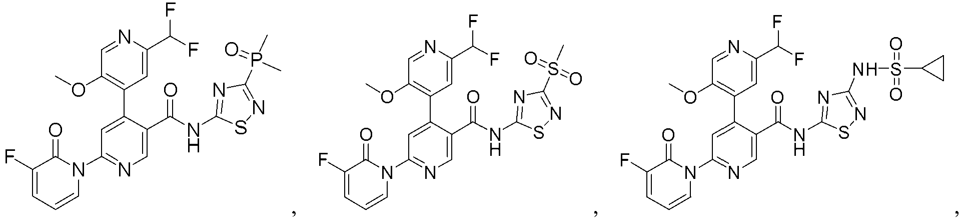

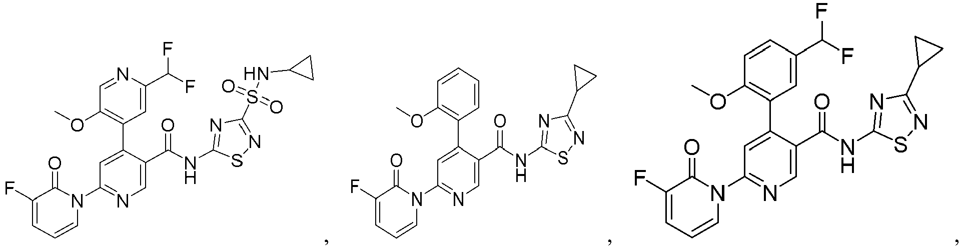

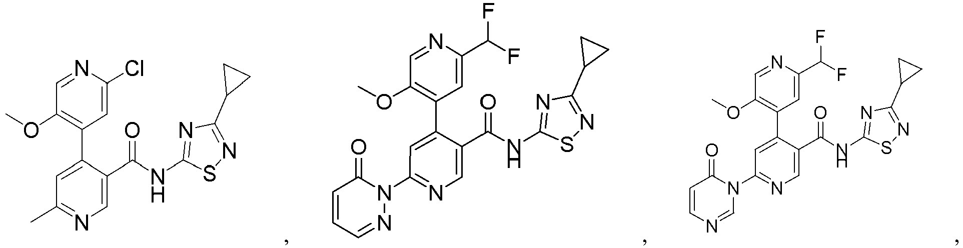

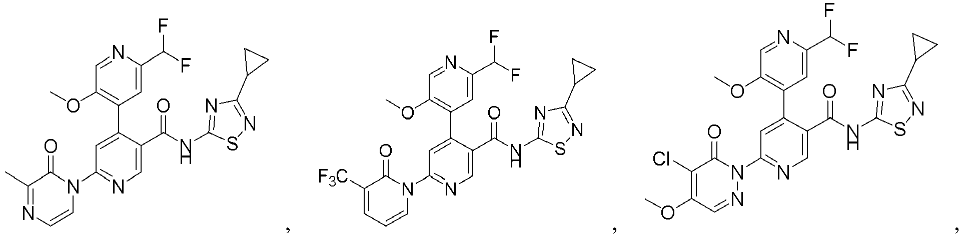

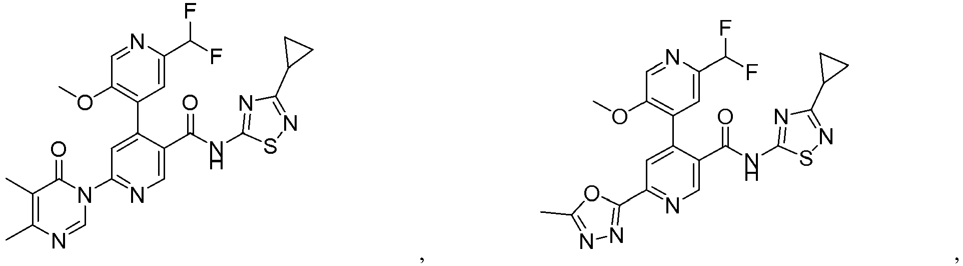

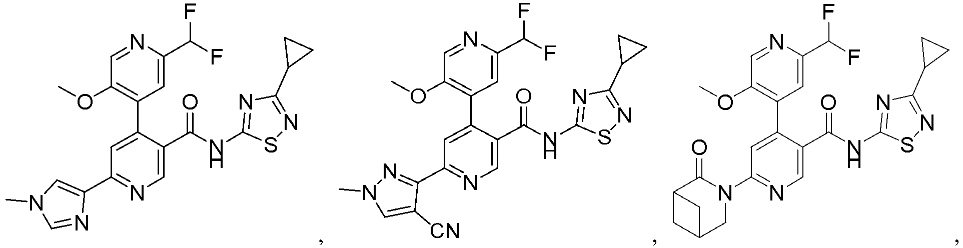

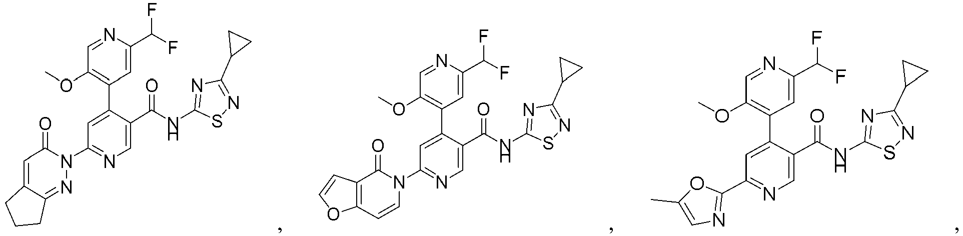

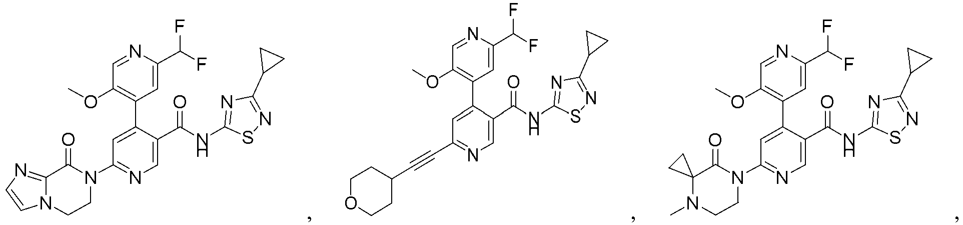

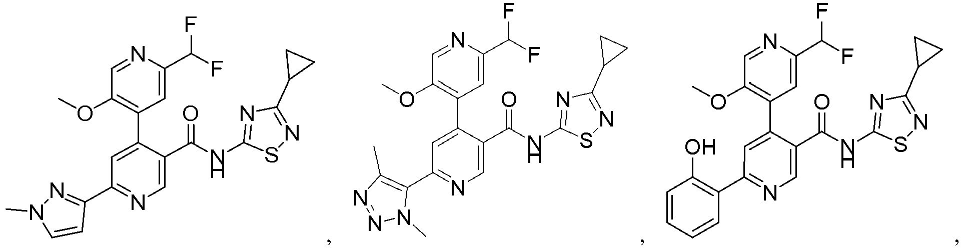

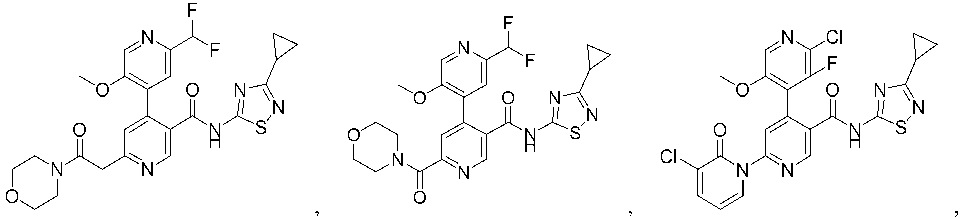

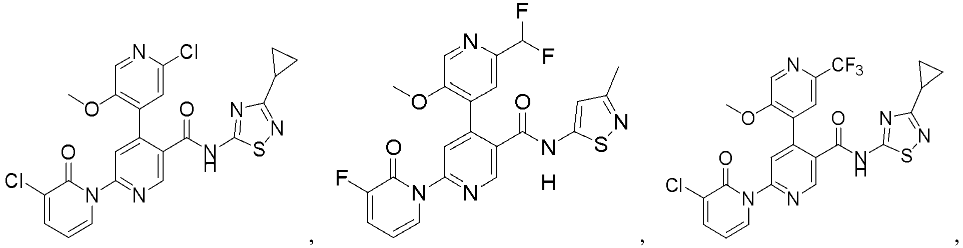

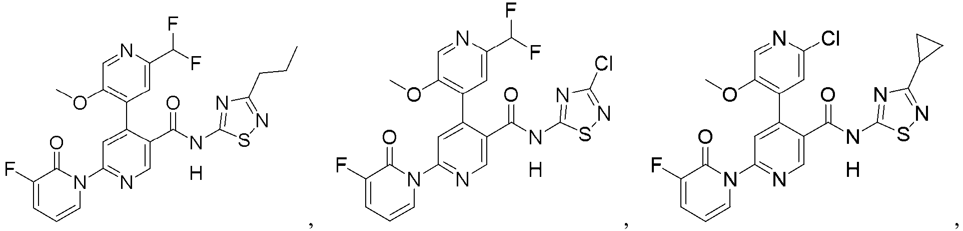

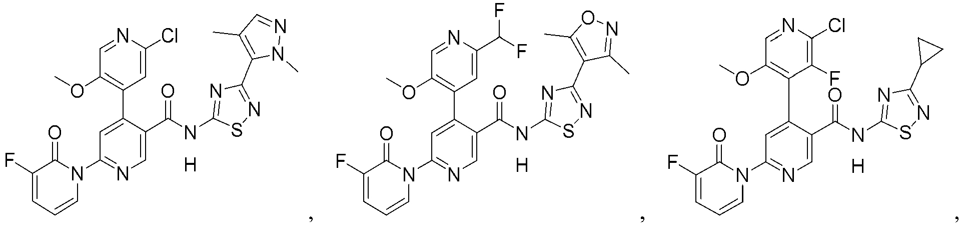

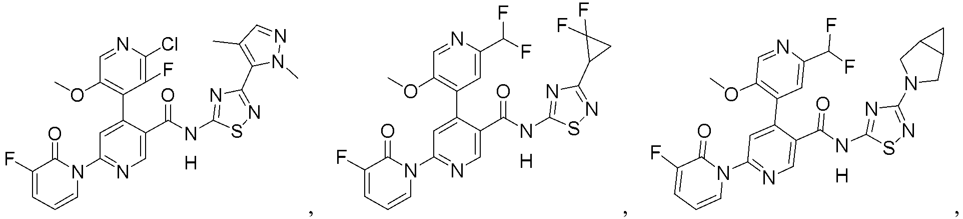

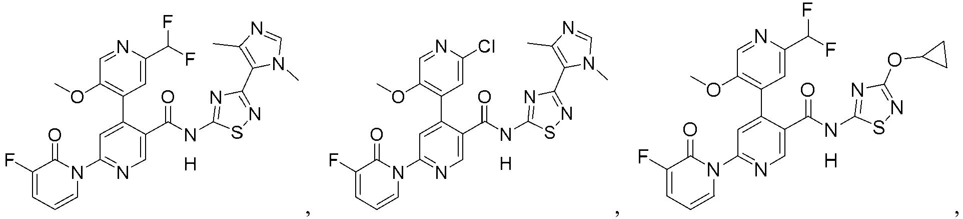

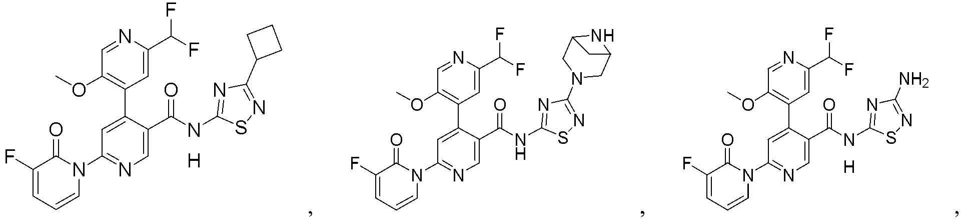

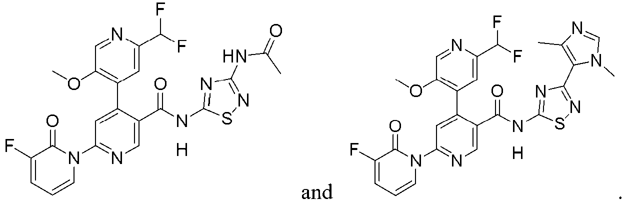

The present invention provides a pharmaceutical composition, comprising:
(1) a (therapeutically effective amount of) substance A, which is the compound of Formula I, the pharmaceutically acceptable salt thereof or the isotopic compound thereof as described in any embodiment of the present invention; and
(2) a pharmaceutical excipient.

The present invention provides use of a substance A or the pharmaceutical composition described above in preparation of a polymerase theta inhibitor, wherein said substance A is the compound of Formula I, the pharmaceutically acceptable salt thereof or the isotopic compound thereof as previously defined.

The present invention provides use of a substance A in preparation of a polymerase theta inhibitor, wherein said substance A is the compound of Formula I, the pharmaceutically acceptable salt thereof or the isotopic compound thereof as previously defined.

The present invention provides use of a substance A or the pharmaceutical composition described above in preparation of a medicament, wherein said substance A is the compound of Formula I, the pharmaceutically acceptable salt thereof or the isotopic compound thereof as described in any embodiment of the present invention; and said medicament is useful in treatment and/or prevention of lung cancer, breast cancer, HR-deficient ovarian cancer, gastric cancer, prostate cancer, pancreatic cancer or colon cancer.

The present invention provides use of a substance A in preparation of a medicament, wherein said substance A is the compound of Formula I, the pharmaceutically acceptable salt thereof or the isotopic compound thereof as described in any embodiment of the present invention; and said medicament is useful in treatment and/or prevention of lung cancer, breast cancer, HR-deficient ovarian cancer, gastric cancer, prostate cancer, pancreatic cancer or colon cancer.

The present invention provides use of a substance A or the pharmaceutical composition described above in preparation of a medicament for treatment and/or prevention of polymerase theta-related diseases, wherein said substance A is the compound of Formula I, the pharmaceutically acceptable salt thereof or the isotopic compound thereof as described in any embodiment of the present invention; and said polymerase theta-related diseases comprise lung cancer, breast cancer, HR-deficient ovarian cancer, gastric cancer, prostate cancer, pancreatic cancer or colon cancer.

The present invention provides a substance A or the pharmaceutical composition described above as a medicament, wherein said substance A is the compound of Formula I, the pharmaceutically acceptable salt thereof or the isotopic compound thereof as described in any embodiment of the present invention.

The present invention provides a substance A or the pharmaceutical composition described above for use in treatment and/or prevention of lung cancer, breast cancer, HR-deficient ovarian cancer, gastric cancer, prostate cancer, pancreatic cancer or colon cancer, wherein said substance A is the compound of Formula I, the pharmaceutically acceptable salt thereof or the isotopic compound thereof as described in any embodiment of the present invention.

The present invention provides a method for treating diseases, comprising the step of: administering a (therapeutically effective amount of) substance A or the pharmaceutical composition described above to a subject, wherein said substance A is the compound of Formula I, the pharmaceutically acceptable salt thereof or the isotopic compound thereof as described in any embodiment of the present invention; and said disease is lung cancer, breast cancer, HR-deficient ovarian cancer, gastric cancer, prostate cancer, pancreatic cancer or colon cancer.

The present invention provides a compound of Formula II or a salt thereof: 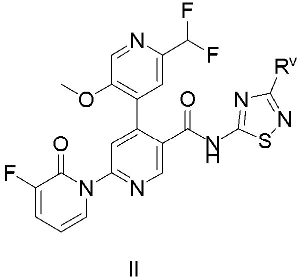 R^{v} is halogen.

In a preferred embodiment, said compound of Formula II is

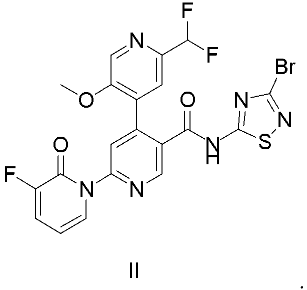

### Interpretation of Terms

Those skilled in the art will understand that, according to the conventions used in the art, the 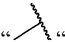 in the structural formula of the group described in the present invention indicates that the corresponding group is linked a further fragment or group in the compound via this site.

The term "a group B unsubstituted or substituted by one or more groups A" means that one or more hydrogen atoms in the group B are independently substituted by the group A, or B is not substituted. When a plurality of groups A appear at the same time, their definitions are independent of each other without mutual influence, unless otherwise specified. For example, "C₆₋₁₀ aryl substituted by 3 halogens" means that C₆₋₁₀ aryl is to be substituted by 3 halogens that have the definitions independent of each other without mutual influence, including but not limited to: 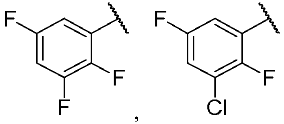 or the like.

The term "more" refers to 2 or more, for example, 2, 3, 4, or 5, preferably, 2 or 3.

In the claims of the present application, the term "more" in "satisfying one or more of the following conditions" refers to 2, 3, 4, or more, where the maximum value of "more" is the largest numerical value of the conditions specified in each claim. For example, if a claim lists eight conditions, the phrase "one or more" in "which satisfies one or more of the following conditions" in that claim refers to any integer from 1 to 8, for example, 1, 2, 3, 4, 5, 6, 7, or 8.

The term "pharmaceutically acceptable" means a relatively non-toxic and safe condition suitable for use in patients.

The term "pharmaceutically acceptable salt" means the salt resulting from the reaction of a compound with a pharmaceutically acceptable acid or alkali. When the compound contains relatively acidic functional groups, an alkali addition salt may be obtained by contacting the compound with a sufficient amount of a pharmaceutically acceptable alkali in a suitable inert solvent. The pharmaceutically acceptable alkali addition salt includes, but is not limited to: a sodium salt, a potassium salt, a calcium salt, an aluminum salt, a magnesium salt, a bismuth salt, an ammonium salt or the like. When the compound contains relatively alkaline functional groups, an acid addition salt may be obtained by contacting the compound with a sufficient amount of a pharmaceutically acceptable acid in a suitable inert solvent. The pharmacologically acceptable acid addition salt includes, but is not limited to: hydrochloride, sulfate, formate, mesylate or the like. For details, see Handbook of Pharmaceutical Salts: Properties, Selection, and Use (P. Heinrich Stahl, Camille G. Wermuth, 2011, 2nd Revised Edition).

The "-" in the group means the fact that the group is attached to the rest of the molecule via this site. For example, -NHC(O)R^{b} means that the N atom therein is attached to the rest of the molecule.

The term "halogen" means fluorine, chlorine, bromine or iodine. Halogen substitution in the present invention includes, but is not limited to, substitution by one, two, or three halogens, and in general, multiple substitutions occur on one carbon atom.

The term "oxo" means =O, indicating the substitution of two hydrogen on the same carbon atom by an oxygen atom. That is, a methylene group is substituted by a carbonyl group.

The term "alkyl" means a linear or branched saturated monovalent hydrocarbon group with a specified number of carbon atoms (for example, C₁₋₆). The alkyl groups include, but are not limited to: methyl, ethyl, n-propyl, isopropyl, n-butyl, isobutyl, sec-butyl, tert-butyl, n-amyl, n-hexyl or the like.

The term "alkoxy" means the group R^{X}-O-, and R^{X} is defined in the same way as the term "alkyl". The alkoxy groups include, but are not limited to: methoxy, ethoxy, n-propoxy, isopropoxy or the like.

The term "alkenyl" means a linear or branched unsaturated monovalent hydrocarbon group with a specified number of carbon atoms (for example, C₂₋₆), with one or more (for example, 1, 2, or 3) carbon-carbon sp² double bonds. The alkenyl groups include, but are not limited to: vinyl, 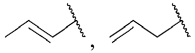 or the like. The term "alkynyl" means a linear or branched unsaturated monovalent hydrocarbon group with a specified number of carbon atoms (for example, C₂₋₆), with one or more (for example, 1, 2, or 3) carbon-carbon sp³ triple bonds. The alkynyl groups include, but are not limited to: acetylenyl, 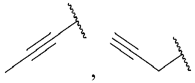 or the like. Unless otherwise specially specified in the present invention, the term "cycloalkyl" means a cyclic saturated monovalent hydrocarbon group with a specified number of carbon atoms (for example, 3-10 membered), which is monocyclic. The monocyclic rings include, but are not limited to: 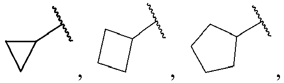 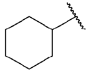 or the like.

Unless otherwise specially specified in the present invention, the term "aryl" means a cyclic unsaturated monovalent hydrocarbon group with a specified number of carbon atoms (for example, 6-12 membered), which is monocyclic or polycyclic (for example, 2 or 3 rings). In case of a polycyclic ring, monocyclic rings share two atoms and a bond, and is (at least one/each ring is) aromatic. The aryl group is attached to the rest of a molecule by a ring that is aromatic or not. The aryl groups include, but are not limited to: phenyl, naphthyl, 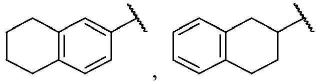 or the like.

Unless otherwise specially specified in the present invention, the term "cycloalkenyl" means a cyclic unsaturated monovalent hydrocarbon group with a specified number of carbon atoms (for example, 4-12 membered), which is monocyclic or polycyclic (for example, 2 or 3 rings, or a bridged, spiro or fused ring). The monocyclic-ring cycloalkenyl groups include, but are not limited to: 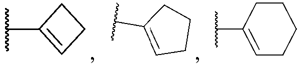 or the like. The bridged-ring cycloalkenyl groups include, but are not limited to: 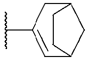 or the like. The spiro-ring cycloalkenyl groups include, but are not limited to, 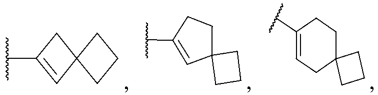 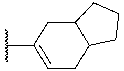 or the like. The fused-ring cycloalkenyl groups include, but are not limited to, 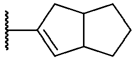 or the like.

Unless otherwise specially specified in the present invention, the term "heterocycloalkyl" means a monocyclic, bridged, spiro, or fused ring with a specified number (for example, 3-12 membered) of cyclic atoms, a specified number (for example, 1, 2 or 3) of heteroatoms, and a specified heteroatomic species (one or more of N, O and S). A monocyclic heterocycloalkyl group is attached to the rest of a molecule via a carbon atom or heteroatom. The monocyclic heterocycloalkyl groups include, but are not limited to: 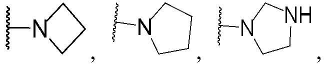 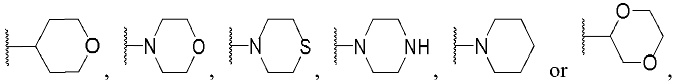 etc. The spiro heterocycloalkyl groups include, but are not limited to: 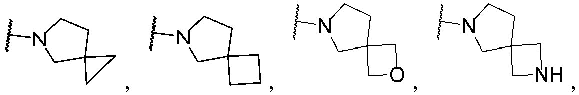 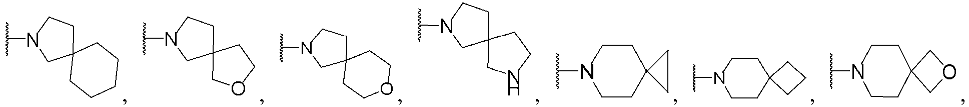 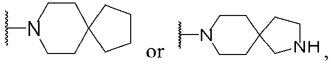 etc. The bridged-ring hetercycloalkyl groups include, but are not limited to: 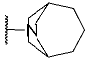 or the like. The fused heterocycloalkyl groups include, but are not limited to 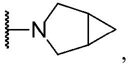 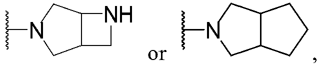 etc.

Unless otherwise specially specified in the present invention, the term "heterocycloalkenyl" means a cyclic unsaturated monovalent group with a specified number (for example, 4-12 membered) of cyclic atoms, a specified number (for example, 1, 2 or 3) of heteroatoms, and a specified heteroatomic species (one or more of N, O and S), and this group is monocyclic or polycyclic (for example, 2 or 3, a spiro-ring). The monocyclic-ring hetercycloalkenyl groups include, but are not limited to: 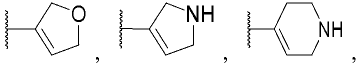 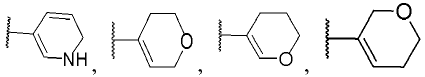 etc. The spiro-ring heterocycloalkenyl groups include, but are not limited to 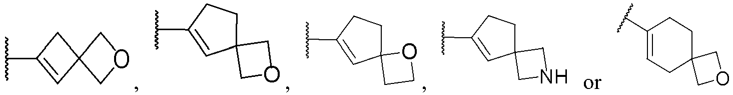 etc.

Unless otherwise specially specified in the present invention, the term "heteroaryl" means a cyclic unsaturated monovalent group with a specified number of cyclic atoms (for example, 5-12 membered), a specified number of heteroatom(s) (for example, 1, 2 or 3), and a specified heteroatom species (one or more of N, O and S), which are monocyclic or polycyclic, where the monocyclic rings share two atoms and a bond, and at least one ring is aromatic. The heteroaryl group is attached to the rest of a molecule by a carbon atom or heteroatom, by a ring with or without heteroatoms, or by a ring that is aromatic or not. The heteroaryl groups include, but are not limited to: 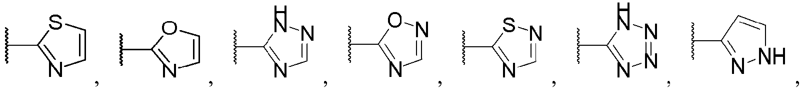 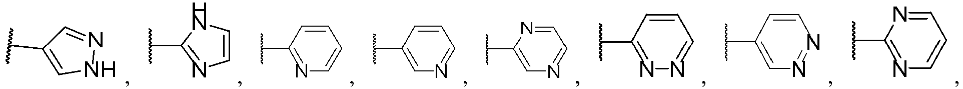 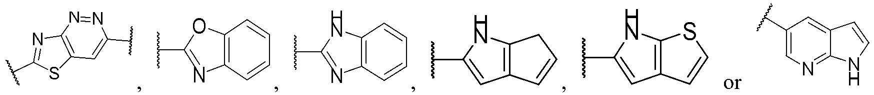 etc.

The term "isotopic compound" means a compound in which one or more atoms have the isotopic abundance that differ from their natural abundance. For example, one or more atoms in a compound are substituted by an atom with a lower mass in nature, for instance, one hydrogen atom in a compound is substituted by deuterium, or C is substituted by ¹³C.

The term "pharmaceutical excipient" refers to a vehicle or additive used in the production of drugs and the formulation of prescriptions, encompassing all substances included in a pharmaceutical preparation other than the active ingredients. A reference can be made to Part IV in the Pharmacopoeia of the People's Republic of China (2015 Edition), or Handbook of Pharmaceutical Excipients (Raymond C Rowe, 2009 Sixth Edition).

The term "treatment" refers to a therapeutic therapy. When a specific disease or condition is involved, treatment means: (1) alleviating one or more biological manifestations of the disease or condition, (2) interfering with (a) one or more points in the biological cascade that causes or contributes to the condition or (b) one or more biological manifestations of the condition, (3) ameliorating one or more symptoms, effects, or side effects associated with the condition, or one or more symptoms, effects, or side effects related to the condition or its treatment, or (4) slowing down the progression of the condition or one or more biological manifestations of the condition.

The term "prevention" refers to the reduction in the risk of acquiring or developing a disease or disorder.

The term "therapeutically effective amount" refers to an amount of the compound that is sufficient to effectively treat the disease or condition described herein when administered to a patient. The "therapeutically effective amount" varies depending on the compound, the condition and its severity, and the age of the patient to be treated, but can be adjusted as needed by one skilled in the art.

The term "patient" refers to any animal that is about to receive or has received administration of the compound or composition according to embodiments of the present invention, preferably a mammal, and most preferably a human. The term "mammal" includes any mammal. Examples of mammals include, but are not limited to, cattle, horses, sheep, pigs, cats, dogs, mice, rats, rabbits, guinea pigs, monkeys, humans, etc., with humans being the most preferred.

In the present invention, "room temperature" means "20-40°C".

Without departing from the common knowledge in the art, all the preferred conditions above can be randomly combined to implement the preferred embodiments of the present invention.

The reagents and raw materials used in the present invention are commercially available.

The positive progressive effect of the present invention lies in that: the ATPase activity and protein inhibition effect of the compound of the present invention are greatly improved as compared to the prior art.

### DETAILED DESCRIPTION OF THE PREFERRED EMBODIMENTS

The present invention will be further illustrated below by the way of embodiments, which are not intended to limit the present invention. For the test methods without specific conditions indicated in the following embodiments, the specific conditions were selected according to conventional methods and conditions, or according to the product instructions.

All the compounds of the present invention may be synthesized with different methods by a person skilled in the field of organic chemistry. The following describes general synthesis schemes for preparing the compounds of the present invention. These schemes are generic, which, however, does not imply the limitation to the possible techniques used by a person skilled in the art to prepare the compounds disclosed herein. The different methods for preparing the compounds of the present invention are obvious to a person skilled in the art. In addition, the various steps in synthesis may be performed alternately in sequence to obtain one or more of the desired compounds. Examples of the preparation of the compounds of the present invention by the method described in the general schemes are given in the Preparation and Examples sections described below. The preparation of compounds containing chiral center embodiments may be carried out by means of the techniques mastered by a person skilled in the art. For example, chiral compounds may be prepared by separating racemic products by chiral resolution by means of HPLC; or, example compounds may be prepared by known methods to obtain chiral compounds.

The chemical reactions and synthesis techniques described herein are performed in the reagents and solvents described herein, and the corresponding reaction yields are also affected by the reagents and solvents used. In addition, it should be understood that in the synthesis method described below, all the reaction conditions mentioned, including the choice of solvent, the reaction atmosphere, the reaction temperature, the experimental duration, and the feeding sequence for reaction, should be regarded as the standard operating conditions for the reaction, which should be easily identifiable by a person skilled in the art. Meanwhile, it is also understandable by a technician in the field of organic synthesis. The functional groups present on each part of a molecule must be compatible with the reagents used and the reaction itself. In case of the limitation of the incompatibility of some functional groups on each part of the molecule with the reaction conditions, an alternative method must be used, which is obvious to a person skilled in the art. To derive the desired compounds of the present invention, obviously, it is necessary to make judgment on the adjustment of the sequence of synthesis steps, or on the selection of a specific synthesis process scheme. This is understandable and easily recognizable to a person skilled in the field of organic synthesis. It should also be appreciated that another major consideration for any synthetic route designed in the art is the rational selection of a protective group to protect the tolerance of reactive functional groups present in the compounds described in the present invention. For details, a reference can be made to the work of Greene et al., authorities in the field of chemistry (Protective Groups in Organic Synthesis, Third Edition, Wiley and Sons (1999)).

### Examples

The compounds and the intermediates used in the preparation of the compounds may be prepared using the procedures shown in the following examples and related procedures. The methods and conditions used in these examples and the actual compounds prepared in these examples are not meant to be limited, but imply to elaborate how to prepare the relevant compounds. The starting materials and reagents used in these examples, when not prepared by the procedures described herein, are often commercially available, or have been reported in the relevant chemical literature, or may be prepared by using the procedures described in the chemical literature.

In the examples given herein, the term "drying and concentrating" generally means the addition of anhydrous sodium sulfate or magnesium sulfate drying solution to an organic solvent, followed by filtration and removal of the solvent from the filtrate (usually under reduced pressure and at a temperature suitable for the stability of the compound being prepared). In case of chromatographic column methods, conventional column chromatography or rapid column chromatography is typically used for column separation and purification, or a medium-pressure chromatograph (Biotage Isola One) preloaded with silica-gel column is used for elution in a specified solvent or solvent mixture. In some cases, the final product is rapidly purified by preparative thin-layer chromatography (TLC) using silica gel plates of 20 cm x 20 cm x 0.5 mm or 20 cm x 20 cm x 1 mm in an appropriate solvent system. Preparative high-performance liquid chromatography (HPLC) is performed using a reversed-phase column (Waters Sunfire C18, Waters Xbridge C18, or a similar reversed-phase column) with a size appropriate for the amount of compound being separated; elution is typically performed by adding methanol or acetonitrile of gradient concentrations to an aqueous phase, with an eluent containing 0.05% or 0.1% formic acid, trifluoroacetic acid, or 10 mM ammonium acetate, at an elution rate matching the size of the reversed-phase column used and the resolution of the product to be prepared.

### List of Abbreviations

**Table 1**

| **Abbreviation** | **Explanation** | **Abbreviation** | **Explanation** |
|---|---|---|---|
| MS | Mass spectrometry | NMR | Nuclear magnetic resonance |
| TLC | Thin-layer | HPLC | High performance liquid |
| | chromatography | | chromatography |
| LCMS | Liquid chromatography-mass spectrometry | °C | Degree centigrade |
| Pd(dtbpf)Cl₂ | (1,1'-Bis (di-t-butylphosphino)ferrocene) palladium dichloride | K₃PO₄ | Potassium phosphate |
| Quinolin-8-ol | 8-Hydroxyquinoline | K₂CO₃ | Potassium carbonate |
| Cul | Cuprous iodide | TCFH | N,N,N',N'-tetramethylchloroformam idinium hexafluorophosphate |
| NMI | N-methylimidazole | ACN | Acetonitrile |

### Example 1: N-(3-cyclopropyl-1,2,4-thiadiazol-5-yl)-2"-difluoromethyl-3-fluoro-5"-methoxy-2-oxo-2H-[1,2':4',4"-tripyridin]-5'-carboxamide

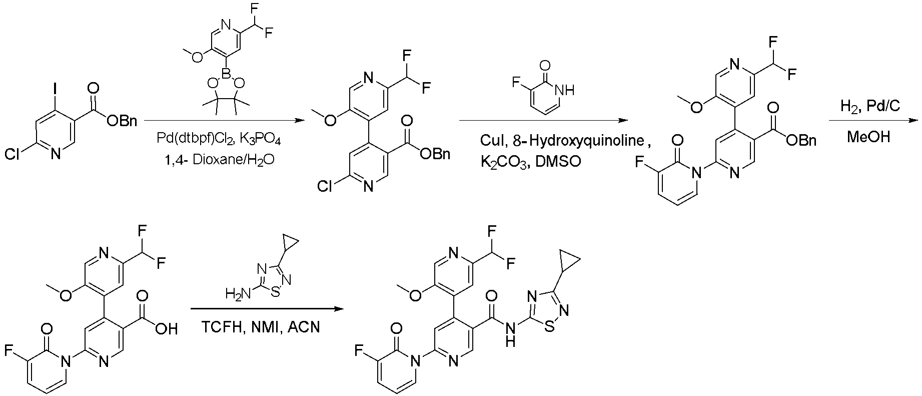

### Step 1: Benzyl 6-chloro-2'-difluoromethyl-5'-methoxy-4,4'-bipyridyl-3-carboxylate

At room temperature under the protection of nitrogen, 2-difluoromethyl-5-methoxy-4-pyridine borate (4.58 g, 16.08 mmol), potassium phosphate (8.53 g, 40.21 mmol) and (1,1'-bis (di-t-butylphosphino)ferrocene)palladium dichloride (865 mg, 0.294 mmol) was added to the mixed solution of benzyl 6-chloro-4-iodine niacinate (5.0 g, 13.40 mmol) in 1,4-dioxane (50 mL) and water (10 mL) sequentially. Under the protection of nitrogen, the reaction mixture was stirred at 90°C for 1.5 hours. After complete reaction, water was added; the resulting mixture was extracted with ethyl acetate; the organic phase was washed with saturated saline, dried over anhydrous sodium sulfate, and concentrated under reduced pressure. The residues was purified by column chromatography (eluent: petroleum ether/ethyl acetate, gradient: 0-30% ethyl acetate) to obtain the title compound (white solid, 3.06 g, yield: 56.5%). LC/MS (ESI) m/z: 405.1 [M+H]⁺.

### Step 2: Benzyl 2"-difluoromethyl-3-fluoro-5"-methoxy-2-oxo-2H-[1,2':4',4"-tripyridin]-5'-carboxylate

At room temperature under the protection of nitrogen, benzyl 6-chloro-2'-difluoromethyl-5'-methoxy-4,4'-bipyridyl-3-carboxylate (3.0 g, 7.42 mmol), 3-fluoropyridin-2(1H)one (1.26 g, 11.15 mmol), cuprous iodide (707 mg, 3.71 mmol), 8-hydroxyquinoline (108 mg, 0.74 mmol) and potassium carbonate (2.05 g, 14.85 mmol) were dissolved in dimethylsulfoxide (30 mL), and the reaction mixture was subjected to nitrogen displacement three times and stirred at 100°C for 1 h under the protection of nitrogen. After complete reaction, water was added to the reaction mixture which was then extracted with ethyl acetate three time, the organic phases was washed with saturated brine, dried over anhydrous sodium sulfate and concentrated under reduced pressure, and the residues was purified by the column chromatography (eluent: petroleum ether/ethyl acetate, gradient: 30%-70% ethyl acetate) to obtain the title compound (white solid, 1.41 g, yield: 39.5%). LC/MS (ESI) m/z: 482.2 [M+H]⁺.

### Step 3: 2"-Difluoromethyl-3-fluoro-5"-methoxy-2-oxo-2H-[1,2':4',4"-tripyridin]-5'-carboxylic acid

At room temperature under the protection of nitrogen, 10% palladium on carbon (300 mg) was added to the solution of benzyl 2"-difluoromethyl-3-fluoro-5"-methoxy-2-oxo-2H-[1,2':4',4"-tripyridin]-5'-carboxylate(1.4 g, 2.91 mmol) in methanol (14 mL), and the mixture was subjected to nitrogen displacement three times, and stirred at room temperature to react for 3 h in an atmosphere of hydrogen under one atmospheric pressure. After complete reaction, the reaction mixture was filtered, and the filtrate was concentrated under reduced pressure to obtain the title compound (brown solid, 1.01 g, yield: 88.7%). LC/MS (ESI) m/z: 392.1 [M+H]⁺.

### Step 4: N-(3-cyclopropyl-1,2,4-thiadiazol-5-yl)-2"-difluoromethyl-3-fluoro-5"-methoxy-2-oxo-2H-[1,2':4',4"-tripyridin]-5'-carboxamide

At room temperature, 2"-difluoromethyl-3-fluoro-5"-methoxy-2-oxo-2H-[1,2':4',4"-tripyridin]-5'-carboxylic acid (20 mg, 0.051 mmol), 3-cyclopropyl-1,2,4-thiadiazol-5-amine (10.8 mg, 0.077 mmol) and 1-methylimidazole (16.80 mg, 0.21 mmol) was dissolved in acetonitrile (2 mL), and the mixture was stirred at 75°C for 5 min. Then, N,N,N',N'-tetramethylchloroformamidinium hexafluorophosphate (21.5 mg, 0.077 mmol) was added, and the mixture was further stirred at 75°C to react for 1 h. After complete reaction, the reaction mixture was concentrated under reduced pressure, and the residues was subjected to the preparative reverse phase liquid chromatography to obtain the title compound (white solid, 6 mg, yield: 22.8%).

¹H NMR (400 MHz, DMSO-*d*₆) δ 13.85 - 13.53 (m, 1H), 9.03 (s, 1H), 8.50 (s, 1H), 8.06 (s, 1H), 7.86 (d, *J* = 7.1 Hz, 1H), 7.73 (s, 1H), 7.62 - 7.48 (m, 1H), 7.00 (t, *J* = 55.1 Hz, 1H), 6.47 - 6.36 (m, 1H), 3.66 (s, 3H), 2.25 - 2.13 (m, 1H), 1.06 - 0.99 (m, 2H), 0.99 - 0.93 (m, 2H). LC/MS (ESI) (m/z)*:* 515 [M+H]⁺.

The example compounds in Table 2 was synthesized using commercially available raw materials with reference to the synthesis steps of Example 1.

**Table 2:**

| Examples | Structure and name | Analysis data |
|---|---|---|
| 2 | 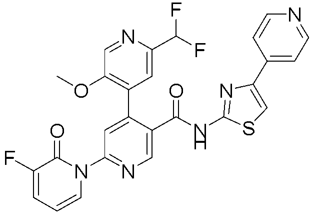 2"-(difluoromethyl)-3-fluoro-5"-methoxy-2-oxo-N-(4-(pyridin-4-yl)thiazol-2-yl)-2H-[1,2':4',4"-terpyridine]-5'-carboxamide | ¹H NMR (400 MHz, DMSO-d₆) δ 13.25 (s, 1H), 9.02 (s, 1H), 8.66 (d, J = 6.1 Hz, 2H), 8.52 (s, 1H), 8.13 (s, 1H), 8.07 (s, 1H), 7.92 (d, J = 6.0 Hz, 2H), 7.87 (d, J = 7.1 Hz, 1H), 7.75 (s, 1H), 7.61 - 7.51 (m, 1H), 7.00 (t, J = 55.1 Hz, 1H), 6.45 - 6.40 (m, 1H), 3.71 (s, 3H). LC/MS (ESI) (m/z): 551 [M+H]⁺. |
| 3 | 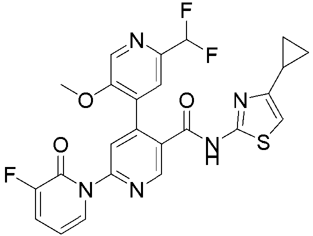 N-(4-cyclopropylthiazol-2-yl)-2"-(difluoromethyl)-3-fluoro-5"-methoxy-2-oxo-2H-[1,2':4',4"-terpyridine]-5'-carboxamide | ¹H NMR (400 MHz, DMSO-d₆) δ 12.87 (s, 1H), 8.94 (s, 1H), 8.50 (s, 1H), 8.03 (s, 1H), 7.85 (d, J = 7.1 Hz, 1H), 7.71 (s, 1H), 7.60 - 7.52 (m, 1H), 6.99 (t, J = 55.1 Hz, 1H), 6.85 (s, 1H), 6.42 (td, J = 7.3, 4.7 Hz, 1H), 3.69 (s, 3H), 2.06 - 1.98 (m, 1H), 0.92 - 0.85 (m, 2H), 0.81 - 0.75 (m, 2H). LC/MS (ESI) (m/z): 514 [M+H]⁺. |
| 4 | 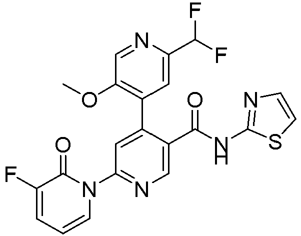 2"-(difluoromethyl)-3-fluoro-5"-methoxy-2-oxo-N-(thiazol-2-yl)-2H-[1,2':4',4"-terpyridine]-5'-carboxamide | ¹H NMR (400 MHz, DMSO-d₆) δ 12.93 (s, 1H), 8.97 (s, 1H), 8.50 (s, 1H), 8.05 (s, 1H), 7.86 (d, J = 7.0 Hz, 1H), 7.73 (s, 1H), 7.55 (d, J = 3.8 Hz, 2H), 7.29 (d, J = 3.3 Hz, 1H), 7.00 (t, J = 55.0 Hz, 1H), 6.45 - 6.39 (m, 1H), 3.68 (s, 3H). LC/MS (ESI) (m/z): 474 [M+H]⁺. |
| 5 | 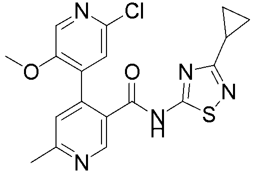 2'-chloro-N-(3-cyclopropyl-1,2,4-thiadiazol-5-yl)-5'-methoxy-6-methyl-[4,4'-bipyridine]-3-carboxamide | ¹H NMR (400 MHz, DMSO- d₆) δ 8.86 (s, 1H), 8.15 (s, 1H), 7.54 (s, 1H), 7.42 (s, 1H), 3.55 (s, 3H), 2.58 (s, 3H), 2.22 - 2.11 (m, 1H), 1.02 (dd, J = 13.2, 6.7 Hz, 2H), 0.93 (dd, J = 12.2, 5.1 Hz, 2H). LC/MS (ESI) (m/z): 402 [M+H]⁺. |
| 6 | 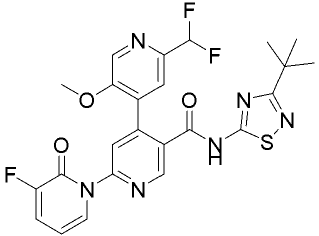 N-(3-(tert-butyl)-1,2,4-thiadiazol-5-yl)-2"-(difluoromethyl)-3-fluoro-5"-methoxy-2-oxo-2H-[1,2':4',4"-terpyridine]-5'-carboxamide | ¹H NMR (400 MHz, DMSO-d₆) δ 13.80 (s, 1H), 9.06 (s, 1H), 8.51 (s, 1H), 8.08 (s, 1H), 7.87 (d, J = 7.2 Hz, 1H), 7.74 (s, 1H), 7.61 - 7.52 (m, 1H), 7.00 (t , J = 55.0 Hz, 1H), 6.47 - 6.37 (m, 1H), 3.66 (s, 3H), 1.37 (s, 9H). LC/MS (ESI) (m/z): 531 [M+H]⁺. |
| 7 | 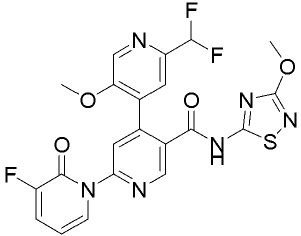 2"-(difluoromethyl)-3-fluoro-5"-methoxy-N-(3-methoxy-1,2,4-thiadiazol-5-yl)-2-oxo-2H-[1,2':4',4"-terpyridine]-5'-carboxamide | ¹H NMR (400 MHz, DMSO-d₆) δ 13.78 (s, 1H), 9.03 (s, 1H), 8.52 (s, 1H), 8.10 (s, 1H), 7.86 (d, J = 7.2 Hz, 1H), 7.76 (s, 1H), 7.60 - 7.53 (m, 1H), 7.01 (t, J = 55.1 Hz, 1H), 6.43 (td, J = 7.3, 4.7 Hz, 1H), 3.96 (s, 3H), 3.69 (s, 3H). LC/MS (ESI) (m/z): 505 [M+H]⁺. |
| 8 | 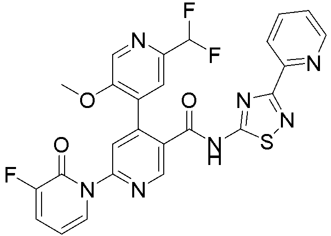 2"-(difluoromethyl)-3-fluoro-5"-methoxy-2-oxo-N-(3-(pyridin-2-yl)-1,2,4-thiadiazol-5-yl)-2H-[1,2':4',4"-terpyridine]-5'-carboxamide | ¹H NMR (400 MHz, DMSO-d₆) δ 14.06 (s, 1H), 9.09 (s, 1H), 8.73 (d, J = 4.2 Hz, 1H), 8.52 (s, 1H), 8.24 (d, J = 7.9 Hz, 1H), 8.13 (s, 1H), 8.04 - 7.96 (m, 1H), 7.88 (d, J = 7.1 Hz, 1H), 7.79 (s, 1H), 7.62 - 7.47 (m, 2H), 7.02 (t, J = 55.1 Hz, 1H), 6.50 - 6.38 (m, 1H), 3.69 (s, 3H). LC/MS (ESI) (m/z): 552 [M+H]⁺. |
| 9 | 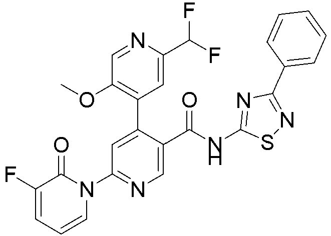 2"-(difluoromethyl)-3-fluoro-5"-methoxy-2-oxo-N-(3-phenyl-1,2,4-thiadiazol-5-yl)-2H-[1,2':4',4"-terpyridine]-5'-carboxamide | ¹H NMR (400 MHz, DMSO- d₆) δ 9.14 (s, 1H), 8.50 (s, 1H), 8.19 (d, J = 6.4 Hz, 2H), 8.01 (s, 1H), 7.89 (d, J = 7.1 Hz, 1H), 7.71 (s, 1H), 7.61 - 7.42 (m, 4H), 7.00 (t, J = 55.2 Hz, 1H), 6.44 - 6.40 (m,1H), 3.68 (s, 3H). LC/MS (ESI) (m/z): 551 [M+H]⁺. |
| 10 | 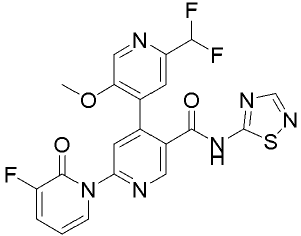 2"-(difluoromethyl)-3-fluoro-5"-methoxy-2-oxo-N-(1,2,4-thiadiazol-5-yl)-2H-[1,2':4',4''-terpyridine]-5'-carboxamide | ¹H NMR (400 MHz, DMSO-d₆) δ 13.83 (s, 1H), 9.05 (s, 1H), 8.57 (s, 1H), 8.50 (s, 1H), 8.11 (s, 1H), 7.87 (d, J = 7.1 Hz, 1H), 7.77 (s, 1H), 7.60 - 7.54 (m, 1H), 7.01 (t, J = 55.0 Hz, 1H), 6.47 - 6.40 (m, 1H), 3.64 (s, 3H). LC/MS (ESI) (m/z): 475 [M+H]⁺. |
| 11 | 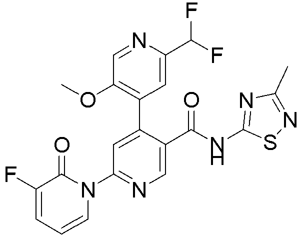 2"-(difluoromethyl)-3-fluoro-5"-methoxy-N-(3-methyl-1,2,4-thiadiazol-5-yl)-2-oxo-2H-[1,2':4',4"-terpyridine]-5'-carboxamide | ¹H NMR (400 MHz, DMSO-d₆) δ 13.67 (s, 1H), 9.03 (s, 1H), 8.51 (s, 1H), 8.10 (s, 1H), 7.87 (d, J = 7.1 Hz, 1H), 7.76 (s, 1H), 7.61 - 7.54 (m, 1H), 7.01 (t, J = 55.0 Hz, 1H), 6.47 - 6.39 (m, 1H), 3.66 (s, 3H), 2.54 (s, 3H). LC/MS (ESI) (m/z): 489 [M+H]⁺. |
| 12 | 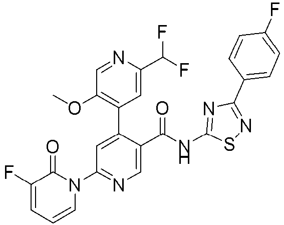 2"-(difluoromethyl)-3-fluoro-N-(3-(4-fluorophenyl)-1,2,4-thiadiazol-5-yl)-5"-methoxy-2-oxo-2H-[1,2':4',4"-terpyridine]-5'-carboxamide | ¹H NMR (400 MHz, DMSO-d₆) δ 13.99 (s, 1H), 9.09 (s, 1H), 8.52 (s, 1H), 8.24 (dd, J = 8.7, 5.6 Hz, 2H), 8.11 (s, 1H), 7.88 (d, J = 7.1 Hz, 1H), 7.78 (s, 1H), 7.64 - 7.52 (m, 1H), 7.38 (t, J = 8.8 Hz, 2H), 7.02 (t, J = 55.1 Hz, 1H), 6.46 - 6.41 (m, 1H), 3.68 (s, 3H). LC/MS (ESI) (m/z): 569 [M+H]⁺. |
| 13 | 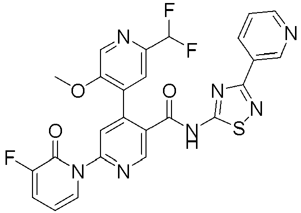 2"-(difluoromethyl)-3-fluoro-5"-methoxy-2-oxo-N-(3-(pyridin-3-yl)-1,2,4-thiadiazol-5-yl)-2H-[1,2':4',4"-terpyridine]-5'-carboxamide | ¹H NMR (400 MHz, DMSO-d₆) δ 9.34 (s, 1H), 9.13 (s, 1H), 8.68 (s, 1H), 8.50 - 8.48 (m, 2H), 8.05 (s, 1H), 7.88 (d, J = 7.1 Hz, 1H), 7.74 (s, 1H), 7.56 (t, J = 7.9 Hz, 2H), 7.01 (t, J = 55.1 Hz, 1H), 6.45 - 6.40 (m, 1H), 3.68 (s, 3H). LC/MS (ESI) (m/z): 552 [M+H]⁺. |
| 14 | 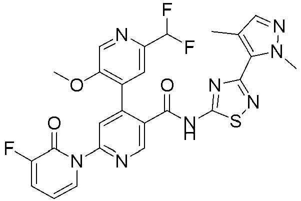 2"-(difluoromethyl)-N-(3-(1,4-dimethyl-1H-pyrazol-5-yl)-1,2,4-thiadiazol-5-yl)-3-fluoro-5"-methoxy-2-oxo-2H-[1,2':4',4"-terpyridine]-5'-carboxamide | ¹H NMR (400 MHz, DMSO-d₆) δ 13.99 (s, 1H), 9.10 (s, 1H), 8.52 (s, 1H), 8.12 (s, 1H), 7.88 (d, J = 7.2 Hz, 1H), 7.78 (s, 1H), 7.62 - 7.54 (m, 1H), 7.40 (s, 1H), 7.01 (t, J = 55.0 Hz, 1H), 6.46 - 6.41 (m, 1H), 4.15 (s, 3H), 3.68 (s, 3H), 2.32 (s, 3H). LC/MS (ESI) (m/z): 569 [M+H]⁺. |
| 15 | 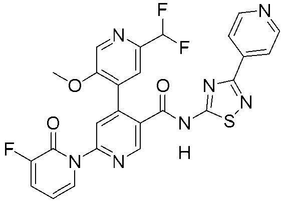 2"-(difluoromethyl)-3-fluoro-5"-methoxy-2-oxo-N-(3-(pyridin-4-yl)-1,2,4-thiadiazol-5-yl)-2H-[1,2':4',4"-terpyridine]-5'-carboxamide | ¹H NMR (400 MHz, CD₃OD) δ 9.05 (s, 1H), 8.69 - 8.68 (m, 2H), 8.40 (s, 1H), 8.23 -8.22 (m, 2H), 8.11 (s, 1H), 7.94 - 7.92 (m, 1H), 7.84 (s, 1H), 7.51 - 7.44 (m, 1H), 6.77 (t, J = 55.4 Hz, 1H), 6.53 - 6.46 (m, 1H), 3.76 (s, 3H). LC/MS (ESI) (m/z): 552 [M+H]⁺. |
| 16 | 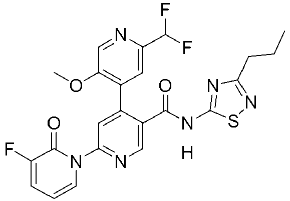 2"-(difluoromethyl)-3-fluoro-5"-methoxy-2-oxo-N-(3-propyl-1,2,4-thiadiazol-5-yl)-2H-[1,2':4',4"-terpyridine]-5'-carboxamide | ¹H NMR (400 MHz, DMSO-d₆) δ 9.00 (s, 1H), 8.39 (s, 1H), 8.11 - 8.09 (m, 1H), 7.93 - 7.89 (m, 1H), 7.82 (s, 1H), 7.50 - 7.44 (m, 1H), 6.76 (t, J = 55.3 Hz, 1H), 6.52 - 6.46 (m, 1H), 3.74 (s, 3H), 2.82 (t, J = 7.4 Hz, 2H), 1.86 - 1.79 (m, 2H), 0.97 (t, J = 7.4 Hz, 3H). LC/MS (ESI) (m/z): 517 [M+H]⁺. |
| 17 | 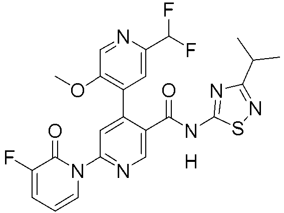 2"-(difluoromethyl)-3-fluoro-N-(3-isopropyl-1,2,4-thiadiazol-5-yl)-5"-methoxy-2-oxo-2H-[1,2':4',4"-terpyridine]-5'-carboxamide | ¹H NMR (400 MHz, DMSO-d₆) δ 13.75 (s, 1H), 9.04 (s, 1H), 8.51 (s, 1H), 8.09 (s, 1H), 7.87 (d, J = 7.1 Hz, 1H), 7.75 (s, 1H), 7.60 - 7.53 (m, 1H), 7.15 - 6.86 (m, 1H), 6.45 - 6.39 (m, 1H), 3.66 (s, 3H), 3.18 - 3.10 (m, 1H), 1.33 - 1.28 (m, 6H). LC/MS (ESI) (m/z): 517 [M+H]⁺. |
| 18 | 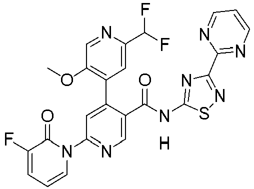 2"-(difluoromethyl)-3-fluoro-5"-methoxy-2-oxo-N-(3-(pyrimidin-2-yl)-1,2,4-thiadiazol-5-yl)-2H-[1,2':4',4"-terpyridine]-5'-carboxamide | ¹H NMR (400 MHz, DMSO-d₆) δ 14.11 (s, 1H), 9.10 (s, 1H), 8.99 (d, J = 4.8 Hz, 2H), 8.52 (s, 1H), 8.11 (s, 1H), 7.89 (d, J = 7.1 Hz, 1H), 7.78 (s, 1H), 7.66 - 7.61 (m, 1H), 7.60 - 7.54 (m, 1H), 7.02 (t, J = 55.1 Hz, 1H), 6.54 - 6.31 (m, 1H), 3.69 (s, 3H). LC/MS (ESI) (m/z): 553 [M+H]⁺. |
| 19 | 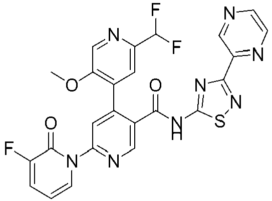 2"-(difluoromethyl)-3-fluoro-5"-methoxy-2-oxo-N-(3-(pyrazin-2-yl)-1,2,4-thiadiazol-5-yl)-2H-[1,2':4',4"-terpyridine]-5'-carboxamide | ¹H NMR (400 MHz, DMSO-d₆) δ 9.39 (s, 1H), 9.17 (s, 1H), 8.76 (s, 1H), 8.72 (s, 1H), 8.49 (s, 1H), 7.96 (s, 1H), 7.90 - 7.88 (m, 1H), 7.68 (s, 1H), 7.59 - 7.51 (m, 1H), 6.99 (t, J = 55.2, 1H), 6.44 - 6.39 (m, 1H), 3.68 (s, 3H). LC/MS (ESI) (m/z): 553 [M+H]⁺. |
| 20 | 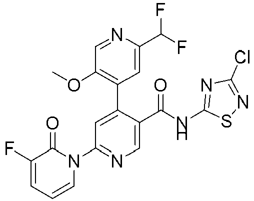 N-(3-chloro-1,2,4-thiadiazol-5-yl)-2"-(difluoromethyl)-3-fluoro-5"-methoxy-2-oxo-2H-[1,2':4',4"-terpyridine]-5'-carboxamide | ¹H NMR (400 MHz, DMSO-d₆) δ 14.18 (s, 1H), 9.06 (s, 1H), 8.52 (s, 1H), 8.11 (s, 1H), 7.87 (d, J = 7.2 Hz, 1H), 7.76 (s, 1H), 7.60 -7.54 (m, 1H), 7.01 (t, J = 55.0 Hz, 1H), 6.44 - 6.40 (m, 1H), 3.69 (s, 3H). LC/MS (ESI) (m/z): 509 [M+H]⁺. |
| 21 | 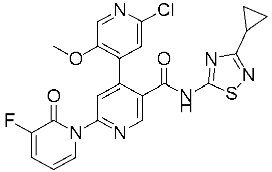 2"-chloro-N-(3-cyclopropyl-1,2,4-thiadiazol-5-yl)-3-fluoro-5"-methoxy-2-oxo-2H-[1,2':4',4"-terpyridine]-5'-carboxamide | ¹H NMR (400 MHz, DMSO-d₆) δ 13.68 (s, 1H), 9.02 (s, 1H), 8.22 (s, 1H), 8.08 (s, 1H), 7.87 - 7.85 (m, 1H), 7.61 - 7.52 (m, 2H), 6.45 - 6.40 (m, 1H), 3.59 (s, 3H), 2.23 - 2.18 (m, 1H), 1.07 - 0.92 (m, 4H). LC/MS (ESI) (m/z): 499 [M+H]⁺. |
| 22 | 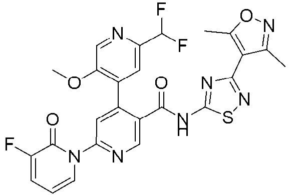 2"-(difluoromethyl)-N-(3-(3,5-dimethylisoxazol-4-yl)-1,2,4-thiadiazol-5-yl)-3-fluoro-5"-methoxy-2-oxo-2H-[1,2':4',4"-terpyridine]-5'-carboxamide | ¹H NMR (400 MHz, DMSO-d₆) δ 13.93 (s, 1H), 9.11 (s, 1H), 8.51 (s, 1H), 8.08 (s, 1H), 7.88 (d, J = 7.1 Hz, 1H), 7.75 (s, 1H), 7.57 (s, 1H), 7.07 (d, J = 55.1 Hz, 1H), 6.43 (d, J = 4.7 Hz, 1H), 3.67 (s, 3H), 2.78 (s, 3H), 2.53 (s, 3H). LC/MS (ESI) (m/z): 570 [M+H]⁺. |

### Example 23: N-(3-(4-chlorophenyl)-1,2,4-thiadiazol-5-yl)-2"-difluoromethyl-3-fluoro-5"-methoxy-2-oxo-2H-[1,2': 4',4"-tripyridinl-5'-carboxamide

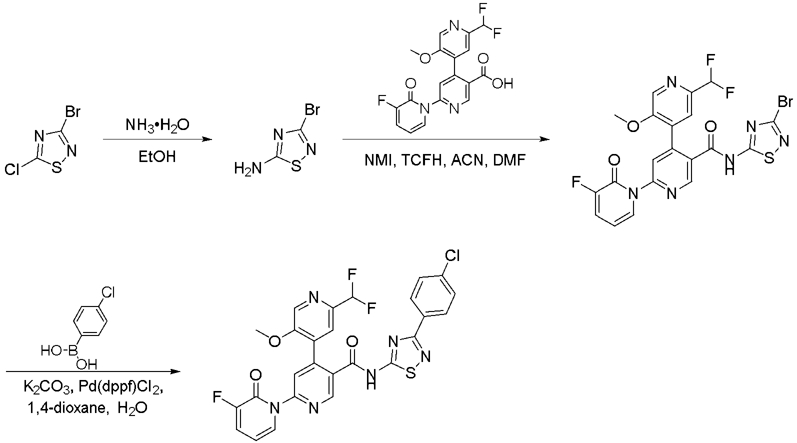

### Step 1: 3-Bromo-1,2,4-thiadiazol-5-amine

Under the protection of nitrogen, 3-bromo-5-chloro-1,2,4-thiadiazole (500 mg, 2.51 mmol) was dissolved in absolute ethanol (5 mL), followed by the slow dropwise addition of ammonia water (0.34 mL, 5.01 mmol, 25% w/w), and the mixture was then stirred at 70°C to react for 3 h. The reaction mixture was cooled to room temperature, and a saturated aqueous sodium bicarbonate solution (10 mL) was added, with the precipitation of white solids. The reaction mixture was filtered, and the filter cake was concentrated under reduced pressure to obtain the title compound (white solid, 220 mg, yield: 48.8%). LC/MS (ESI) m/z: 179.9 [M+H]⁺.

### Step 2: N-(3-bromo-1,2,4-thiadiazol-5-yl)-2"-difluoromethyl-3-fluoro-5"-methoxy-2-oxo-2H-[1,2': 4',4"-tripyridinl-5'-carboxamide

At room temperature, 2"-difluoromethyl-3-fluoro-5"-methoxy-2-oxo-2H-[1,2': 4',4"-tripyridin]-5'-carboxylic acid (200 mg, 0.511 mmol), 3-bromo-1,2,4-thiadiazol-5-amine (92.01 mg, 0.511 mmol), N-methylimidazole (168 mg, 2.04 mmol) was dissolved in acetonitrile (3 mL) and N,N-dimethylcarboxamide (1 mL), and then stirred at 70°C for 5 min, N,N,N',N'-tetramethylchloroformamidinium hexafluorophosphate (215 mg, 0.77 mmol) was added to the reaction mixture which subsequently changed from turbid to clear, and the reaction mixture was further stirred at 70°C to react for 1 h. LCMS showed complete reaction. Ethyl acetate and water was added to the reaction mixture, and the organic phase was separated, washed with saturated brine, dried over anhydrous sodium sulfate and filtered, the filtrate was concentrated under reduced pressure, and the residues was purified by the silica-gel column chromatography (eluent: petroleum ether/ethyl acetate, gradient: 0-55% ethyl acetate) to obtain the title compound (white solid, 160 mg, yield: 56%). LC/MS (ESI) m/z: 553.3 [M+H]⁺.

### Step 3: N-(3-(4-chlorophenyl )-1,2,4-thiadiazol-5-yl)-2"-difluoromethyl-3-fluoro-5"-methoxy-2-oxo-2H-[1,2': 4',4"-tripyridin]-5'-carboxamide

Under the protection of nitrogen, N-(3-bromo-1,2,4-thiadiazol-5-yl)-2"-difluoromethyl-3-fluoro-5"-methoxy-2-oxo-2H-[1,2':4',4"-tripyridin]-5'-carboxamide (20 mg, 0.036 mmol), 4-chlorobenzeneboronic acid (8.5 mg, 0.054 mmol) and potassium carbonate (15 mg, 0.108 mmol) was dissolved in 1,4-dioxane (3 mL) and water (0.6 mL), 1'-bis(diphenylphosphino)ferrocene)dichloropalladium(II) (5.3 mg, 0.007 mmol) was then added under the protection of nitrogen, and the mixture was stirred at 90°C to react for 16 h. The reaction mixture was cooled to room temperature, ethyl acetate and water was then added to separate an ethyl acetate phase, which was then washed with saturated brine, dried over anhydrous sodium sulfate and filtered, and the filtrate was concentrated under reduced pressure. The residues was subjected to the silica-gel column chromatography (eluent: dichloromethane /methanol, gradient: 0-3% methanol ) and the preparative reverse phase liquid chromatography to obtain the title compound (white solid, 1 mg, yield: 4.7%). ¹H NMR (400 MHz, DMSO-d₆) δ 14.01 (s, 1H), 9.12 (s, 1H), 8.50 (s, 1H), 8.19 (d, J = 8.6 Hz, 2H), 8.11 - 7.97 (m, 1H), 7.88 (d, J = 7.1 Hz, 1H), 7.73 (s, 1H), 7.60 - 7.54 (m, 3H), 7.00 (t, J = 55.1 Hz, 1H), 6.45 - 6.40 (m, 1H), 3.68 (s, 3H). ¹⁹F NMR (376 MHz, DMSO-d₆) δ -113.43 (s), -132.05 (s). LC/MS (ESI) m/z: 585.3 [M+H]⁺.

### Example 24: 2"-(difluoromethyl)-3-fluoro-5"-methoxy-N-(3-morpholin-1,2,4-thiadiazol-5-yl)-2-oxo-2H-[1,2':4',4"-terpyridinl-5'-carboxamide

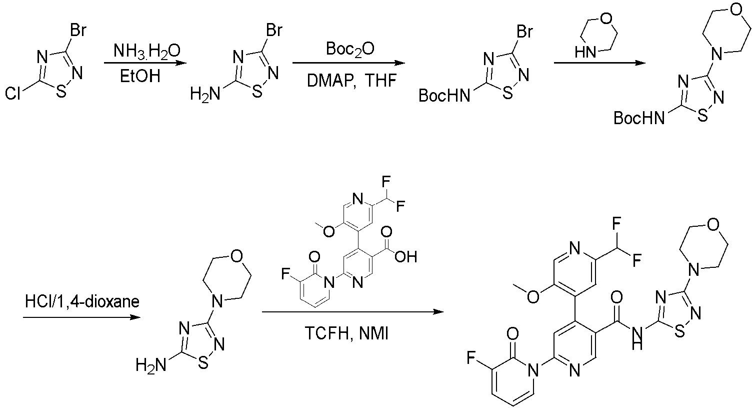

### Step 1: 3-Bromo-1,2,4-thiadiazol-5-amine

3-Bromo-5-chloro-1,2,4-thiadiazole (5.5 g, 27.6 mmol) was dissolved in ethanol (16 mL), followed by the addition of ammonia water (4 mL), and the reaction mixture was stirred at 70°C to react for 3 h. Saturated ammonium chloride was added to the mixture, which was then filtered, and the filter cake was dried under reduced pressure to obtain the title compound (white solid, 4 g, yield: 80.6%). LC/MS (ESI) m/z: 182.0 [M+H]⁺.

### Step 2: Tert-butyl (3-bromo-1,2,4-thiadiazol-5-vl)carbamate

3-Bromo-1,2,4-thiadiazol-5-amine (300 mg, 1.67 mmol) was dissolved in tetrahydrofuran (10 mL), followed by the addition of 4-dimethylaminopyridine (10 mg, 0.082 mmol) and di-tert-butyl dicarbonate (436 mg, 2.0 mmol), and the reaction mixture was stirred at 50°C for 3 h. The mixture was concentrated under reduced pressure; and the residues was purified by silica-gel column chromatography (eluent: petroleum ether/ethyl acetate, gradient: 0-20% ethyl acetate) to obtain the title compound (white solid, 430 mg, yield: 91.5%).

LC/MS (ESI) m/z: 280.0 [M+H]⁺.

### Step 3: Tert-butyl (3-morpholinyl-1,2,4-thiadiazol-5-yl)carbamate

Tert-butyl (3-bromo-1,2,4-thiadiazol-5-yl)carbamate (100 mg, 0.357 mmol) was dissolved in morpholine (1 mL), and the reaction mixture was stirred at 120°C for 3 h. The reaction mixture was diluted with ethyl acetate, washed with saturated brine, dried over anhydrous sodium sulfate, and concentrated under reduced pressure. The residues was purified by silica-gel column chromatography (eluent: petroleum ether/ethyl acetate, gradient: 20-30% ethyl acetate) to obtain the title compound (white solid, 80 mg, yield: 78.3%). LC/MS (ESI) m/z: 287.2 [M+H]⁺.

### Step 4: 3-Morpholin-1,2,4-thiadiazol-5-amine hydrochloride

Tert-butyl (3-morpholinyl-1,2,4-thiadiazol-5-yl)carbamate (80 mg, 0.279 mmol) was dissolved in the solution of 4 N hydrochloric acid/dioxane (3 mL), and the reaction mixture was stirred at room temperature to react for 16 h. The reaction mixture was concentrated under reduced pressure to obtain the title compound (60 mg, white solid, yield: 96.4%). LC/MS (ESI) m/z:187.2 [M+H]⁺.

### Step 5: 2"-(Difluoromethyl)-3-fluoro-5"-methoxy-N-(3-morpholinyl-1,2,4-thiadiazol-5-yl)-2-oxo-2H-[1,2":4',4"-terpyridin]-5'-carboxamide

At room temperature, 2"-(difluoromethyl)-3-fluoro-5"-methoxy-2-oxo-2H-[1,2':4',4"-terpyridin]-5'-carboxylic acid (20 mg, 0.051 mmol), 3-morpholin-1,2,4-thiadiazol-5-amine hydrochloride (14 mg, 0.061 mmol), and N-methylimidazole (17 mg, 0.207 mmol) was dissolved in acetonitrile (3 mL), and then stirred at 70°C for 5 min, N,N,N',N'-tetramethylchloroformamidinium hexafluorophosphate (22 mg, 0.078 mmol) was added to the reaction mixture, and then, the reaction mixture was stirred at 70°C to react for 1 h. The reaction mixture was filtered, and subjected to the preparative HPLC to obtain the title compound (8.16 mg, white solid, yield: 28.5%). ¹H NMR (400 MHz, DMSO-*d₆*) δ 13.60 (s, 1H), 9.02 (s, 1H), 8.52 (s, 1H), 8.08 (s, 1H), 7.89 - 7.83 (m, 1H), 7.74 (s, 1H), 7.61 - 7.53 (m, 1H), 7.01 (t, *J =* 55.1 Hz, 1H), 6.47 - 6.39 (m, 1H), 3.70 (s, 3H), 3.69 - 3.65 (m, 4H), 3.54 - 3.49 (m, 4H). LC/MS (ESI) m/z: 560.2 [M+H)⁺.

### Example 25: N-(3-(2-oxa-6-azaspiro[3.3]heptan-6-yl)-1,2,4-thiadiazol-5-yl)-2"-(difluoromethyl)-3-fluoro-5"-methoxy-2-oxo-2H-[1,2':4',4"-bipyridin]-5'-carboxamide

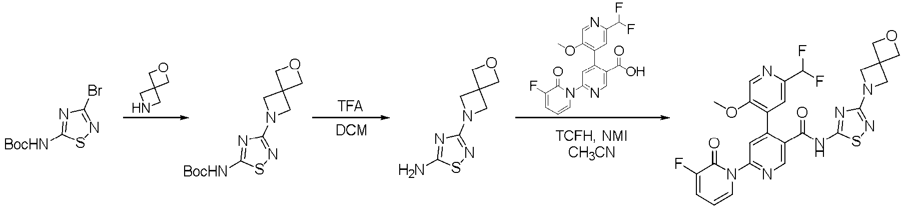

### Step 1: Tert-butyl (3-(2-oxa-6-azaspiro[3.3]heptan-6-yl)-1,2,4-thiadiazol-5-yl)carbamate

Tert-butyl (3-bromo-1,2,4-thiadiazol-5-yl)carbamate (50 mg, 0.178 mmol) was dissolved in 2-oxa-6-azaspiro[3.3]heptane (0.5 mL), and the reaction mixture was stirred at 100°C for 3 h. The reaction mixture was diluted with ethyl acetate, and the organic phase was washed with saturated brine, dried over anhydrous sodium sulfate, and concentrated under reduced pressure. The residues was purified by silica-gel column chromatography (eluent: petroleum ether/ethyl acetate, gradient: 30-50% ethyl acetate) to obtain the title compound (white solid, 20 mg, yield: 37.6%). LC/MS (ESI) m/z: 299.2 [M+H]⁺.

### Step 2: 3-(2-oxa-6-azaspiro[3.3]heptan-6-yl)-1,2,4-thiadiazol-5-amine

Tert-butyl (3-(2-oxa-6-azaspiro[3.3]heptan-6-yl)-1,2,4-thiadiazol-5-yl)carbamate (20 mg, 0.067 mmol) was dissolved in the solution of dichloromethane (2 mL), trifluoroacetic acid (0.2 mL) was added to the reaction mixture, and the reaction mixture was stirred at room temperature to react for 1 h. The reaction mixture was regulated to the pH of 7-8 with sodium bicarbonate, and then extracted with dichloromethane three times. The organic phase was washed with saturated brine, dried over anhydrous sodium sulfate, and concentrated under reduced pressure to obtain the title compound (10 mg, white solid, yield: 75.3%). LC/MS (ESI) m/z:199.1 [M+H]⁺.

### Step 3: N-(3-(2-oxa-6-azasniro[3.3]heptan-6-yl)-1,2,4-thiadiazol-5-yl)-2"-(difluoromethyl)-3-fluoro-5"-methoxy-2-oxo-2H-[1,2':4',4"-bipyridin]-5'-carboxamide

At room temperature, 2"-(difluoromethyl)-3-fluoro-5"-methoxy-2-oxo-2H-[1,2':4',4"-terpyridin]-5'-carboxylic acid (20 mg, 0.051 mmol), 3-(2-oxa-6-azaspiro[3.3]heptan-6-yl)-1,2,4-thiadiazol-5-amine (10 mg, 0.050 mmol), and N-methylimidazole (17 mg, 0.207 mmol) was dissolved in acetonitrile (3 mL) and then stirred at 70°C for 5 min, N,N,N',N'-tetramethylchloroformamidinium hexafluorophosphate (22 mg, 0.078 mmol) was added to the reaction mixture which was substantially clear, and then, the reaction mixture was stirred at 70°C to react for 1 h. The reaction mixture was filtered and then purified by the preparative HPLC to obtain the title compound (1.29 mg, white solid, yield: 4.4%). ¹H NMR (400 MHz, DMSO-*d₆*) δ 13.65 (s, 1H), 9.00 (s, 1H), 8.51 (s, 1H), 8.07 (s, 1H), 7.86 (m, 1H), 7.73 (s, 1H), 7.61 - 7.52 (m, 1H), 7.00 (t, *J* = 55.1 Hz, 1H), 6.42 (m, 1H), 4.71 (s, 4H), 4.19 (s, 4H), 3.68 (s, 3H). LC/MS (ESI) m/z: 572.2 [M+H]⁺.

### Example 26: 2"-chloro-N-(3-(1,4-dimethyl-1H-pyrazol-5-yl)-1,2,4-thiadiazol-5-yl)-3-fluoro-5"-methoxy-2-oxo-2H-[1,2':4',4"-terpyridin]-5'-carboxamide

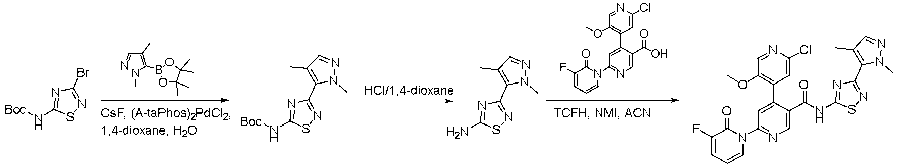

### Step 1: Tert-butyl 3-(1,4-dimethyl-1H-pyrazol-5-yl)-1,2,4-thiadiazol-5-yl carbamate

Under the protection of nitrogen, tert-butyl (3-bromo-1,2,4-thiadiazol-5-yl)carbamate (100 mg, 0.357 mmol), 1,4-dimethyl-5-(4,4,5,5-tetramethyl-1,3,2-dioxaboran-2-yl)-1H-pyrazole (95 mg, 0.428 mmol), cesium fluoride (108 mg, 0.711 mmol) and dichlorobis[di-tert-butyl-(4-dimethylaminophenyl)phosphino]palladium (II) (25 mg, 0.035 mmol) was dissolved in dioxane (5 mL) and water (0.5 mL), and the reaction mixture was stirred at 90°C to react for 16 h. The reaction mixture was diluted with ethyl acetate, washed with saturated brine, dried over anhydrous sodium sulfate, and concentrated under reduced pressure. The residues was purified by silica-gel column chromatography (eluent: petroleum ether/ethyl acetate, gradient: 10-20% ethyl acetate) to obtain the title compound (white solid, 100 mg, yield: 94.9%). LC/MS (ESI) m/z: 296.2 [M+H]⁺.

### Step 2: 3-(1,4-Dimethyl-1H-pyrazol-5-yl)-1,2,4-thiadiazol-5-amine hydrochloride

Tert-butyl 3-(1,4-dimethyl-1H-pyrazol-5-yl)-1,2,4-thiadiazol-5-yl carbamate (100 mg, 0.339 mmol) was dissolved in the solution of 4 N dioxane hydrochloride (5 mL), and the reaction mixture was stirred at room temperature to react for 16 h. The reaction mixture was concentrated under reduced pressure and pulped with ethyl acetate to obtain the title compound (70 mg, light yellow solid, yield: 89.2%). LC/MS (ESI) m/z: 196.1 [M+H]⁺.

### Step 3: 2"-Chloro-N-(3-(1,4-dimethyl-1H-pyrazol-5-yl)-1,2,4-thiadiazol-5-yl)-3-fluoro-5"-methoxy-2-oxo-2H-[1,2':4',4"-terpyridin]-5'-carboxamide

At room temperature, 2"-chloro-3-fluoro-5"-methoxy-2-oxo-2H-[1,2':4',4"-terpyridin]-5'-carboxylic acid (30 mg, 0.080 mmol), 3-(1,4-dimethyl-1H-pyrazol-5-yl)-1,2,4-thiadiazol-5-amine hydrochloride (22 mg, 0.097 mmol), and N-methylimidazole (26 mg, 0.317 mmol) was dissolved in acetonitrile (5 mL) and N,N-dimethylcarboxamide (2 mL), and then stirred at 70°C for 5 min, N,N,N',N'-tetramethylchloroformamidinium hexafluorophosphate (45 mg, 0.160 mmol) was added to the reaction mixture, and then, the reaction mixture was stirred at 70°C to react for 1 h. The reaction mixture was filtered, the filtrate was purified by the preparative HPLC to obtain the title compound (8.06 mg, white solid, yield: 18.3%). ¹H NMR (400 MHz, DMSO-*d*₆) δ 13.98 (s, 1H), 9.10 (s, 1H), 8.24 (s, 1H), 8.10 (s, 1H), 7.91 - 7.85 (m, 1H), 7.60 (s, 1H), 7.60 - 7.54 (m, 1H), 7.39 (s, 1H), 6.50 - 6.38 (m, 1H), 4.15 (s, 3H), 3.61 (s, 3H), 2.31 (s, 3H). LC/MS (ESI) m/z: 553.1 [M+H]⁺.

### Example 27: N-(3-(3,6-diazabicyclo[3.1.1]heptan-3-yl)-1,2,4-thiadiazol-5-yl)-2"-(difluoromethyl)-3-fluoro-5"-methoxy-2-oxo-2H-[1,2':4',4"-terpyridin]-5'-carboxamide

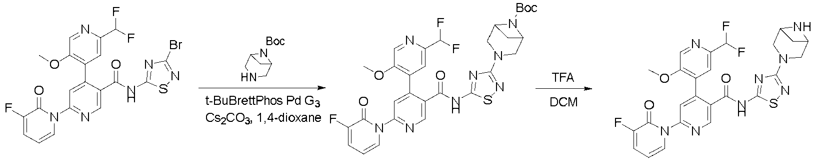

### Step 1: Tert-butyl 3-(5-(2"-(difluoromethyl)-3-fluoro-5"-methoxy-2-oxo-2H-[1,2':4',4"-terpyridin]-5'-carboxamide)-1,2,4-thiadiazol-3-yl)-3,6-diazabicyclo[3,1,1]heptan-6-carboxylate

Under the protection of nitrogen, N-(3-bromo-1,2,4-thiadiazol-5-yl)-2"-(difluoromethyl)-3-fluoro-5"-methoxy-2-oxo-2H-[1,2':4',4"-terpyridin]-5'-carboxamide (150 mg, 0.271 mmol) was dissolved in a solution of anhydrous 1,4-dioxane (5 mL), 3,6-diazabicyclo[3.1.1]heptan-6-tert-butyl carboxylate (108 mg, 0.545 mmol), cesium carbonate (353 mg, 1.08 mmol) and methansulfonate 2-(di-tert-butylphosphinoyl)-3,6-dimethoxy-2',4',6'-triisopropyl-1,1'-biphenyl(2-amino-1,1'-biphenyl-2-yl)palladium (II) (12 mg, 0.014 mmol) was then added to the reaction mixture, and the reaction mixture was stirred at 130°C under the protection of nitrogen to react for 1 h. The mixture was filtered and then concentrated under reduced pressure. The residues was purified by silica-gel column chromatography (eluent: dichloromethane/methanol, gradient: 0-10% methanol) to obtain the title compound (white solid, 35 mg, yield: 19.3%). LC/MS (ESI) m/z: 671.2 [M+H]⁺.

### Step 2: N-(3-(3,6-diazabicyclo[3.1.1]heptan-3-yl)-1,2,4-thiadiazol-5-yl)-2"-(difluoromethyl)-3-fluoro-5"-methoxy-2-oxo-2H-[1,2':4',4"-terpyridin]-5'-carboxamide

In the ice-water bath, 3-(5-(2"-(difluoromethyl)-3-fluoro-5"-methoxy-2-oxo-2H-[1,2':4',4"-terpyridin]-5'-carboxamide)-1,2,4-thiadiazol-3-yl)-3,6-diazabicyclo[3.1.1]heptan-6-tert-butyl carboxylate (35 mg, 0.052 mmol) was dissolved in the solution of dichloromethane (2.5 mL), and trifluoroacetic acid (0.5 mL) was added to the reaction mixture, after which the reaction mixture was heated to room temperature and then stirred to react for 16 h. The reaction mixture was concentrated under reduced pressure, and the residues was purified by the preparative HPLC (YMC-Actus Triart C18 250*21mm, 30-100% methanol in H₂O and 0.1% FA) to obtain the title compound (13.42 mg, light yellow solid, yield: 45.1%). ¹H NMR (400 MHz, DMSO-*d₆*) δ 9.10 (s, 1H), 8.47 (s, 1H), 7.89 (s, 1H), 7.87 - 7.82 (m, 1H), 7.65 (s, 1H), 7.59 - 7.51 (m, 1H), 6.97 (t, *J* = 55.1 Hz, 1H), 6.47 - 6.39 (m, 1H), 4.35 (d, *J =* 6.3 Hz, 2H), 3.97 - 3.88 (m, 4H), 3.69 (s, 3H), 2.88 - 2.79 (m, 1H), 1.84 (d, *J* = 10.0 Hz, 1H). LC/MS (ESI) (m/z): 571 [M+H]⁺.

### Example 28: N-(3-(3-azabicyclo[3,1,0]hex-3-yl)-1,2,4-thiadiazol-5-yl)-2"-(difluoromethyl)-3-fluoro-5"-methoxy-2-oxo-2H-[1,2':4',4"-terpyridin]-5'-carboxamide

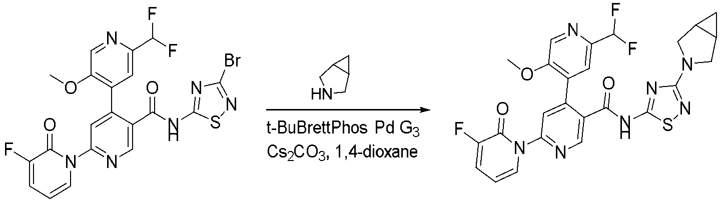

### Step 1: N-(3-(3-azabicyclo[3,1,0]hex-3-yl)-1,2,4-thiadiazol-5-yl)-2"-(difluoromethyl)-3-fluoro-5"-methoxy-2-oxo-2H-[1,2':4',4"-terpyridin]-5'-carboxamide

Under the protection of nitrogen, N-(3-bromo-1,2,4-thiadiazol-5-yl)-2"-(difluoromethyl)-3-fluoro-5"-methoxy-2-oxo-2H-[1,2':4',4"-terpyridin]-5'-carboxamide (50 mg, 0.09 mmol) was dissolved in a solution of anhydrous dioxane (3 mL), 3-azabicyclo[3.1.0]hexane hydrochloride (22 mg, 0.18 mmol), cesium carbonate (118 mg, 0.36 mmol) and methansulfonate 2-(di-tert-butylphosphinoyl)-3,6-dimethoxy-2',4',6'-triisopropyl-1,1'-biphenyl(2-amino-1,1'-biphenyl-2-yl)palladium (II) (4 mg, 0.005 mmol) was then sequentially added to the reaction mixture, and the reaction mixture was stirred at 110°C under the protection of nitrogen to react for 3 h. The mixture was diluted with ethyl acetate, sequentially washed with water and saturated brine, dried over anhydrous sodium sulfate, filtered, and concentrated under reduced pressure . The residues was purified by the preparative HPLC (YMC-Actus Triart C18 250*21mm, 30-100% methanol in H₂O and 0.1% FA) to obtain the title compound (white solid, 1.52 mg, yield: 3.0%). ¹H NMR (400 MHz, DMSO-*d₆*) δ 13.58 (s, 1H), 9.00 (s, 1H), 8.51 (s, 1H), 8.07 (s, 1H), 7.89 - 7.82 (m, 1H), 7.73 (s, 1H), 7.60 - 7.53 (m, 1H), 7.00 (t, *J =* 55.0 Hz, 1H), 6.46 - 6.38 (m, 1H), 3.73 - 3.70 (m, 1H), 3.69 (s, 3H), 3.68 - 3.66 (m, 1H), 3.49 - 3.45 (m, 2H), 1.67 - 1.59 (m, 2H), 0.75 - 0.66 (m, 1H), 0.23 - 0.17 (m, 1H). LC/MS (ESI) (m/z): 556 [M+H]⁺.

### Example 29: 2"-(difluoromethyl)-3-fluoro-5"-methoxy-N-(3-(6-methyl-3,6-diazabicyclo[3.1.1]heptan-3-yl)-1,2,4-thiadiazol-5-y)-2-oxo-2H-[1,2':4',4"-terpyridin]-5'-carboxamide

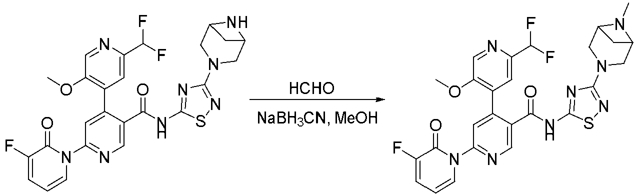

### Step 1: 2"-(Difluoromethyl)-3-fluoro-5"-methoxy-N-(3-(6-methyl-3,6-diazabicyclo[3.1.1]heptan-3-yl)-1,2,4-thiadiazol-5-yl)-2-oxo-2H-[1,2':4',4"-terpyridin]-5'-carboxamide

At room temperature, N-(3-(3,6-diazabicyclo[3.1.1]heptan-3-yl)-1,2,4-thiadiazol-5-yl)-2"-(difluoromethyl)-3-fluoro-5"-methoxy-2-oxo-2H-[1,2':4',4"-terpyridin]-5'-carboxamide (10 mg, 0.018 mmol) was dissolved in the solution of methanol (3 mL), paraformaldehyde (2 mg, 0.067 mmol) and acetic acid (one drop) was then added to the reaction mixture and then stirred at room temperature to react for 1 h, and next, sodium borohydride acetate (3 mg, 0.048 mmol) was added to the reaction mixture, after which the mixture was further stirred at 50°C to react for 16 h. The reaction mixture was filtered and then purified by the preparative HPLC (YMC-Actus Triart C18 250* 21mm, 30-100% methanol in H₂O and 0.1% FA) to obtain the title compound (3.60 mg, white solid, yield: 35.1%). ¹H NMR (400 MHz, CDCl₃) δ 10.93 (s, 1H), 8.82 (s, 1H), 8.17 (s, 1H), 8.12 (s, 1H), 7.90 - 7.86 (m, 1H), 7.62 (s, 1H), 7.34 - 7.27 (m, 1H), 6.66 (t, *J* = 55.5 Hz, 1H), 6.44 - 6.34 (m, 1H), 4.06 - 4.03 (m, 1H), 4.03 - 3.98 (m, 5H), 3.59 (s, 3H), 3.51 - 3.42 (m, 1H), 2.66 (s, 3H), 1.79 (d, *J* = 10.0 Hz, 1H). LC/MS (ESI) (m/z): 585 [M+H]⁺.

### Example 30: N-(3-amino-1,2,4-thiadiazol-5-yl)-2"-(difluoromethyl)-3-fluoro-5"-methoxy-2-oxo-2H-[1,2':4',4"-terpyridin]-5'-carboxamide

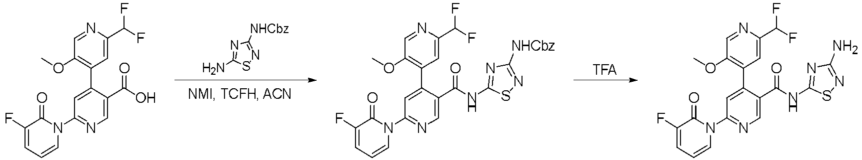

### Step 1: Benzyl (5-(2"-(difluoromethyl)-3-fluoro-5"-methoxy-2-oxo-2H-[1,2':4',4"-terpyridin]-5'-formamido)-1,2,4-thiadiazol-3-yl)carbamate

Under the protection of nitrogen, benzyl 2"-(difluoromethyl)-3-fluoro-5"-methoxy-2-oxo-2H-[1,2':4',4"-terpyridin]-5'-carboxylic acid (78.2 mg, 0.20 mmol), (5-amino-1,2,4-thiadiazol-3-yl)carbamate (50 mg, 0.20 mmol) and N-methylimidazole (65.6 mg, 0.78 mmol) was dissolved in anhydrous acetonitrile (2 mL), and stirred at 70°C for 5 min, N,N,N',N'-tetramethylchloroformamidinium hexafluorophosphate (84.1 mg, 0.30 mmol) was added to the reaction mixture, and then, the reaction mixture was stirred at 70°C to react for 1 h. The reaction mixture was concentrated under reduced pressure; and the residues was purified by silica-gel column chromatography (eluent: dichloromethane/methanol, gradient: 0-3% methanol )to obtain the title compound (white solid, 75 mg, yield: 60%). LC/MS (ESI) m/z: 624.3 [M+H]⁺.

### Step 2: N-(3-amino-1,2,4-thiadiazol-5-yl)-2"-(difluoromethyl)-3-fluoro-5"-methoxy-2-oxo-2H-[1,2":4',4"-terpyridin]-5'-carboxamide

At room temperature, trifluoroacetic acid (2 mL) was added to a flask containing benzyl (5-(2"-(difluoromethyl)-3-fluoro-5"-methoxy-2-oxo-2H-[1,2':4',4"-terpyridin]-5'-formamido)-1,2,4-thiadiazol-3-yl)carbamate (70 mg, 0.11 mmol), and then, the reaction mixture was stirred at 80°C to react for 1 h. The reaction mixture was cooled to room temperature, and then concentrated under reduced pressure to remove trifluoroacetic acid, and the residues was diluted with ethyl acetate and regulated to the pH of 8 with a saturated aqueous sodium bicarbonate solution. Then, the aqueous phase was extracted with ethyl acetate, the organic phases were combined, dried over anhydrous sodium sulfate and filtered, and the filtrate was concentrated under reduced pressure. The residues was purified by the preparative HPLC (Welch-Ultimate AQ-C18, 21.2*250mm,5µm, 10-95% acetonitrile in H₂O containing 0.1% FA) to obtain the title compound (white solid, 6 mg, yield: 33%). ¹H NMR (400 MHz, DMSO-*d₆*) δ 13.29 (s, 1H), 8.99 (s, 1H), 8.52 (s, 1H), 8.07 (s, 1H), 7.86 (d, *J* = 7.1 Hz, 1H), 7.74 (s, 1H), 7.62 - 7.46 (m, 1H), 7.00 (t*, J =* 55.0 Hz, 1H), 6.48 - 6.29 (m, 3H), 3.70 (s, 3H). LC/MS (ESI) (m/z): 490 [M+H]⁺.

### Example 31: N-(3-acetamino-1,2,4-thiadiazol-5-yl)-2"-(difluoromethyl)-3-fluoro-5"-methoxy-2-oxo-2H-[1,2':4',4"-terpyridin]-5'-carboxamide

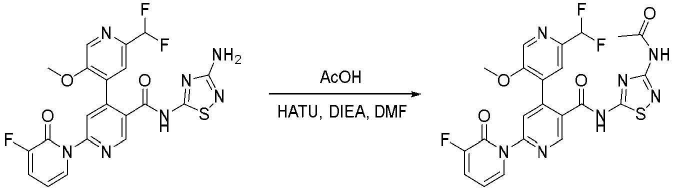

### Step 1: N-(3-acetamino-1,2,4-thiadiazol-5-yl)-2"-(difluoromethyl)-3-fluoro-5"-methoxy-2-oxo-2H-[1,2':4',4"-terpyridin]-5'-carboxamide

Under the protection of nitrogen, N-(3-amino-1,2,4-thiadiazol-5-yl)-2"-(difluoromethyl)-3-fluoro-5"-methoxy-2-oxo-2H-[1,2':4',4"-terpyridin]-5'-carboxamide (15 mg, 0.03 mmol), acetic acid (0.02 mL, 0.305 mmol) and N,N-diisopropylethylamine (11.9 mg, 0.092 mmol) was dissolved in anhydrous N,N-dimethylcarboxamide (1 mL), followed by the addition of 2-(7-azabenzotriazol)-N,N,N",N"-tetramethylurea hexafluorophosphate (17.5 mg, 0.046 mmol). The reaction mixture was stirred at room temperature for 1 hours. The reaction mixture was filtered, the filtrate was purified by the preparative HPLC (Welch XBridge C18 250*21 mm, 10-95% acetonitrile in H₂O containing 0.1% FA) to obtain the title compound (white solid, 7 mg, yield: 43%). ¹H NMR (400 MHz, DMSO-*d₆*) δ 13.79 (s, 1H), 10.88 (s, 1H), 9.04 (s, 1H), 8.51 (s, 1H), 8.10 (s, 1H), 7.86 (d, *J=* 7.2 Hz, 1H), 7.76 (s, 1H), 7.60 - 7.52 (m, 1H), 7.01 (t, *J=* 55.1 Hz, 1H), 6.45 - 6.40 (m, 1H), 3.68 (s, 3H), 2.12 (s, 3H). LC/MS (ESI) (m/z): 532 [M+H]⁺.

### Example 32: 2"-(difluoromethyl)-N-(3-(1,4-dimethyl-1H-imidazol-5-yl)-1,2,4-thiadiazol-5-yl)-3-fluoro-5"-methoxy-2-oxo-2H-[1,2':4',4"-terpyridin]-5'-carboxamide

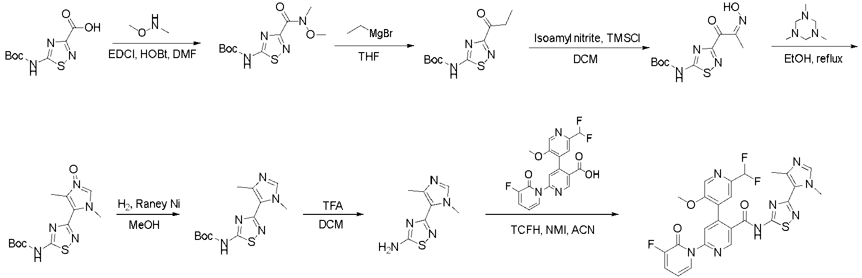

### Step 1: Tert-butyl (3-(methoxy(methyl)carbamyl)-1,2,4-thiadiazol-5-yl)carbamate

At room temperature, 5-((tert-butoxycarbonyl)amino)-1,2,4-thiadiazol-3-carboxylic acid (600 mg, 2.45 mmol, crude product ), dimethylhydroxylamine hydrochloride (358.3 mg, 3.67 mmol), N,N-diisopropylethylamine (1 mL, 6.12 mmol) and 1-hydroxylbenzotriazole (495.9 mg, 3.67 mmol) was dissolved in N,N-dimethylcarboxamide (10 mL), followed by the addition of 1-(3-dimethylaminopropyl)-3-ethyl carbodiimide hydrochloride (701.6 mg, 3.67 mmol), and the reaction mixture was then stirred at room temperature to react for 1 h. Ethyl acetate and water was added to the reaction mixture for layer separation, the organic phase was washed with saturated brine, dried over anhydrous sodium sulfate and filtered, and the filtrate was concentrated under reduced pressure. The residues was subjected to the silica-gel column chromatography (eluent: petroleum ether/ethyl acetate, gradient: 0-50% ethyl acetate) to obtain the title compound (white solid, 450 mg, yield: 64%). LC/MS (ESI) (m/z): 289 [M+H]⁺.

### Step 2: Tert-butyl (3-propionyl-1,2,4-thiadiazol-5-yl)carbamate

Under the protection of nitrogen, tert-butyl (3-(methoxy(methyl)carbamyl)-1,2,4-thiadiazol-5-yl)carbamate (450 mg, 1.56 mmol) was dissolved in tetrahydrofuran (5 mL), the reaction mixture was cooled to -78 °C, followed by the slow dropwise addition of 1.0 M ethylmagnesium bromide (2.0 mL, 2.03 mmol), and the reaction mixture was further stirred at -78°C to react for 1 h. The reaction mixture was quenched with a saturated aqueous ammonium chloride solution at 0°C and extracted with ethyl acetate twice. The organic phases was combined, washed with water and saturated brine, dried over anhydrous sodium sulfate and filtered. The filtrate was concentrated under reduced pressure, and the residues was subjected to the silica-gel column chromatography (eluent: petroleum ether/ethyl acetate, gradient: 0-50% ethyl acetate) to obtain the title compound (white solid, 160 mg, yield: 40%). LC/MS (ESI) (m/z): 258 [M+H]⁺.

### Step 3: Tert-butyl (Z)-(3-(2-(hydroxylimino)propionyl)-1,2,4-thiadiazol-5-yl)carbamate

Under the protection of nitrogen, tert-butyl (3-propionyl-1,2,4-thiadiazol-5-yl)carbamate (250 mg, 0.97 mmol) and trimethylchlorosilane (126.7 mg, 1.17 mmol) was dissolved in anhydrous dichloromethane (5 mL), the reaction mixture was cooled to -20 °C, followed by the addition of isoamyl nitrite (136.6 mg, 1.17 mmol), and then, the mixture was heated to room temperature and stirred to react for 16 h. Dichloromethane and water was added to the reaction mixture, the organic phases was separated, washed with saturated brine, dried over anhydrous sodium sulfate and filtered, and the filtrate was concentrated under reduced pressure to obtain the title compound (brown solid, 210 mg, yield: 75%). LC/MS (ESI) m/z: 287.2 [M+H]⁺.

### Step 4: 5-(5-((Tert-butoxycarbonyl)amino)-1,2,4-thiadiazol-3-yl)-1,4-dimethyl-1H-imidazol3-oxide

At room temperature, tert-butyl (Z)-(3-(2-(hydroxylimino)propionyl)-1,2,4-thiadiazol-5-yl)carbamate (50 mg, 0.175 mmol) was added to a flask containing absolute ethanol (3 mL), followed by the addition of 1,3,5-trimethylhexahydro-1,3,5-triazine (67.6 mg, 0.524 mmol) at room temperature. The reaction mixture was stirred under refluxing to react for 1 h. The reaction mixture was cooled to room temperature, and concentrated under reduced pressure to obtain the title compound (yellow solid, 50 mg, crude product, yield: 100%). LC/MS (ESI) m/z: 312 [M+H]⁺.

### Step 5: Tert-butyl (3-(1,4-dimethyl-1H-imidazol-5-yl)-1,2,4-thiadiazol-5-yl)carbamate

Under the protection of nitrogen, 5-(5-((tert-butoxycarbonyl)amino)-1,2,4-thiadiazol-3-yl)-1,4-dimethyl-1H-imidazol3-oxide (50 mg, 0.161 mmol) was dissolved in absolute methanol (1 mL), followed by the addition of raney nickel (10 mg) under the protection of nitrogen. The reaction mixture was subjected to nitrogen displacement three times, and then stirred at room temperature in an atmosphere of hydrogen under one atmospheric pressure to react for 1 h. The reaction mixture was filtered through diatomite, and the filtrate was concentrated under reduced pressure to obtain the title compound (brown oil, 26 mg, yield: 55%). LC/MS (ESI) m/z: 296.3 [M+H]⁺.

### Step 6: 3-(1,4-Dimethyl-1H-imidazol-5-yl)-1,2,4-thiadiazol-5-amine

At room temperature, tert-butyl (3-(1,4-dimethyl-1H-imidazol-5-yl)-1,2,4-thiadiazol-5-yl)carbamate (50 mg, 0.161 mmol) was dissolved in anhydrous dichloromethane (2 mL), followed by the slow dropwise addition of trifluoroacetic acid (0.5 mL) in an ice-water bath. The reaction mixture was stirred at room temperature to react for 1 h. The reaction mixture was concentrated under reduced pressure to remove trifluoroacetic acid, then diluted with the addition of water, regulated to the pH of 8 with a saturated aqueous sodium bicarbonate solution, and then extracted with ethyl acetate. The organic phase was washed with saturated brine, dried over anhydrous sodium sulfate and filtered, and the filtrate concentrated under reduced pressure to obtain the title compound (yellow oil, 15 mg, yield: 87%). LC/MS (ESI) m/z: 196.3 [M+H]⁺.

### Step 7: 2"-(Difluoromethyl)-N-(3-(1,4-dimethyl-1H-imidazol-5-yl)-1,2,4-thiadiazol-5-yl)-3-fluoro-5"-methoxy-2-oxo-2H-[1,2':4',4"-terpyridin]-5'-carboxamide

Under the protection of nitrogen, 2"-(difluoromethyl)-3-fluoro-5"-methoxy-2-oxo-2H-[1,2':4',4"-terpyridin]-5'-carboxylic acid (30 mg, 0.077 mmol), 3-(1,4-dimethyl-1H-imidazol-5-yl)-1,2,4-thiadiazol-5-amine (15 mg, 0.077 mmol) and N-methylimidazole (25 mg, 0.31 mmol) was dissolved in anhydrous acetonitrile (2 mL) and stirred at 70°C for 5 min, N,N,N',N'-tetramethylchloroformamidinium hexafluorophosphate (32.3 mg, 0.12 mmol) was added to the reaction mixture, and then, the reaction mixture was stirred at 70°C to react for 1 h. The reaction mixture was filtered, the filtrate was purified by the preparative HPLC (Welch XBridge xb 5um 21.2*250mm, 30-95% methanol in H₂O containing 0.1% TFA) to obtain the title compound (white solid, 0.4 mg, yield: 0.92%). ¹H NMR (400 MHz, DMSO-*d₆*) δ 9.10 (s, 1H), 8.78 (s, 1H), 8.53 (s, 1H), 8.13 (s, 1H), 7.88 (d, *J=* 7.1 Hz, 1H), 7.79 (s, 1H), 7.64 - 7.54 (m, 1H), 7.02 (t, *J=* 55.0 Hz, 1H), 6.47 - 6.42 (m, 1H), 4.09 (s, 3H), 3.69 (s, 3H), 2.63 (s, 3H). LC/MS (ESI) (m/z): 569 [M+H]⁺.

### Example 33: N-(3-cyclobutyl-1,2,4-thiadiazol-5-yl)-2"-(difluoromethyl)-3-fluoro-5"-methoxy-2-oxo-2H-[1,2":4'4"-bipyridin]-5'-carboxamide

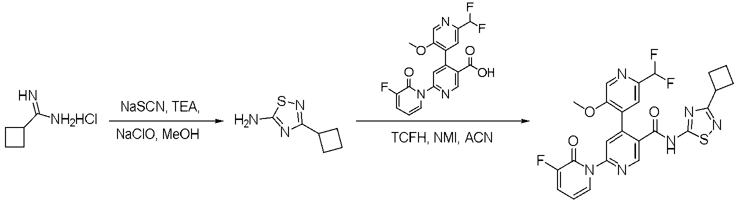

### Step 1: 3-Cyclobutyl-1,2,4-thiadiazol-5-amine

At room temperature, sodium thiocyanate (196 mg, 2.41 mmol) was dissolved in methanol (30 mL), and the mixture was then cooled to -20°C, followed by the sequential addition of triethylamine (0.31 mL, 2.23 mmol) and cyclobutanecarboxamidine Hydrochloride (250 mg, 1.86 mmol). After addition, the mixture was stirred at -20°C to react for 45 min, followed by the addition of triethylamine (0.26 mL, 1.86 mmol) and then the dropwise addition of 8% aqueous sodium hypochlorite solution (1.73 g, 1.86 mmol). After addition, the mixture was further stirred at-20°C to react for 2 h, then heated to room temperature and stirred overnight. The reaction mixture was concentrated under reduced pressure, ethyl acetate and water was added to the residues for liquid separation, and the aqueous phase was extracted with ethyl acetate. The organic phases was combined, sequentially washed with water and saturated brine and concentrated under reduced pressure, and the residues was purified by the silica-gel column chromatography (eluent: petroleum ether/ethyl acetate, gradient: 2-50% ethyl acetate) to obtain the title compound (white solid, 50 mg, yield: 17%). LC/MS (ESI) m/z: 156.3 [M+H]⁺.

### Step 2: N-(3-cyclobutyl-1,2,4-thiadiazol-5-yl)-2"-(difluoromethyl)-3-fluoro-5"-methoxy-2-oxo-2H-[1,2':4',4"-terpyridin]-5'-carboxamide

At room temperature, 3-cyclobutyl-1,2,4-thiadiazol-5-amine (15 mg, 0.097 mmol), 2"-(difluoromethyl)-3-fluoro-5"-methoxy-2-oxo-2H-[1,2':4',4"-terpyridin]-5'-carboxylic acid (38 mg, 0.097 mmol), N-methylimidazole (48 mg, 0.58 mmol) and acetonitrile (2 mL) was added to a reaction flask and then stirred at 70°C for 1 min, the solution of N,N,N',N'-tetramethylchloroformamidinium hexafluorophosphate (54 mg, 0.19 mmol) in acetonitrile (1 mL) was added to the reaction mixture, and then, the reaction mixture was stirred at 70°C to react for 1 h. The mixture was filtered and then purified by the preparative HPLC (YMC-Actus Triart C18 250*21 mm, 10-100% acetonitrile in H₂O containing 0.1% FA) to obtain the title compound (15 mg, white solid, yield: 29%). ¹H NMR (400 MHz, DMSO-*d₆*) δ 13.76 (s, 1H), 9.04 (s, 1H), 8.51 (s, 1H), 8.10 (s, 1H), 7.87 (d, *J* = 7.0 Hz, 1H), 7.76 (s, 1H), 7.61 - 7.53 (m, 1H), 7.01 (t, *J =* 55.0 Hz, 1H), 6.45 - 6.40 (m, 1H), 3.77 - 3.71 (m, 1H), 3.67 (s, 3H), 2.40 - 2.31 (m, 4H), 2.12 - 1.83 (m, 2H). LC/MS (ESI) (m/z): 529 [M+H]⁺.

### Effect Example

### Experimental Approaches:

### 1. Biochemical Activity Assay (ADP Glo Assay) of Pol θ Inhibitor

The N-terminus of the Pol θ protein contains a helicase domain, which has an ATPase activity and can hydrolyze ATP into ADP, and the ATPase activity of the Pol θ protein and the inhibitory effect of small molecule compounds on the protein may be analyzed by ADP-Glo^{™} Kinase Assay (Promega, V9102). The specific assay method was as follows:
1) The helicase domain of the Pol θ protein was purified by an insect system, to obtain the protein with a purity of >90%. ssDNA (5'-CCAGTGAATTGTTGCTCGGTACCTGCTAAC-3', Hangzhou Yocon Biotechnology Co., Ltd.) was ordered as the substrate for the Pol θ protein; and a reaction buffer containing 10 mM DTT (Dithiothreitol), 20 mM MgCl₂ and Tris-HCl pH 7.5 was prepared.
2) The 2x ssDNA-Pol θ premix was prepared; the reaction was conducted in a 384-well plate; a control group with only buffer is set, and the premix was added to the rest of the groups. The compound was added with an automated pipette (Thermo, Multidrop 8) and diluted at an initial concentration of 10 µM and at a dilution ratio of 1/3; and a total of 9 detection points were set, with 2 replicates in each group.
3) The 2x ATP solution was prepared, and all wells were added with 2x ATP solution in equal volume, and allowed to stand at the room temperature for 60 min.
4) According to the instructions of Promega reagent, ADP-Glo Detection Reagent was added to the reaction system, which was allowed to stand at the room temperature for 60 min.
5) According to the instructions of Promega reagent, Kinase Detection Reagent was added to the reaction system, which was allowed to stand at room temperature for 60 min; chemiluminescence signals were detected by a microplate reader (Thermo, VarioskanLUX); and the reading interval for each well was set to 1000 ms. Data analysis was conduct on the measurement results: the CV% of the control group should be less than 10%, and the value z' should be greater than 0.5. Data satisfying the above quality control results were used to calculate the inhibition rate of the compound (inhibition rate (%) = 100 * (average value of control group - average value of experimental group)/(average value of control group - average value of buffer control group). GraphPad Prism8 was used to perform nonlinear regression to fit the curves of the inhibition rates of the compounds to obtain IC₅₀ values, with the results shown in Table 3.

### 2. Cell Viability Assay of Pol θ Inhibitor

The cell viability assay, i.e., the commonly used CTG assay, was conducted for the efficacy of the inhibitor in vitro using the CellTiter Glo reagent (Promega, G7573). The specific assay method was as follows:
1) DLD1wild type (ATCC, CCL-221) and DLD1 BRCA2-/- cells were cultured, digested with trypsin (0.025% Trypsin-EDTA, Hyclone) one day before the assays, centrifuged at 1000 rpm for 3 min, and then collected. The cells were counted with a counter (Shanghai Monwei Biomedical Technology Co., Ltd., SmartCell600A.SC1006), and seeded into 96-well white culture plates at an appropriate seeding ratio,
2) 24 hours after cell plating, dosing treatment was conducted, with the day denoted as Day 0; the compound was added using the automated pipette (Thermo, Multidrop 8); the plate was set to a 96-well plate; the initial concentration was set to 30 µM, and dilution was conducted at the dilution ratio of 1/3, and a total of 9 detection points were set, with 2 replicates in each group; and the cells were incubated in a 5% CO₂ incubator at 37°C.
3) On Day 3 and Day 7, the culture solution was changed twice, and the compound was added according to Step 2.
4) On Day 10, the cell culture plates were taken out; the same volume of CTG reagent (the CTG reagent needed to be restored in temperature for premixing in advance according to the reagent instructions) was added, and gently and evenly mixed on a thermomixer (Hangzhou Allsheng Instrument Co., Ltd., MSC-100) for 10 min (speed: 300); and chemiluminescence detection was conducted using the microplate reader (Thermo, VarioskanLUX).
5) Data analysis was conduct on the measurement results: the CV% of the control group should be less than 20%, and the value z' should be greater than 0.5. Data satisfying the above quality control results were used to calculate the cell viability: (inhibition rate (%) = 100 * (average value of control group - average value of experimental group)/(average value of control group - average value of culture solution control group). GraphPad Prism8 was used to perform nonlinear regression to fit the curves of the inhibition rates of the compounds to obtain IC₅₀ values, with the results shown in Table 3.

After testing, the example compounds of the present application show a strong inhibitory effect on the DLD1 BRCA2-/- cells and a very weak inhibitory effect on the DLD1 wild-type cells, indicating that the example compounds of the present application have good selectivity in inhibiting the proliferation of the two types of cells.

### Data list:

NT = Not detected;

**Table 3: Specific data from biochemical experiments and cell viability assay experiments:**

| Examples | Biochemical experiment IC₅₀ (nM) | IC₅₀ (nM) for cell (DLD1-BRCA2-/-) viability assay | IC₅₀ (nM) for cell (DLD1 wild type) viability assay | Examples | Biochemical experiment IC₅₀ (nM) | IC₅₀ (nM) for cell (DLD1-BRCA2-/-) viability assay | IC₅₀ (nM) for cell (DLD1 wild type) viability assay |
|---|---|---|---|---|---|---|---|
| 1 | 6.92 | 48.2 | >50000 | 18 | 25.1 | 5.62 | 4298 |
| 2 | 5.58 | 41.2 | >50000 | 19 | 32.1 | 56.23 | > 5000 |
| 3 | 10.3 | 79.4 | >50000 | 20 | 8.31 | 22.38 | > 5000 |
| 4 | 20.9 | 43 | >50000 | 21 | 8.57 | 7.64 | > 5000 |
| 5 | 132 | NT | >50000 | 22 | 3.41 | 5.01 | > 5000 |
| 6 | 15.2 | 52.1 | >50000 | 23 | 170 | 185 | >50000 |
| 7 | 4.02 | 56.2 | >50000 | 24 | 20.3 | 15.3 | > 5000 |
| 8 | 8.23 | 56.2 | 19201 | 25 | 42.3 | 242 | > 5000 |
| 9 | 13.4 | 32.9 | >50000 | 26 | 2.29 | 0.82 | > 5000 |
| 10 | 5.81 | 89.1 | >50000 | 27 | 158 | NT | > 5000 |
| 11 | 14.0 | 171 | >50000 | 28 | 11.0 | 24.2 | > 5000 |
| 12 | 24.9 | 25.1 | 23263 | 29 | 244 | NT | > 5000 |
| 13 | 17.6 | 54.1 | >50000 | 30 | 34.1 | NT | > 5000 |
| 14 | 3.63 | 4.82 | >50000 | 31 | 14.4 | NT | > 5000 |
| 15 | 5.58 | 10.79 | > 5000 | 32 | 6.11 | 13.1 | > 5000 |
| 16 | 9.69 | 10.0 | > 5000 | 33 | 4.11 | 8.57 | > 5000 |
| 17 | 8.98 | 3.98 | > 5000 | | | | |

## Claims

1. A compound of Formula I, a pharmaceutically acceptable salt thereof or an isotopic compound thereof, 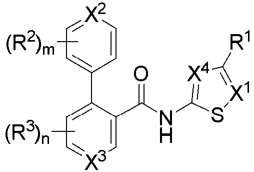 wherein,
m and n are independently 0, 1, 2, 3 or 4;
X¹ and X⁴ are independently N or CR^{a};
X² and X³ are independently N or CH;
each R^{a} is independently hydrogen, halogen, CN, OH, deuterium, cyano, -NHR^{f}, -C(O)NHR^{g}, C₁₋₆ alkyl unsubstituted or substituted by one or more R¹⁻¹, or C₁₋₆ alkoxy unsubstituted or substituted by one or more R¹⁻²;
each of R¹⁻¹ and R¹⁻² is independently halogen;
R¹ is hydrogen, deuterium, cyano, hydroxyl, halogen, amino, -NHC(O)R^{b}, -NHS(O)₂R^{c}, -S(O)₂NHR^{d}, 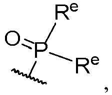 -NHR^{f}, -C(O)NHR^{g}, -OR^{h}, -SRⁱ, -C(O)R^{j}, -S(O)₂R^{k}, -C(O)N(R^{m})₂, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷, or 5-12 membered heteroaryl unsubstituted or substituted by one or more R¹⁻⁸;
each of R^{b}, R^{c}, R^{d}, R^{e}, R^{f}, R^{g}, R^{h}, Rⁱ, R^{j}, R^{k} and R^{m} is independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹², or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently deuterium, halogen, C₁₋₆ alkyl, -S(O)₂C₁₋₆ alkyl or -P(O)(C₁₋₆ alkyl)₂;
each R² is independently deuterium, halogen, cyano, amino, hydroxyl, C₁₋₆ alkyl unsubstituted or substituted by one or more R²⁻¹, C₁₋₆ alkoxy unsubstituted or substituted by one or more R²⁻², C₂₋₆ alkenyl unsubstituted or substituted by one or more R²⁻³, C₂₋₆ alkynyl unsubstituted or substituted by one or more R²⁻⁴, C₃₋₁₀ cycloalkyl unsubstituted or substituted by one or more R²⁻⁵, 3-12 membered heterocycloalkyl unsubstituted or substituted by one or more R²⁻⁶, 4-12 membered heterocycloalkenyl unsubstituted or substituted by one or more R²⁻¹³, -COOR²⁻⁷, -C(O)R²⁻⁸, -C(O)N(R²⁻⁹)₂, 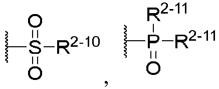 or N(R²⁻¹²)₂;
each of R²⁻¹, R²⁻², R²⁻³, R²⁻⁴, R²⁻⁵, R²⁻⁶ and R²⁻¹³ is independently deuterium, hydroxyl, cyano or halogen;
each of R²⁻⁷, R²⁻⁸, R²⁻⁹ and R²⁻¹² is independently hydrogen or C₁₋₆ alkyl;
each of R²⁻¹⁰ and R²⁻¹¹ is independently hydroxyl or C₁₋₆ alkyl;
each R³ is independently C₁₋₆ alkyl unsubstituted or substituted by one or more R³⁻¹, C₂₋₆ alkenyl unsubstituted or substituted by one or more R³⁻², C₂₋₆ alkynyl unsubstituted or substituted by one or more R³⁻³, C₃₋₆ cycloalkyl unsubstituted or substituted by one or more R³⁻⁴, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, -0-5-12 membered heteroaryl, C₆₋₁₂ aryl unsubstituted or substituted by one or more R³⁻¹⁰, -N(R³⁻⁷)₂ or 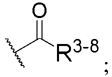
each of R³⁻¹, R³⁻² and R³⁻³ is independently 3-12 membered heterocycloalkyl unsubstituted or substituted by one or more R³⁻¹⁻¹, 5-12 membered heteroaryl unsubstituted or substituted by one or more R³⁻¹⁻² or 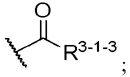
each of R³⁻⁴, R³⁻⁵ and R³⁻⁹ is independently oxo, halogen, cyano, hydroxyl, C₁₋₆ alkyl unsubstituted or substituted by one or more R³⁻⁵⁻¹, C₁₋₆ alkoxy unsubstituted or substituted by one or more R³⁻⁵⁻², C₃₋₆ cycloalkyl unsubstituted or substituted by one or more R³⁻⁵⁻⁵, 3-12 membered heterocycloalkyl unsubstituted or substituted by one or more R³⁻⁵⁻⁶, -N(R³⁻⁵⁻³)₂, 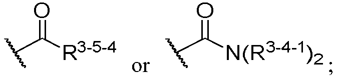
each of R³⁻⁶ and R³⁻¹⁰ is independently oxo, halogen, cyano, hydroxyl, C₁₋₆ alkyl unsubstituted or substituted by one or more R³⁻⁶⁻¹, C₁₋₆ alkoxy unsubstituted or substituted by one or more R³⁻⁵⁻², C₃₋₆ cycloalkyl unsubstituted or substituted by one or more R³⁻⁵⁻⁵, 3-12 membered heterocycloalkyl unsubstituted or substituted by one or more R³⁻⁶⁻², -N(R³⁻⁶⁻³)₂, 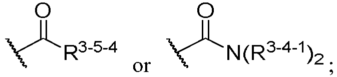
each R³⁻⁷ is independently hydrogen, C₁₋₆ alkyl unsubstituted or substituted by one or more R³⁻⁷⁻¹, 3-12 membered heterocycloalkyl unsubstituted or substituted by one or more R³⁻⁸⁻¹, 5-12 membered heteroaryl unsubstituted or substituted by one or more R³⁻⁷⁻² or 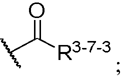
each R³⁻⁸ is independently C₁₋₆ alkyl unsubstituted or substituted by one or more R³⁻⁷⁻¹, 3-12 membered heterocycloalkyl unsubstituted or substituted by one or more R³⁻⁸⁻¹, 5-12 membered heteroaryl unsubstituted or substituted by one or more R³⁻⁷⁻² or 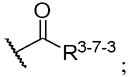
each of R³⁻¹⁻¹ and R³⁻¹⁻² is independently hydroxyl, cyano, halogen or C₁₋₆ alkyl;
each R³⁻¹⁻³ is independently 3-12 membered heterocycloalkyl unsubstituted or substituted by one or more R³⁻¹⁻³⁻¹;
each R³⁻¹⁻³⁻¹ is independently hydroxyl, cyano, halogen or C₁₋₆ alkyl;
each of R³⁻⁴⁻¹, R³⁻⁵⁻³ and R³⁻⁶⁻³ is independently hydrogen or C₁₋₆ alkyl;
each of R³⁻⁵⁻¹, R³⁻⁵⁻² and R³⁻⁷⁻¹ is independently deuterium, hydroxyl, cyano or halogen;
each of R³⁻⁵⁻⁴ and R³⁻⁷⁻³ is independently C₁₋₆ alkyl or C₃₋₆ cycloalkyl;
each of R³⁻⁵⁻⁵, R³⁻⁵⁻⁶ and R³⁻⁶⁻² is independently hydroxyl, cyano, halogen or C₁₋₆ alkyl;
each R³⁻⁶⁻¹ is independently hydroxyl, cyano, halogen or C₁₋₆ alkoxy;
each of R³⁻⁷⁻² and R³⁻⁸⁻¹ is independently hydroxyl, cyano, halogen or C₁₋₆ alkyl; and
a heteroatom in each of the 3-12 membered heterocycloalkyl, the 4-12 membered heterocycloalkenyl, the 5-12 membered heteroaryl and -O-5-12 membered heteroaryl is independently selected from one, two or three of N, O and S, and the number of heteroatom(s) is independently 1, 2 or 3.

2. The compound of Formula I, the pharmaceutically acceptable salt thereof or the isotopic compound thereof according to claim 1, wherein R¹ is hydrogen, deuterium, cyano, hydroxyl, halogen, -NHC(O)R^{b}, -NHS(O)₂R^{c}, -S(O)₂NHR^{d}, 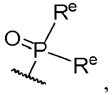 -NHR^{f}, -C(O)NHR^{g}, -OR^{h}, -SRⁱ, -C(O)R^{j}, -S(O)₂R^{k} -C(O)N(R^{m})₂, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷, or 5-12 membered heteroaryl unsubstituted or substituted by one or more R¹⁻⁸;
and/or , each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, -S(O)₂C₁₋₆ alkyl or -P(O)(C₁₋₆ alkyl)₂;
preferably, said compound of Formula I satisfies one or two of the following conditions:
(1) in each R^{a}, said hydrogen is independently fluorine, chlorine, bromine or iodine;
(2) in each R^{a}, C₁₋₆ alkyl in said C₁₋₆ alkyl unsubstituted or substituted by one or more R¹⁻¹ is independently methyl, ethyl, n-propyl, isopropyl, n-butyl, isobutyl, sec-butyl or tert-butyl;
(3) in each R^{a}, C₁₋₆ alkoxy in said C₁₋₆ alkoxy unsubstituted or substituted by one or more R¹⁻² is independently methoxy, ethoxy, n-propoxy, isopropoxy, n-butoxy, isobutoxy, sec-butoxy, or tert-butoxy;
(4) in each of R¹⁻¹ and R¹⁻², said hydrogen is independently fluorine, chlorine, bromine or iodine;
(5) in R¹, said hydrogen is independently fluorine, chlorine, bromine or iodine, for example, fluorine or chlorine, for another example, fluorine;
(6) in R¹, C₁₋₆ alkyl in said C₁₋₆ alkyl unsubstituted or substituted by one or more R¹⁻³ is independently methyl, ethyl, n-propyl, isopropyl, n-butyl, isobutyl, sec-butyl or tert-butyl, for example, methyl, n-propyl, isopropyl or tert-butyl, for another example, methyl, isopropyl or tert-butyl;
(7) in R¹, C₁₋₆ alkoxy in said C₁₋₆ alkoxy unsubstituted or substituted by one or more R¹⁻⁴ is independently methoxy, ethoxy, n-propoxy, isopropoxy, n-butoxy, isobutoxy, sec-butoxy, or tert-butoxy, for example, methoxy;
(8) in R¹, C₃₋₁₀ cycloalkyl in said C₃₋₁₀ cycloalkyl unsubstituted or substituted by one or more R¹⁻⁵ is independently cyclopropyl, cyclobutyl, cyclopentyl or cyclohexyl, for example, cyclopropyl or cyclobutyl, for another example, cyclopropyl;
(9) in R¹, 3-12 membered heterocycloalkyl in said 3-12 membered heterocycloalkyl unsubstituted or substituted by one or more R¹⁻⁶ is independently 5-8 membered heterocycloalkyl;
(10) in R¹, 3-12 membered heterocycloalkyl in said 3-12 membered heterocycloalkyl unsubstituted or substituted by one or more R¹⁻⁶ has a heteroatom that is independently N and/or O;
(11) in R¹, 3-12 membered heterocycloalkyl in said 3-12 membered heterocycloalkyl unsubstituted or substituted by one or more R¹⁻⁶ has the number of heteroatom(s) that is independently 1 or 2, for example, 2;
(12) in R¹, 3-12 membered heterocycloalkyl in said 3-12 membered heterocycloalkyl unsubstituted or substituted by one or more R¹⁻⁶ is independently a monocyclic ring, a bridged ring, a fused ring or a spiro ring; in said spiro ring, fused ring or bridged ring, the number of rings can be 2;
said spiro ring can be 3-membered spiro 5-membered heterocyclyl, 3-membered spiro 6-membered heterocyclyl, 4-membered spiro 4-membered heterocyclyl, 4-membered spiro 5-membered heterocyclyl, 4-membered spiro 6-membered heterocyclyl, 4-membered spiro 7-membered heterocyclyl, 5-membered spiro 4-membered heterocyclyl, 5-membered spiro 5-membered heterocyclyl, 5-membered spiro 6-membered heterocyclyl, 5-membered spiro 7-membered heterocyclyl, 6-membered spiro 4-membered heterocyclyl, 6-membered spiro 5-membered heterocyclyl or 6-membered spiro 6-membered heterocyclyl, for example, 4-membered spiro 4-membered heterocyclyl;
said fused ring can be as set forth in scheme 1 or scheme 2 as follows: in scheme 1: said fused ring is 4-membered fused 5-membered heterocyclyl, 4-membered fused 6-membered heterocyclyl, 5-membered fused 5-membered heterocyclyl, 5-membered fused 6-membered heterocyclyl or 6-membered fused 6-membered heterocyclyl, for example, 5-membered fused 5-membered heterocyclyl; in scheme 2: said fused ring is 3-membered fused 5-membered heterocyclyl, 4-membered fused 5-membered heterocyclyl, 4-membered fused 6-membered heterocyclyl, 5-membered fused 5-membered heterocyclyl, 5-membered fused 6-membered heterocyclyl or 6-membered fused 6-membered heterocyclyl, for example, 3-membered fused 5-membered heterocyclyl or 5-membered fused 5-membered heterocyclyl;
said bridged ring can be 4-membered bridged 5-membered heterocyclyl, 4-membered bridged 6-membered heterocyclyl, 5-membered bridged 6-membered heterocyclyl or 6-membered bridged 6-membered heterocyclyl, for example, 4-membered bridged 6-membered heterocyclyl;
(13) in R¹, C₆₋₁₂ aryl in said C₆₋₁₂ aryl unsubstituted or substituted by one or more R¹⁻⁷ is independently phenyl or naphthyl, for example, phenyl;
(14) in R¹, 5-12 membered heteroaryl in said 5-12 membered heteroaryl unsubstituted or substituted by one or more R¹⁻⁸ is a monocyclic ring;
(15) in R¹, 5-12 membered heteroaryl in said 5-12 membered heteroaryl unsubstituted or substituted by one or more R¹⁻⁸ is independently 5-6 membered heteroaryl;
(16) in R¹, 5-12 membered heteroaryl in said 5-12 membered heteroaryl unsubstituted or substituted by one or more R¹⁻⁸ has a heteroatom that is N and/or O, for example, N;
(17) in R¹, 5-12 membered heteroaryl in said 5-12 membered heteroaryl unsubstituted or substituted by one or more R¹⁻⁸ has the number of heteroatom(s) that is 1 or 2;
(18) in R^{b}, R^{c}, R^{d}, each R^{e}, R^{f}, R^{g}, R^{h}, Rⁱ, R^{j}, R^{k} and R^{m}, C₃₋₁₀ cycloalkyl in said C₃₋₁₀ cycloalkyl unsubstituted or substituted by one or more R¹⁻⁹ is independently cyclopropyl, cyclobutyl, cyclopentyl or cyclohexyl, for example, cyclopropyl;
(19) in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ is independently a monocyclic ring, a bridged ring or a fused ring;
in said fused ring or bridged ring, the number of rings can be 2;
said fused ring can be 4-membered fused 5-membered heterocyclyl, 4-membered fused 6-membered heterocyclyl, 5-membered fused 5-membered heterocyclyl, 5-membered fused 6-membered heterocyclyl or 6-membered fused 6-membered heterocyclyl, for example, 5-membered fused 5-membered heterocyclyl;
said bridged ring can be 4-membered bridged 5-membered heterocyclyl, 4-membered bridged 6-membered heterocyclyl, 5-membered bridged 6-membered heterocyclyl or 6-membered bridged 6-membered heterocyclyl, for example, 4-membered bridged 6-membered heterocyclyl;
(20) in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ is independently 4-8 membered heterocycloalkyl;
(21) in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ has a heteroatom that is independently N and/or O;
(22) in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ has the number of heteroatom(s) that is independently 1 or 2;
(23) in R^{b}, R^{c}, R^{d}, each R^{e}, R^{f}, R^{g}, R^{h}, Rⁱ, R^{j}, R^{k} and R^{m}, C₆₋₁₂ aryl in said C₆₋₁₂ aryl unsubstituted or substituted by one or more R¹⁻¹¹ is independently phenyl or naphthyl, for example, phenyl;
(24) in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² is a monocyclic ring;
(25) in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² is independently 5-6 membered heteroaryl;
(26) in R^{b}, R^{c}, R^{d}, each R^{e}, R^{f}, R^{g}, R^{h}, Rⁱ, R^{j} and R^{k}, 5-12 membered heteroaryl in said 5-12 membered heteroaryl unsubstituted or substituted by one or more R¹⁻¹² has a heteroatom that is N;
(27) in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² has the number of heteroatom(s) that is independently 1 or 2;
(28) in R^{b}, R^{c}, R^{d}, each R^{e}, R^{f}, R^{g}, R^{h}, Rⁱ, R^{j}, R^{k} and R^{m}, C₁₋₆ alkyl in said C₁₋₆ alkyl unsubstituted or substituted by one or more R¹⁻¹³ is independently methyl, ethyl, n-propyl, isopropyl, n-butyl, isobutyl, sec-butyl or tert-butyl, for example, methyl;
(29) in each of R¹⁻³, R¹⁻⁴ R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, said hydrogen is independently fluorine, chlorine, bromine or iodine, for example, fluorine or chlorine;
(30) in each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, each of said C₁₋₆ alkyl, C₁₋₆ alkyl in said -S(O)₂C₁₋₆ alkyl and C₁₋₆ alkyl in said -P(O)(C₁₋₆ alkyl)₂ is independently methyl, ethyl, n-propyl, isopropyl, n-butyl, isobutyl, sec-butyl or tert-butyl, for example, methyl;
(31) in R¹, "more" in each case refers to 2 or 3, for example, 2;
(32) in each R², said hydrogen is independently fluorine, chlorine, bromine or iodine, fluorine or chlorine;
(33) in each R², C₁₋₆ alkyl in said C₁₋₆ alkyl unsubstituted or substituted by one or more R²⁻¹ is independently methyl, ethyl, n-propyl, isopropyl, n-butyl, isobutyl, sec-butyl or tert-butyl, for example, methyl;
(34) in each R², "more" in each case refers to 2 or 3, for example, 2;
(35) in each R², C₁₋₆ alkoxy in said C₁₋₆ alkoxy unsubstituted or substituted by one or more R²⁻² is independently methoxy, ethoxy, n-propoxy, isopropoxy, n-butoxy, isobutoxy, sec-butoxy, or tert-butoxy, for example, methoxy;
(36) in each of R²⁻¹ and R²⁻², said hydrogen is independently fluorine, chlorine, bromine or iodine, for example, fluorine;
(37) in each R³, C₁₋₆ alkyl in said C₁₋₆ alkyl unsubstituted or substituted by one or more R³⁻¹ is independently methyl, ethyl, n-propyl, isopropyl, n-butyl, isobutyl, sec-butyl or tert-butyl, for example, methyl;
(38) in each R³, 4-12 membered heterocycloalkenyl in said 4-12 membered heterocycloalkenyl unsubstituted or substituted by one or more R³⁻⁹ is a monocyclic ring or a fused ring;
in said fused ring, the number of rings can be 2; and
said fused ring can be 4-membered fused 5 membered heterocycloalkenyl, 4-membered fused 6 membered heterocycloalkenyl, 5-membered fused 5 membered heterocycloalkenyl, 5-membered fused 6 membered heterocycloalkenyl or 6-membered fused 6 membered heterocycloalkenyl, for example, 5-membered fused 6 membered heterocycloalkenyl;
(39) in each R³, 4-12 membered heterocycloalkenyl in said 4-12 membered heterocycloalkenyl unsubstituted or substituted by one or more R³⁻⁹ is independently 5-9 membered heterocycloalkenyl, for example, 5-6 membered heterocycloalkenyl;
(40) in each R³, 4-12 membered heterocycloalkenyl in said 4-12 membered heterocycloalkenyl unsubstituted or substituted by one or more R³⁻⁹ has a heteroatom that is N;
(41) in each R³, 4-12 membered heterocycloalkenyl in said 4-12 membered heterocycloalkenyl unsubstituted or substituted by one or more R³⁻⁹ has the number of heteroatom(s) that is 1 or 2, for example, 1;
(42) in each R³, 4-12 membered heterocycloalkenyl in said 4-12 membered heterocycloalkenyl unsubstituted or substituted by one or more R³⁻⁹ has the number of double bond(s) that is 1 or 2, for example, 2;
(43) in each R³, C₂₋₆ alkynyl in said C₂₋₆ alkynyl unsubstituted or substituted by one or more R³⁻³ is independently ethynyl, propinyl or propargyl, for example, ethynyl;
(44) in each R³, 3-12 membered heterocycloalkyl in said 3-12 membered heterocycloalkyl unsubstituted or substituted by one or more R³⁻⁵ is independently a bridged ring or a spiro ring;
in said spiro ring or bridged ring, the number of rings can be 2, and said spiro ring can be 3-membered spiro 5-membered heterocyclyl, 3-membered spiro 6-membered heterocyclyl, 4-membered spiro 4-membered heterocyclyl, 4-membered spiro 5-membered heterocyclyl, 4-membered spiro 6-membered heterocyclyl, 4-membered spiro 7-membered heterocyclyl, 5-membered spiro 4-membered heterocyclyl, 5-membered spiro 5-membered heterocyclyl, 5-membered spiro 6-membered heterocyclyl, 5-membered spiro 7-membered heterocyclyl, 6-membered spiro 4-membered heterocyclyl, 6-membered spiro 5-membered heterocyclyl or 6-membered spiro 6-membered heterocyclyl, for example, 3-membered spiro 6 membered heterocycloalkyl; and said bridged ring can be 4-membered bridged 5 membered heterocycloalkyl, 4-membered bridged 6 membered heterocycloalkyl, 5-membered bridged 6 membered heterocycloalkyl or 6-membered bridged 6 membered heterocycloalkyl, for example, 4-membered bridged 6 membered heterocycloalkyl;
(45) in each R³, 3-12 membered heterocycloalkyl in said 3-12-membered heterocycloalkane unsubstituted or substituted by one or more R³⁻⁵ is independently 6-8 membered heterocycloalkyl;
(46) in each R³, 3-12 membered heterocycloalkyl in said 3-12 membered heterocycloalkyl unsubstituted or substituted by one or more R³⁻⁵ has a heteroatom that is N;
(47) in each R³, 3-12 membered heterocycloalkyl in said 3-12 membered heterocycloalkyl unsubstituted or substituted by one or more R³⁻⁵ has the number of heteroatom(s) that is independently 1 or 2;
(48) in each R³, 3-12 membered heterocycloalkyl in said 3-12 membered heterocycloalkyl unsubstituted or substituted by one or more R³⁻⁵ is 6-8 membered heterocycloalkyl, said 6-8 membered heterocycloalkyl has a heteroatom that is N, and the number of heteroatom(s) is 1 or 2, for example, 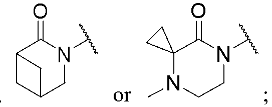
(49) in each R³, C₆₋₁₂ aryl in said C₆₋₁₂ aryl unsubstituted or substituted by one or more R³⁻¹⁰ is phenyl or naphthyl, for example, phenyl;
(50) in each R³, 5-12 membered heteroaryl in said 5-12 membered heteroaryl unsubstituted or substituted by one or more R³⁻⁶ is a monocyclic ring or a fused ring, and
in said fused ring, the number of rings can be 2;
(51) in each R³, 5-12 membered heteroaryl in said 5-12 membered heteroaryl unsubstituted or substituted by one or more R³⁻⁶ is 5-9 membered heteroaryl;
(52) in each R³, 5-12 membered heteroaryl in said 5-12 membered heteroaryl unsubstituted or substituted by one or more R³⁻⁶ has a heteroatom that is N and/or O;
(53) in each R³, "more" in each case refers to 2 or 3, for example, 2;
(54) in each of R³⁻¹ and R³⁻³, 3-12 membered heterocycloalkyl in said 3-12 membered heterocycloalkyl unsubstituted or substituted by one or more R³⁻¹⁻¹ is a monocyclic ring;
(55) in each of R³⁻¹ and R³⁻³, 3-12 membered heterocycloalkyl in said 3-12 membered heterocycloalkyl unsubstituted or substituted by one or more R³⁻¹⁻¹ is 5-6 membered heterocycloalkyl;
(56) in each of R³⁻¹ and R³⁻³, 3-12 membered heterocycloalkyl in said 3-12 membered heterocycloalkyl unsubstituted or substituted by one or more R³⁻¹⁻¹ has a heteroatom that is N and/or O, for example, O;
(57) in each of R³⁻¹ and R³⁻³, 3-12 membered heterocycloalkyl in said 3-12 membered heterocycloalkyl unsubstituted or substituted by one or more R³⁻¹⁻¹ has the number of heteroatom(s) that is 1 or 2, for example, 1;
(58) in each of R³⁻⁵ and R³⁻⁹, said hydrogen is independently fluorine, chlorine, bromine or iodine, for example, fluorine or chlorine;
(59) in each of R³⁻⁵ and R³⁻⁹, C₁₋₆ alkyl in said C₁₋₆ alkyl unsubstituted or substituted by one or more R³⁻⁵⁻¹ is independently methyl, ethyl, n-propyl, isopropyl, n-butyl, sec-butyl, isobutyl or tert-butyl, for example, methyl;
(60) in each of R³⁻⁵ and R³⁻⁹, C₁₋₆ alkyl in said C₁₋₆ alkoxy unsubstituted or substituted by one or more R³⁻⁵⁻¹ is independently methoxy, ethoxy, n-propoxy, isopropoxy, n-butoxy, sec-butoxy, isobutoxy or tert-butoxy, for example, methoxy;
(61) in each of R³⁻⁶ and R³⁻¹⁰, C₁₋₆ alkyl in said C₁₋₆ alkyl unsubstituted or substituted by one or more R³⁻⁶⁻¹ is independently methyl, ethyl, n-propyl, isopropyl, n-butyl, sec-butyl, isobutyl or tert-butyl, for example, methyl;
(62) in each R³⁻⁸, 3-12 membered heterocycloalkyl in said 3-12 membered heterocycloalkyl unsubstituted or substituted by one or more R³⁻⁸⁻¹ is a monocyclic ring;
(63) in each R³⁻⁸, 3-12 membered heterocycloalkyl in said 3-12 membered heterocycloalkyl unsubstituted or substituted by one or more R³⁻⁸⁻¹ is 5-6 membered heterocycloalkyl;
(64) in each R³⁻⁸, 3-12 membered heterocycloalkyl in said 3-12 membered heterocycloalkyl unsubstituted or substituted by one or more R³⁻⁸⁻¹ has a heteroatom that is N and/or O;
(65) in each R³⁻⁸, 3-12 membered heterocycloalkyl in said 3-12 membered heterocycloalkyl unsubstituted or substituted by one or more R³⁻⁸⁻¹ has the number of heteroatom(s) that is 1 or 2, for example, 2;
(66) in each R³⁻¹⁻³, 3-12 membered heterocycloalkyl in said 3-12 membered heterocycloalkyl unsubstituted or substituted by one or more R³⁻¹⁻³⁻¹ is a monocyclic ring;
(67) in each R³⁻¹⁻³, 3-12 membered heterocycloalkyl in said 3-12 membered heterocycloalkyl unsubstituted or substituted by one or more R³⁻¹⁻³⁻¹ is 5-6 membered heterocycloalkyl;
(68) in each R³⁻¹⁻³, 3-12 membered heterocycloalkyl in said 3-12 membered heterocycloalkyl unsubstituted or substituted by one or more R³⁻¹⁻³⁻¹ has a heteroatom that is N and/or O;
(69) in each R³⁻¹⁻³, 3-12 membered heterocycloalkyl in said 3-12 membered heterocycloalkyl unsubstituted or substituted by one or more R³⁻¹⁻³⁻¹ has the number of heteroatom(s) that is 1 or 2, for example, 2; and
(70) in each R³⁻⁵⁻¹, said hydrogen is independently fluorine, chlorine, bromine or iodine, for example, fluorine.

3. The compound of Formula **I,** the pharmaceutically acceptable salt thereof or the isotopic compound thereof according to claim 2, which satisfies one or more of the following conditions:
(1) in R¹, 3-12 membered heterocycloalkyl in said 3-12 membered heterocycloalkyl unsubstituted or substituted by one or more R¹⁻⁶ is independently 5-8 membered heterocycloalkyl, said 5-8 membered heterocycloalkyl has a heteroatom that is N and/or O, and the number of heteroatom(s) is 1 or 2, for example, 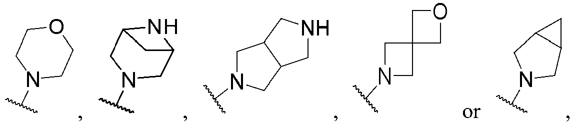 for another example, 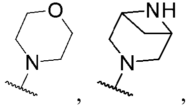 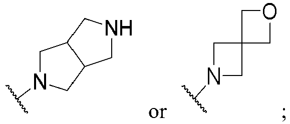
(2) in R¹, 5-12 membered heteroaryl in said 5-12 membered heteroaryl unsubstituted or substituted by one or more R¹⁻⁸ is 5-6 membered heteroaryl, said 5-6 membered heteroaryl has a heteroatom that is N and/or O, and the number of heteroatom(s) is 1 or 2, for example, 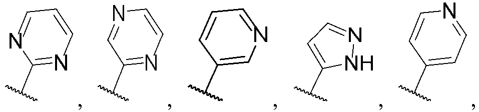 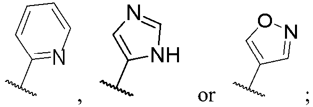 preferably, in R¹, 5-12 membered heteroaryl in said 5-12 membered heteroaryl unsubstituted or substituted by one or more R¹⁻⁸ is 5-6 membered heteroaryl, said 5-6 membered heteroaryl has a heteroatom that is N, and the number of heteroatom(s) is 1 or 2, for example, 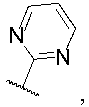 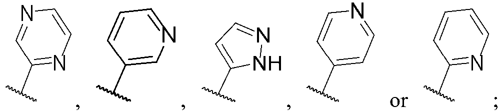
(3) in R^{b}, R^{c}, R^{d}, each R^{e}, R^{f}, R^{g}, R^{h}, Rⁱ, R^{j}, R^{k} and R^{m}, 3-12 membered heterocycloalkyl in said 3-12 membered heterocycloalkyl unsubstituted or substituted by one or more R¹⁻¹⁰ is independently 4-8 membered heterocycloalkyl, said 4-8 membered heterocycloalkyl has a heteroatom that is N and/or O, and the number of heteroatom(s) is 1 or 2, for example, 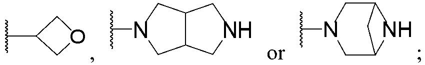
(4) in R^{b}, R^{c}, R^{d}, each R^{e}, R^{f}, R^{g}, R^{h}, Rⁱ, R^{j}, R^{k} and R^{m}, 5-12 membered heteroaryl in said 5-12 membered heteroaryl unsubstituted or substituted by one or more R¹⁻¹² is independently 5-6 membered heteroaryl, said 5-6 membered heteroaryl has a heteroatom that is N, and the number of heteroatom(s) is 1 or 2, for example, 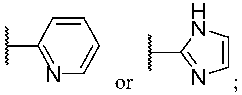
(5) in each R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, said -S(O)₂C₁₋₆ alkyl is 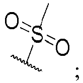
(6) in each R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³, -P(O)(C₁₋₆ alkyl)₂ is 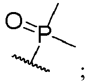
(7) in each R², said C₁₋₆ alkyl substituted by one or more R²⁻¹ is 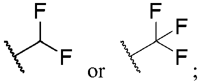
(8) in each R³, 4-12 membered heterocycloalkenylin said 4-12 membered heterocycloalkenyl unsubstituted or substituted by one or more R³⁻⁹ is independently 5-9 membered heterocycloalkenyl, said 5-9 membered heterocycloalkenyl has a heteroatom that is N, the number of heteroatom(s) is 1 or 2, and in said 5-9 membered heterocycloalkenyl, the number of double bond(s) is 1 or 2, for example, 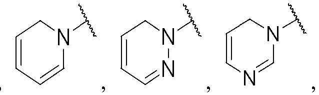 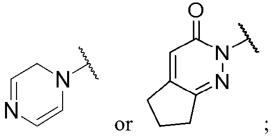
(9) in each R³, 5-12 membered heteroaryl in said 5-12 membered heteroaryl unsubstituted or substituted by one or more R³⁻⁶ is 5-9 membered heteroaryl, said 5-9 membered heteroaryl has a heteroatom that is N and/or O, and the number of heteroatom(s) is 1, 2 or 3, for example, 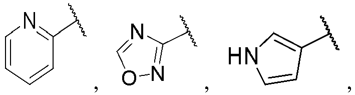 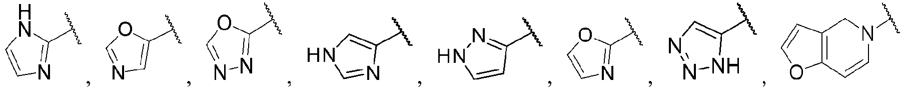 or 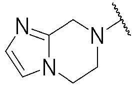 for another example, 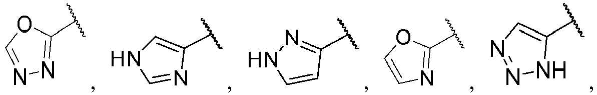 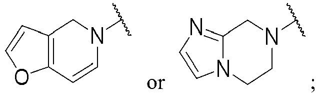
(10) in each of R³⁻¹ and R³⁻³, 3-12 membered heterocycloalkyl in said 3-12 membered heterocycloalkyl unsubstituted or substituted by one or more R³⁻¹⁻¹ is 5-6 membered heterocycloalkyl, said 5-6 membered heterocycloalkyl has a heteroatom that is N and/or O, and the number of heteroatom(s) is 1 or 2, for example, 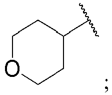
(11) in each R³⁻⁸, 3-12 membered heterocycloalkyl in said 3-12 membered heterocycloalkyl unsubstituted or substituted by one or more R³⁻⁸⁻¹ is 5-6 membered heterocycloalkyl, said 5-6 membered heterocycloalkyl has a heteroatom that is N and/or O, and the number of heteroatom(s) is 1 or 2, for example, 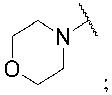
(12) in each R³⁻¹⁻³, 3-12 membered heterocycloalkyl in said 3-12 membered heterocycloalkyl unsubstituted or substituted by one or more R³⁻¹⁻³⁻¹ is 5-6 membered heterocycloalkyl, said 5-6 membered heterocycloalkyl has a heteroatom that is N and/or O, and the number of heteroatom(s) is 1 or 2, for example, 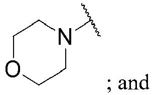
(13) in each of R³⁻⁵ and R³⁻⁹, said C₁₋₆ alkyl substituted by one or more R³⁻⁵⁻¹ is

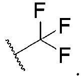

4. The compound of Formula I, the pharmaceutically acceptable salt thereof or the isotopic compound thereof according to any one of claims 1 to 3, which satisfies one or more of the following conditions:
(1) R^{a} is hydrogen;
(2) X² is N;
(3) X³ is N;
(4) R^{b} is as set forth in scheme 1 or scheme 2 as follows: in scheme 1: R^{b} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹²; preferably, R^{b} is C₃₋₁₀ cycloalkyl unsubstituted or substituted by one or more R¹⁻⁹ or C₆₋₁₂ aryl unsubstituted or substituted by one or more R¹⁻¹¹; more preferably, R^{b} is unsubstituted C₃₋₁₀ cycloalkyl or unsubstituted C₆₋₁₂ aryl; in scheme 2: R^{b} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³; preferably, R^{b} is C₃₋₁₀ cycloalkyl unsubstituted or substituted by one or more R¹⁻⁹, C₆₋₁₂ aryl unsubstituted or substituted by one or more R¹⁻¹¹ or C₁₋₆ alkyl unsubstituted or substituted by one or more R¹⁻¹³; more preferably, R^{b} is unsubstituted C₃₋₁₀ cycloalkyl, unsubstituted C₆₋₁₂ aryl or unsubstituted C₁₋₆ alkyl, most preferably, R^{b} is unsubstituted C₁₋₆ alkyl;
(5) R^{c} is C₃₋₁₀ cycloalkyl unsubstituted or substituted by one or more R¹⁻⁹ or 3-12 membered heterocycloalkyl unsubstituted or substituted by one or more R¹⁻¹⁰; preferably, R^{c} is C₃₋₁₀ cycloalkyl unsubstituted or substituted by one or more R¹⁻⁹; more preferably, R^{c} is unsubstituted C₃₋₁₀ cycloalkyl;
(6) R^{d} is C₃₋₁₀ cycloalkyl unsubstituted or substituted by one or more R¹⁻⁹ or 3-12 membered heterocycloalkyl unsubstituted or substituted by one or more R¹⁻¹⁰; preferably, R^{d} is C₃₋₁₀ cycloalkyl unsubstituted or substituted by one or more R¹⁻⁹; more preferably, R^{d} is unsubstituted C₃₋₁₀ cycloalkyl;
(7) R^{e} is C₁₋₆ alkyl unsubstituted or substituted by one or more R¹⁻¹³; preferably, R^{e} is unsubstituted C₁₋₆ alkyl;
(8) R^{f} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹²; preferably, R^{f} is 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹²; more preferably, R^{f} is unsubstituted 3-12 membered heterocycloalkyl, unsubstituted C₆₋₁₂ aryl or unsubstituted 5-12 membered heteroaryl;
(9) R^{g} is C₃₋₁₀ cycloalkyl unsubstituted or substituted by one or more R¹⁻⁹ or 3-12 membered heterocycloalkyl unsubstituted or substituted by one or more R¹⁻¹⁰; preferably, R^{g} is C₃₋₁₀ cycloalkyl unsubstituted or substituted by one or more R¹⁻⁹; more preferably, R^{g} is unsubstituted C₃₋₁₀ cycloalkyl;
(10) R^{h} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹²; preferably, R^{h} is as set forth in scheme 1 or scheme 2 as follows: in scheme 1: R^{h} is 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹²; more preferably, R^{h} is unsubstituted 3-12 membered heterocycloalkyl, unsubstituted C₆₋₁₂ aryl or unsubstituted 5-12 membered heteroaryl; in scheme 2: R^{h} is unsubstituted C₃₋₁₀ cycloalkyl, unsubstituted 3-12 membered heterocycloalkyl or unsubstituted C₆₋₁₂ aryl or unsubstituted 5-12 membered heteroaryl;
(11) Rⁱ is C₃₋₁₀ cycloalkyl unsubstituted or substituted by one or more R¹⁻⁹, 3-12 membered heterocycloalkyl unsubstituted or substituted by one or more R¹⁻¹⁰ or C₆₋₁₂ aryl unsubstituted or substituted by one or more R¹⁻¹¹; preferably, Rⁱ is 3-12 membered heterocycloalkyl unsubstituted or substituted by one or more R¹⁻¹⁰ or C₆₋₁₂ aryl unsubstituted or substituted by one or more R¹⁻¹¹; more preferably, Rⁱ is unsubstituted 3-12 membered heterocycloalkyl or unsubstituted C₆₋₁₂ aryl;
(12) R^{j} is C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹ or 5-12 membered heteroaryl unsubstituted or substituted by one or more R¹⁻¹²; preferably, R^{j} is 3-12 membered heterocycloalkyl unsubstituted or substituted by one or more R¹⁻¹⁰ or 5-12 membered heteroaryl unsubstituted or substituted by one or more R¹⁻¹²; more preferably, R^{j} is unsubstituted 3-12 membered heterocycloalkyl or unsubstituted 5-12 membered heteroaryl;
(13) R^{k} is C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³; preferably, R^{k} is 5-12 membered heteroaryl unsubstituted or substituted by one or more R¹⁻¹² or C₁₋₆ alkyl unsubstituted or substituted by one or more R¹⁻¹³; more preferably, R^{k} is unsubstituted 5-12 membered heteroaryl or unsubstituted C₁₋₆ alkyl;
(14) each R¹⁻³ is independently halogen;
(15) each R¹⁻⁴ is independently halogen;
(16) each R¹⁻⁵ is independently halogen or C₁₋₆ alkyl; preferably, each R¹⁻⁵ is independently C₁₋₆ alkyl;
(17) each R¹⁻⁶ is independently halogen or C₁₋₆ alkyl; preferably, each R¹⁻⁶ is independently C₁₋₆ alkyl;
(18) each R¹⁻⁷ is independently halogen or C₁₋₆ alkyl; preferably, each R¹⁻⁷ is independently halogen;
(19) each R¹⁻⁸ is independently C₁₋₆ alkyl, -S(O)₂C₁₋₆ alkyl or -P(O)(C₁₋₆ alkyl)₂; preferably, R¹⁻⁸ is independently C₁₋₆ alkyl;
(20) R¹ is as set forth in scheme 1 or a scheme 2 as follows: in scheme 1: R¹ is hydrogen, deuterium, cyano, hydroxyl, halogen, -NHC(O)R^{b}, -NHS(O)₂R^{c}, -S(O)₂NHR^{d}, 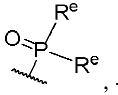 -NHR^{f}, -C(O)NHR^{g}, -OR^{h}, -SRⁱ, - C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷, or 5-12 membered heteroaryl unsubstituted or substituted by one or more R¹⁻⁸;
preferably, R¹ is hydrogen, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷, or 5-12 membered heteroaryl unsubstituted or substituted by one or more R¹⁻⁸;
more preferably, R¹ is hydrogen, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, C₆₋₁₂ aryl unsubstituted or substituted by one or more R¹⁻⁷, or 5-12 membered heteroaryl unsubstituted or substituted by one or more R¹⁻⁸;
most preferably, R¹ is hydrogen, unsubstituted C₁₋₆ alkyl, unsubstituted C₁₋₆ alkoxy, unsubstituted C₃₋₁₀ cycloalkyl, unsubstituted C₆₋₁₂ aryl or unsubstituted 5-12 membered heteroaryl;
in scheme 2: R¹ is hydrogen, deuterium, cyano, hydroxyl, halogen, amino, -NHC(O)R^{b}, -NHS(O)₂R^{c}, - S(O)₂NHR^{d}, 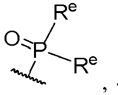 -NHR^{f}, -C(O)NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸; preferably, R¹ is hydrogen, halogen, amino, -NHC(O)R^{b}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸; more preferably, R¹ is hydrogen, halogen, amino, -NHC(O)R^{b}, unsubstituted C₁₋₆ alkyl, unsubstituted C₁₋₆ alkoxy, unsubstituted C₃₋₁₀ cycloalkyl, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸; most preferably, R¹ is hydrogen, unsubstituted C₁₋₆ alkyl, unsubstituted C₁₋₆ alkoxy, unsubstituted C₃₋₁₀ cycloalkyl, unsubstituted 3-12 membered heterocycloalkyl, unsubstituted C₆₋₁₂ aryl, or 5-12 membered heteroaryl unsubstituted or substituted by one or more R¹⁻⁸;
(21) m is 1, 2 or 3; preferably, m is 1 or 2; more preferably, m is 2;
(22) n is 1, 2 or 3; preferably, n is 1;
(23) each of R²⁻¹ and R²⁻² is independently halogen;
(24) each R² is independently halogen, C₁₋₆ alkyl unsubstituted or substituted by one or more R²⁻¹ or C₁₋₆ alkoxy; preferably unsubstituted or substituted by one or more R²⁻², each R² is independently halogen, C₁₋₆ alkyl substituted by one or more R²⁻¹, or unsubstituted C₁₋₆ alkoxy;
(25) each R³⁻¹ is independently 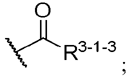
(26) each R³⁻¹⁻³ is independently unsubstituted 3-12 membered heterocycloalkyl;
(27) each R³⁻³ is independently 3-12 membered heterocycloalkyl unsubstituted or substituted by one or more R³⁻¹⁻¹; preferably, each R³⁻³ is independently unsubstituted 3-12 membered heterocycloalkyl;
(28) each R³⁻⁵ is independently oxo or C₁₋₆ alkyl unsubstituted or substituted by one or more R³⁻⁵⁻¹; preferably, each R³⁻⁵ is independently oxo or unsubstituted C₁₋₆ alkyl;
(29) each R³⁻⁶ is independently cyano or C₁₋₆ alkyl unsubstituted or substituted by one or more R³⁻⁶⁻¹; preferably, each R³⁻⁶ is independently cyano or unsubstituted C₁₋₆ alkyl;
(30) each R³⁻¹⁰ is independently hydroxyl;
(31) each R³⁻⁸ is independently 3-12 membered heterocycloalkyl unsubstituted or substituted by one or more R³⁻⁸⁻¹; preferably, each R³⁻⁸ is independently unsubstituted 3-12 membered heterocycloalkyl;
(32) each R³⁻⁹ is independently oxo, halogen, cyano, hydroxyl, C₁-C₆ alkyl unsubstituted or substituted by one or more R³⁻⁵⁻¹ or C₁-C₆ alkoxy unsubstituted or substituted by one or more R³⁻⁵⁻²;
preferably, each R³⁻⁹ is independently oxo, halogen, C₁₋₆ alkyl unsubstituted or substituted by one or more \R³⁻⁵⁻¹, or C₁₋₆ alkoxy unsubstituted or substituted by one or more R³⁻⁵⁻²;
more preferably, each R³⁻⁹ is independently oxo, halogen, C₁₋₆ alkyl substituted by one or more R³⁻⁵⁻¹, or unsubstituted C₁₋₆ alkoxy;
most preferably, each R³⁻⁹ is independently oxo or halogen;
(33) each R³⁻⁵⁻¹ is independently halogen;
(34) each R³ is independently C₁₋₆ alkyl unsubstituted or substituted by one or more R³⁻¹, C₂₋₆ alkynyl unsubstituted or substituted by one or more R³⁻³, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, C₆₋₁₂ aryl unsubstituted or substituted by one or more R³⁻¹⁰ or 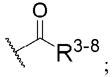
preferably, each R³ is independently C₁₋₆ alkyl unsubstituted or substituted by one or more R³⁻¹, C₃₋₆ cycloalkyl unsubstituted or substituted by one or more R³⁻⁴, 3-12 membered heterocycloalkyl unsubstituted or substituted by one or more R³⁻⁵ or 4-12 membered heterocycloalkenyl unsubstituted or substituted by one or more R³⁻⁹;
more preferably, each R³ is independently C₁₋₆ alkyl unsubstituted or substituted by one or more R³⁻¹ or 4-12 membered heterocycloalkenyl unsubstituted or substituted by one or more R³⁻⁹;
most preferably, each R³ is independently unsubstituted C₁₋₆ alkyl or 4-12 membered heterocycloalkenyl substituted by one or more R³⁻⁹, for example, 4-12 membered heterocycloalkenyl substituted by one or more R³⁻⁹; and
(35) X⁴ is N.

5. The compound of Formula I, the pharmaceutically acceptable salt thereof or the isotopic compound thereof according to any one of claims 1 to 4, wherein said compound of Formula I is as set forth in scheme 1, scheme 2, scheme 3, scheme 4, scheme 5, scheme 6 or scheme 7 as follows:
in scheme 1: m and n are independently 1, 2 or 3;
X¹ is N or CH;
X² is N or CH;
X³ is N;
X⁴ is N or CH;
R¹ is hydrogen, deuterium, cyano, hydroxyl, halogen, -NHC(O)R^{b}, -NHS(O)₂R^{c}, -S(O)₂NHR^{d}, 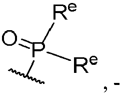 NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸;
R^{b}, R^{c}, R^{d}, each R^{e}, R^{f}, R^{g}, R^{h}, Rⁱ, R^{j} and R^{k} are each independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹² or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, - S(O)₂C₁₋₆ alkyl or -P(O)(C₁₋₆ alkyl)₂;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently C₁₋₆ alkyl unsubstituted or substituted by one or more R³⁻¹, C₂₋₆ alkynyl unsubstituted or substituted by one or more R³⁻³, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, C₆₋₁₂ aryl unsubstituted or substituted by one or more R³⁻¹⁰ or 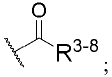
each R³⁻¹ is independently 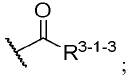
each R³⁻³ is independently 3-12 membered heterocycloalkyl unsubstituted or substituted by one or more R³⁻¹⁻¹;
each R³⁻⁵ is independently oxo or C₁₋₆ alkyl unsubstituted or substituted by one or more R³⁻⁵⁻¹;
each R³⁻⁶ is independently cyano or C₁₋₆ alkyl unsubstituted or substituted by one or more R³⁻⁶⁻¹;
each R³⁻⁹ is independently oxo, halogen, C₁₋₆ alkyl unsubstituted or substituted by one or more R³⁻⁵⁻¹ or C₁₋₆ alkoxy unsubstituted or substituted by one or more R³⁻⁵⁻²;
each R³⁻⁸ is independently 3-12 membered heterocycloalkyl unsubstituted or substituted by one or more R³⁻⁸⁻¹;
each R³⁻¹⁰ is independently hydroxyl;
each R³⁻¹⁻¹ is independently hydroxyl, cyano, halogen or C₁₋₆ alkyl;
each R³⁻¹⁻³ is independently unsubstituted 3-12 membered heterocycloalkyl;
each of R³⁻⁵⁻¹ and R³⁻⁵⁻² is independently deuterium, hydroxyl, cyano or halogen;
each R³⁻⁶⁻¹ is independently hydroxyl, cyano, halogen or C₁₋₆ alkoxy;
each R³⁻⁸⁻¹ is independently hydroxyl, cyano, halogen or C₁₋₆ alkyl;
each of 3-12 membered heterocycloalkyl, 4-12 membered heterocycloalkenyl and 5-12 membered heteroaryl has a heteroatom that is independently selected from one, two or three of N, O and S, and the number of heteroatom(s) is independently 1, 2 or 3;
in scheme 2: m is 1 or 2;
n is 1;
X¹ is N or CH;
X² is N or CH;
X³ is N;
X⁴ is N;
R¹ is hydrogen, deuterium, cyano, hydroxyl, halogen, -NHC(O)R^{b}, -NHS(O)₂R^{c}, -S(O)₂NHR^{d}, 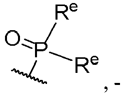 NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷, or 5-12 membered heteroaryl unsubstituted or substituted by one or more R¹⁻⁸;
R^{b}, R^{c}, R^{d}, each R^{e}, R^{f}, R^{g}, R^{h}, Rⁱ, R^{j} and R^{k} are each independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹², or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, - S(O)₂C₁₋₆ alkyl or -P(O)(C₁₋₆ alkyl)₂;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently unsubstituted C₁₋₆ alkyl or 4-12 membered heterocycloalkenyl unsubstituted or substituted by one or more R³⁻⁹;
each R³⁻⁹ is independently oxo or halogen;
each of 3-12 membered heterocycloalkyl, 4-12 membered heterocycloalkenyl and 5-12 membered heteroaryl has a heteroatom that is independently selected from one, two or three of N, O and S, and the number of heteroatom(s) is independently 1, 2 or 3;
in scheme 3: m is 1 or 2;
n is 1;
X¹ is N or CH;
X², X³ and X⁴ are N;
R¹ is hydrogen, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, C₆₋₁₂ aryl unsubstituted or substituted by one or more R¹⁻⁷, or 5-12 membered heteroaryl unsubstituted or substituted by one or more R¹⁻⁸;
each of R¹⁻³ and R¹⁻⁴ is independently halogen;
each of R¹⁻⁵, R¹⁻⁶, R¹⁻⁷ and R¹⁻⁸ is independently halogen or C₁₋₆ alkyl;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently unsubstituted C₁₋₆ alkyl or 4-12 membered heterocycloalkenyl unsubstituted or substituted by one or more R³⁻⁹;
each R³⁻⁹ is independently oxo or halogen;
each of 4-12 membered heterocycloalkenyl and 5-12 membered heteroaryl has a heteroatom that is independently selected from one, two or three of N, O and S, and the number of heteroatom(s) is independently 1, 2 or 3;
in scheme 4: m and n are independently 1, 2 or 3;
X¹ is N or CH;
X² is N or CH;
X³ is N;
X⁴ is N or CH;
R¹ is hydrogen, deuterium, cyano, hydroxyl, halogen, amino, -NHC(O)R^{b}, -NHS(O)₂R^{c}, -S(O)₂NHR^{d}, 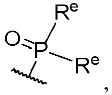 -NHR^{f}, -C(O) NHR^{g}, -OR^{h}, -SRⁱ, -C(O)R^{j}, -S(O)₂R^{k}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷, or 5-12 membered heteroaryl unsubstituted or substituted by one or more R¹⁻⁸;
R^{b}, R^{c}, R^{d}, each R^{e}, R^{f}, R^{g}, R^{h}, Rⁱ, R^{j} and R^{k} are each independently C₃₋₁₀ cycloalkyl unsubstituted or substituted by one or more R¹⁻⁹, 3-12 membered heterocycloalkyl unsubstituted or substituted by one or more R¹⁻¹⁰, C₆₋₁₂ aryl unsubstituted or substituted by one or more R¹⁻¹¹, 5-12 membered heteroaryl unsubstituted or substituted by one or more R¹⁻¹², or C₁₋₆ alkyl unsubstituted or substituted by one or more R¹⁻¹³;
each of R¹⁻³, R¹⁻⁴, R¹⁻⁵, R¹⁻⁶, R¹⁻⁷, R¹⁻⁸, R¹⁻⁹, R¹⁻¹⁰, R¹⁻¹¹, R¹⁻¹² and R¹⁻¹³ is independently halogen, C₁₋₆ alkyl, - S(O)₂C₁₋₆ alkyl or -P(O)(C₁₋₆ alkyl)₂;
each R² is independently halogen, C₁₋₆ alkyl unsubstituted or substituted by one or more R²⁻¹, or C₁₋₆ alkoxy unsubstituted or substituted by one or more R²⁻²;
each of R²⁻¹ and R²⁻² is independently halogen;
each R³ is independently C₁₋₆ alkyl unsubstituted or substituted by one or more R³⁻¹, C₂₋₆ alkynyl unsubstituted or substituted by one or more R³⁻³, 3-12 membered heterocycloalkyl unsubstituted or substituted by one or more R³⁻⁵, 4-12 membered heterocycloalkenyl unsubstituted or substituted by one or more R³⁻⁹, 5-12 membered heteroaryl unsubstituted or substituted by one or more R³⁻⁶, C₆₋₁₂ aryl unsubstituted or substituted by one or more R³⁻¹⁰ or 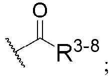
each R³⁻¹ is independently 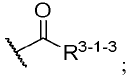
each R³⁻³ is independently 3-12 membered heterocycloalkyl unsubstituted or substituted by one or more R³⁻¹⁻¹;
each R³⁻⁵ is independently oxo or C₁₋₆ alkyl unsubstituted or substituted by one or more R³⁻⁵⁻¹;
each R³⁻⁶ is independently cyano or C₁₋₆ alkyl unsubstituted or substituted by one or more R³⁻⁶⁻¹;
each R³⁻⁹ is independently oxo, halogen, C₁₋₆ alkyl unsubstituted or substituted by one or more R³⁻⁵⁻¹ or C₁₋₆ alkoxy unsubstituted or substituted by one or more R³⁻⁵⁻²;
each R³⁻⁸ is independently 3-12 membered heterocycloalkyl unsubstituted or substituted by one or more R³⁻⁸⁻¹;
each R³⁻¹⁰ is independently hydroxyl;
each R³⁻¹⁻¹ is independently hydroxyl, cyano, halogen or C₁₋₆ alkyl;
each R³⁻¹⁻³ is independently unsubstituted 3-12 membered heterocycloalkyl;
each of R³⁻⁵⁻¹ and R³⁻⁵⁻² is independently deuterium, hydroxyl, cyano or halogen;
each R³⁻⁶⁻¹ is independently hydroxyl, cyano, halogen or C₁₋₆ alkoxy;
each R³⁻⁸⁻¹ is independently hydroxyl, cyano, halogen or C₁₋₆ alkyl;
each of 3-12 membered heterocycloalkyl, 4-12 membered heterocycloalkenyl and 5-12 membered heteroaryl has a heteroatom that is independently selected from one, two or three of N, O and S, and the number of heteroatom(s) is independently 1, 2 or 3;
in scheme 5: m is 1 or 2;
n is 1;
X¹ is N or CH;
X², X³ and X⁴ are N;
R¹ is hydrogen, halogen, amino, -NHC(O)R^{b}, C₁₋₆ alkyl unsubstituted or substituted by one or more R¹⁻³, C₁₋₆ alkoxy unsubstituted or substituted by one or more R¹⁻⁴, C₃₋₁₀ cycloalkyl unsubstituted or substituted by one or more R¹⁻⁵, 3-12 membered heterocycloalkyl unsubstituted or substituted by one or more R¹⁻⁶, C₆₋₁₂ aryl unsubstituted or substituted by one or more R¹⁻⁷ or 5-12 membered heteroaryl unsubstituted or substituted by one or more R¹⁻⁸;
each of R¹⁻³ and R¹⁻⁴ is independently halogen;
each of R¹⁻⁵, R¹⁻⁶, R¹⁻⁷ and R¹⁻⁸ is independently halogen or C₁₋₆ alkyl;
R^{b} is unsubstituted C₁₋₆ alkyl;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
each R³ is independently unsubstituted C₁₋₆ alkyl or 4-12 membered heterocycloalkenyl unsubstituted or substituted by one or more R³⁻⁹;
each R³⁻⁹ is independently oxo or halogen;
each of 4-12 membered heterocycloalkenyl and 5-12 membered heteroaryl has a heteroatom that is independently selected from one, two or three of N, O and S, and the number of heteroatom(s) is independently 1, 2 or 3;
in scheme 6: m is 1 or 2;
n is 1;
X¹ is N or CH;
X², X³ and X⁴ are N;
R¹ is hydrogen, unsubstituted C₁₋₆ alkyl, unsubstituted C₁₋₆ alkoxy, unsubstituted C₃₋₁₀ cycloalkyl, unsubstituted 3-12 membered heterocycloalkyl, unsubstituted C₆₋₁₂ aryl or 5-12 membered heteroaryl unsubstituted or substituted by one or more R¹⁻⁸;
each R¹⁻⁸ is independently C₁₋₆ alkyl;
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
R³ is 4-12 membered heterocycloalkenyl unsubstituted or substituted by one or more R³⁻⁹;
each R³⁻⁹ is independently oxo or halogen;
each of 4-12 membered heterocycloalkenyl and 5-12 membered heteroaryl has a heteroatom that is independently selected from one, two or three of N, O and S, and the number of heteroatom(s) is independently 1, 2 or 3;
in scheme 7: m is 1 or 2;
n is 1;
X² and X³ are N; 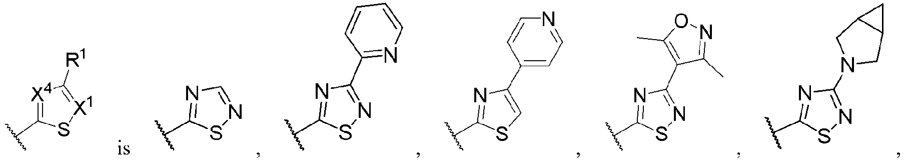 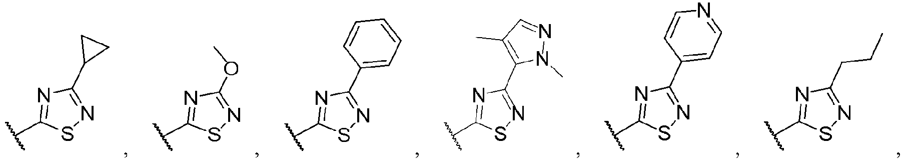 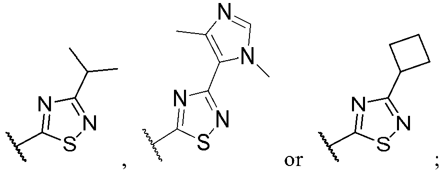
each R² is independently halogen, C₁₋₆ alkyl unsubstituted or substituted by one or more R²⁻¹ or C₁₋₆ alkoxy unsubstituted or substituted by one or more R²⁻²;
each of R²⁻¹ and R²⁻² is independently halogen;
R³ is 4-12 membered heterocycloalkenyl unsubstituted or substituted by one or more R³⁻⁹;
each R³⁻⁹ is independently oxo or halogen; and
each 4-12 membered heterocycloalkenyl has a heteroatom that is independently selected from one, two or three of N, O and S, and the number of heteroatom(s) is independently 1, 2 or 3.

6. The compound of Formula I, the pharmaceutically acceptable salt thereof or the isotopic compound thereof according to any one of claims 1 to 5 , which satisfies one or more of the following conditions:
(1) R¹ is as set forth in scheme 1 or scheme 2 as follows: in scheme 1: R¹ is hydrogen, deuterium, cyano, hydroxyl, fluorine, methyl, 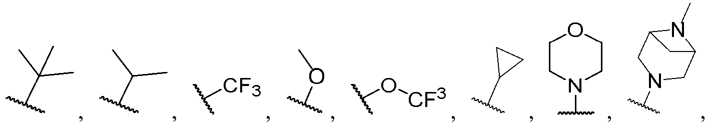 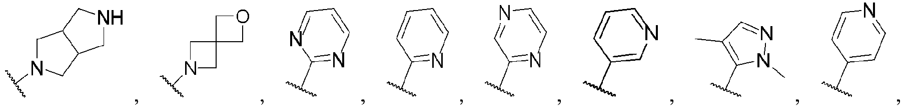 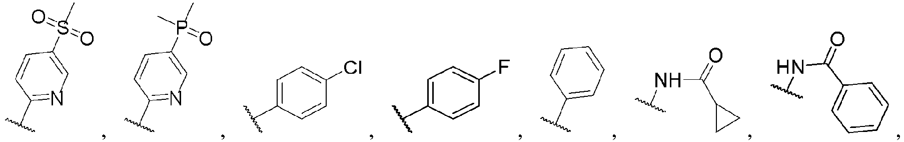 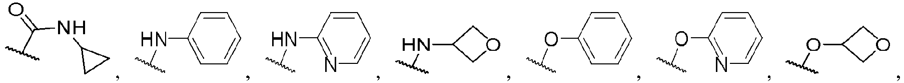 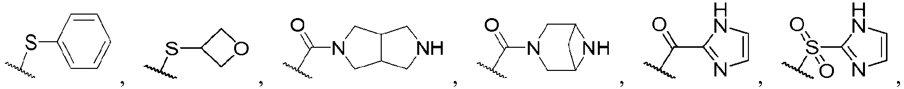 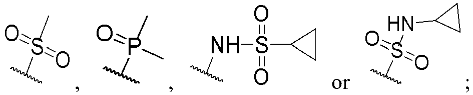 preferably, R¹ is hydrogen, methyl, 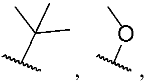 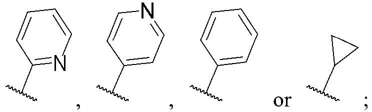 in scheme 2: R¹ is hydrogen, deuterium, cyano, hydroxyl, fluorine, methyl, 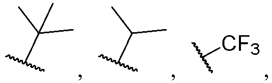 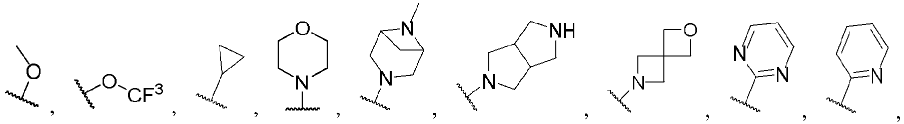 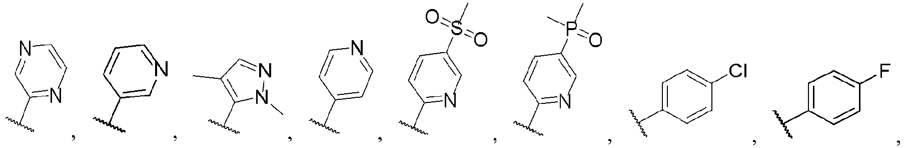 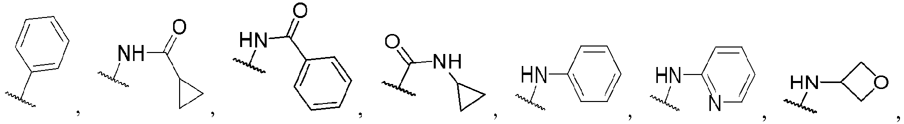 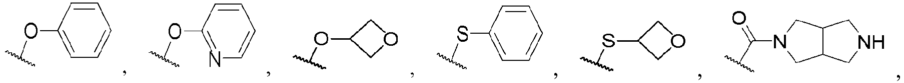 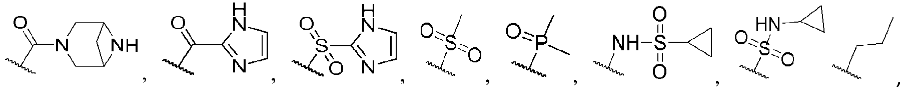 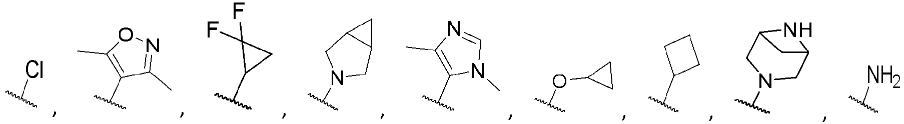 or 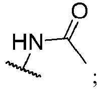 preferably, R¹ is hydrogen, methyl, 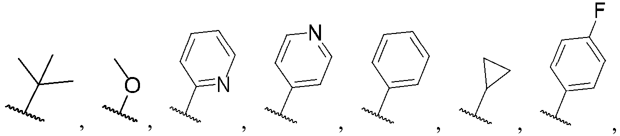 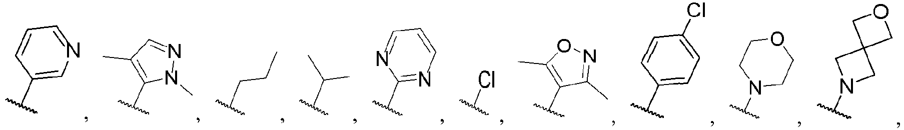 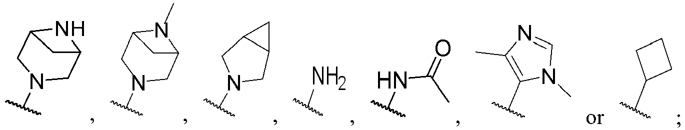
(2) R² is 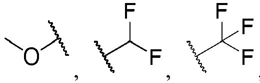 F or Cl; and
(3) R³ is methyl, 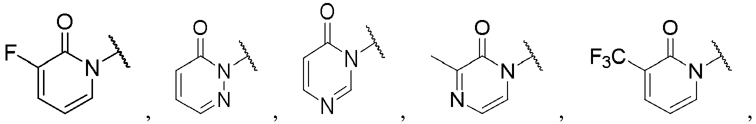 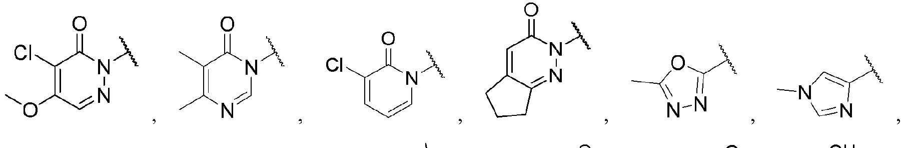 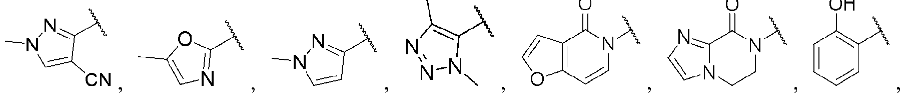 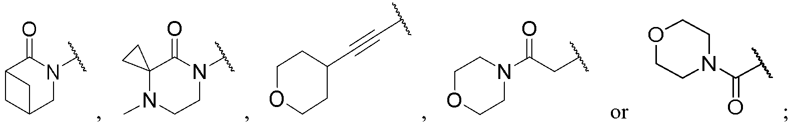 preferably, R³ is 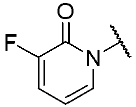 or methyl, for example,

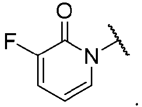

7. The compound of Formula I, the pharmaceutically acceptable salt thereof or the isotopic compound thereof according to claim 6, which satisfies one or more of the following conditions:
(1) 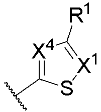 is as set forth in scheme 1 or scheme 2 as follows, in scheme 1 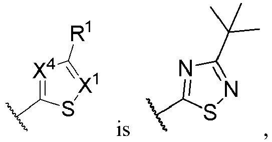 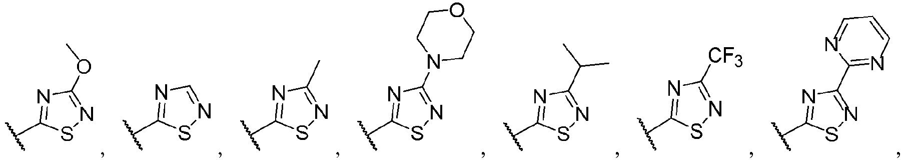 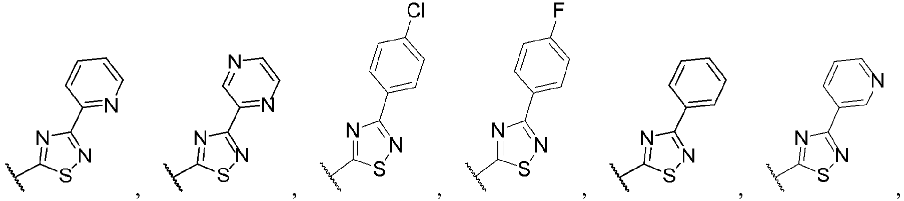 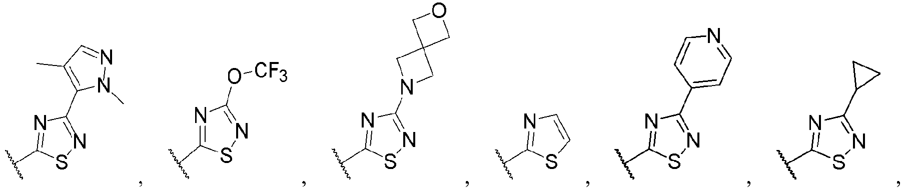 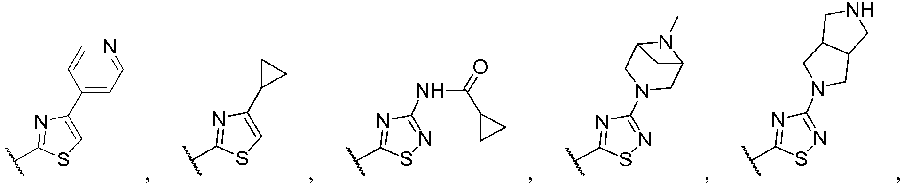 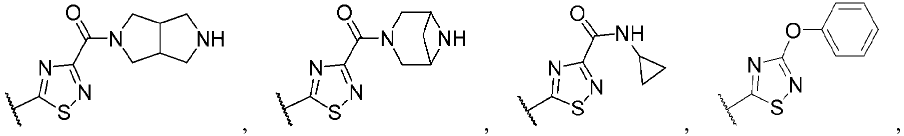 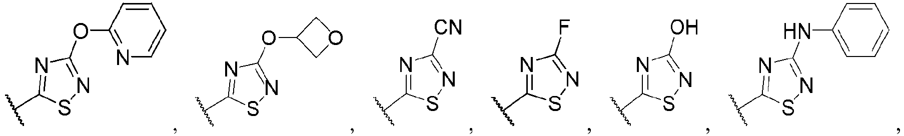 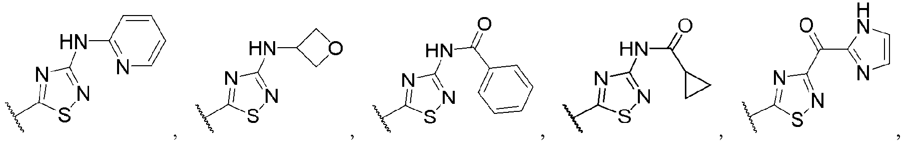 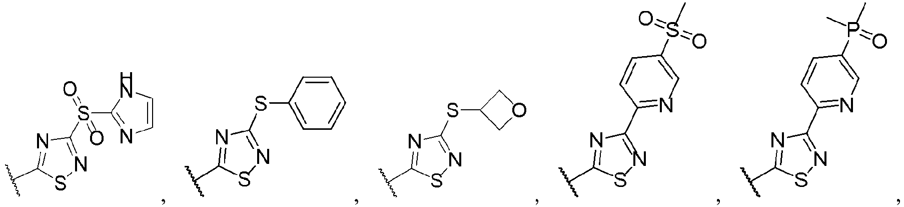 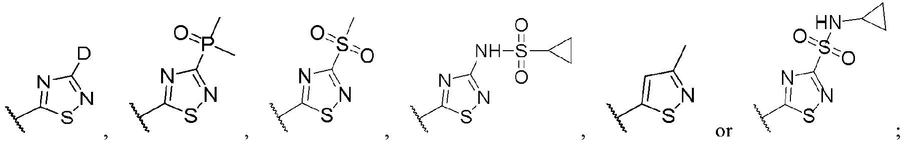 preferably, 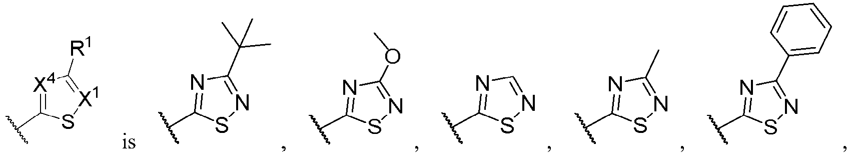 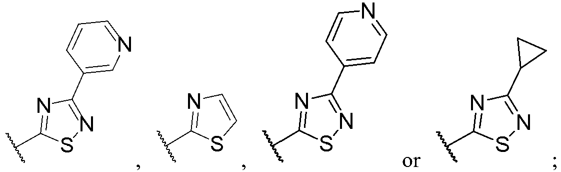 in scheme 2: 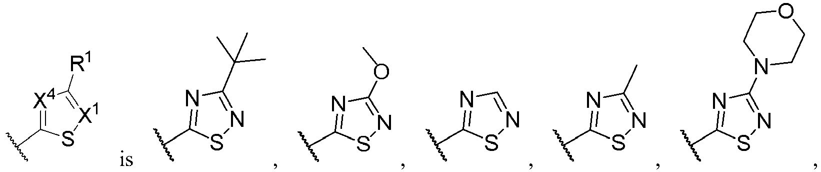 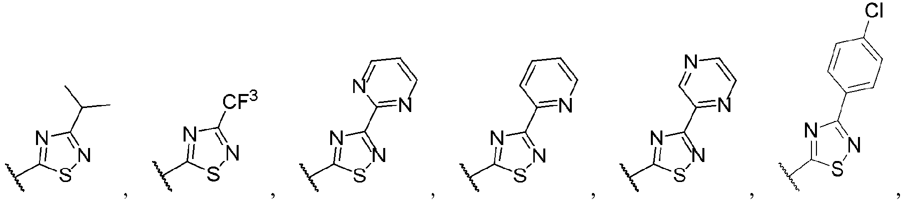 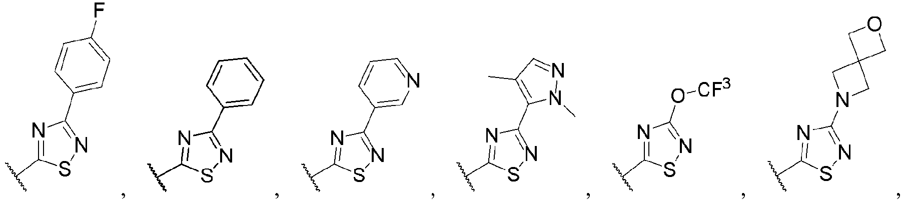 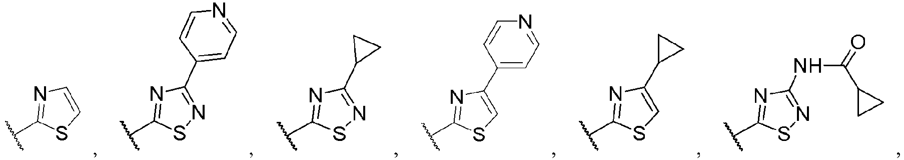 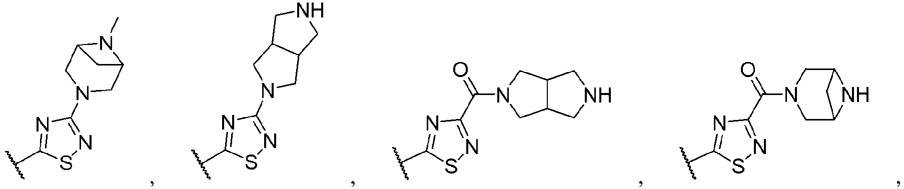 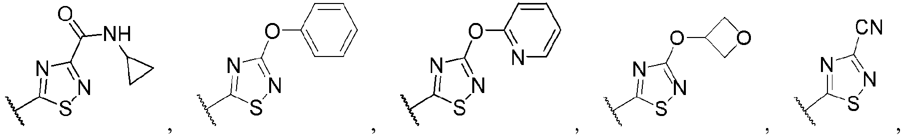 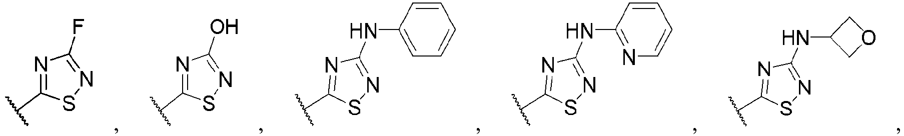 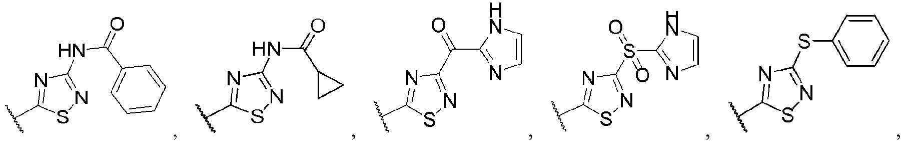 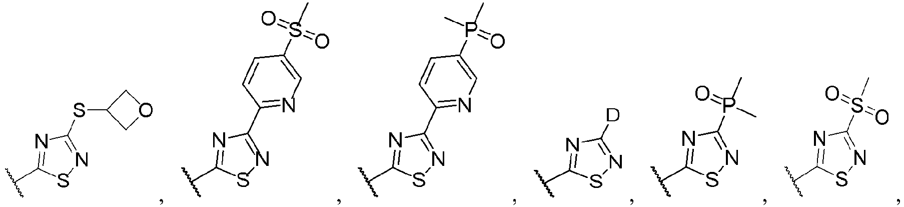 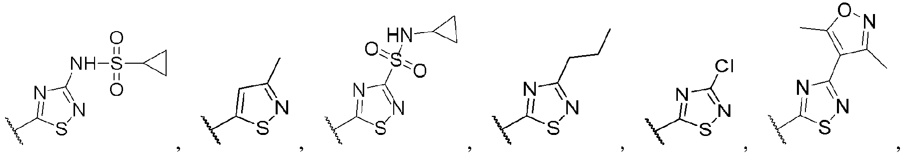 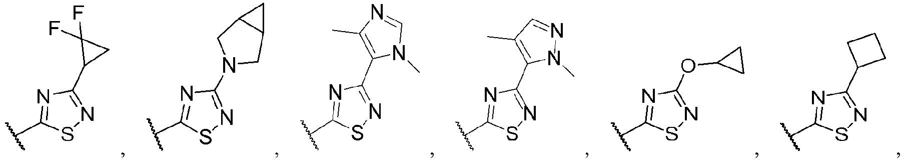 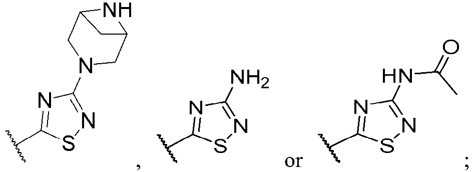 preferably, 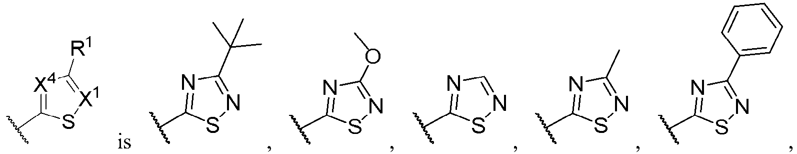 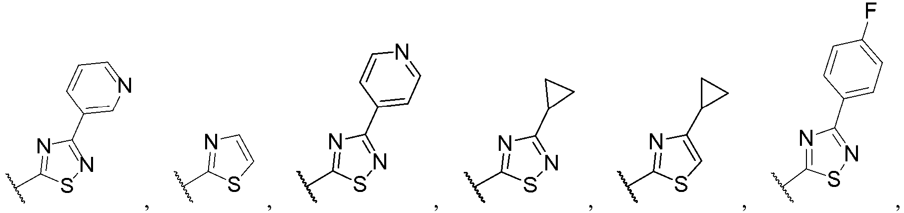 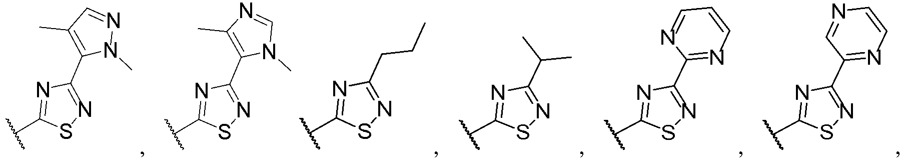 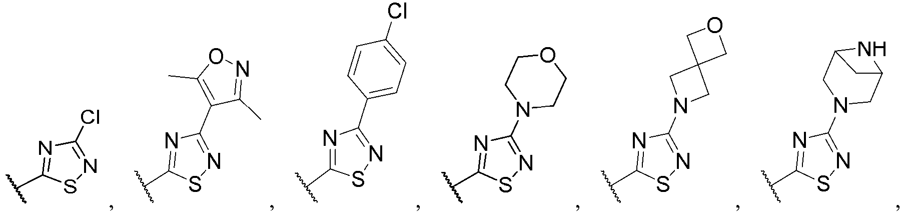 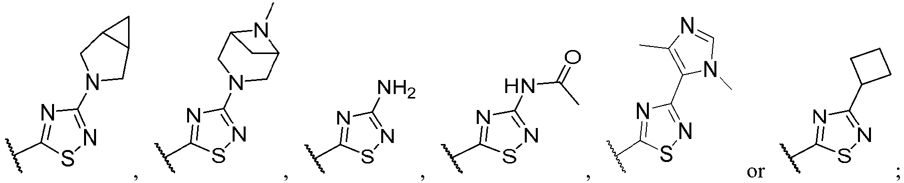 more preferably, 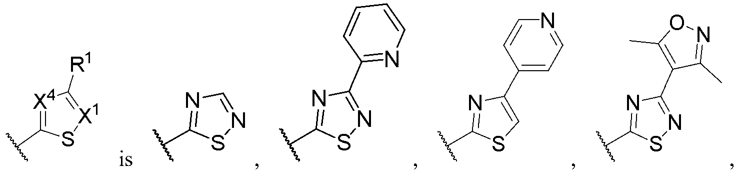 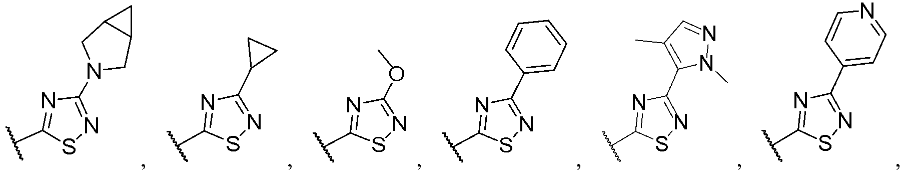 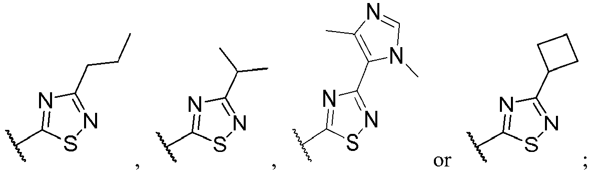 most preferably, 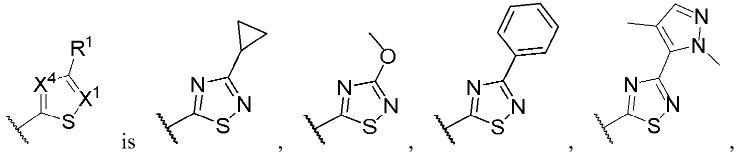 and 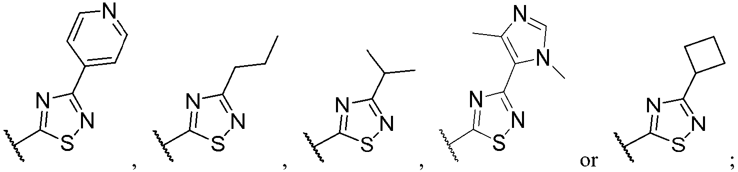
(2) 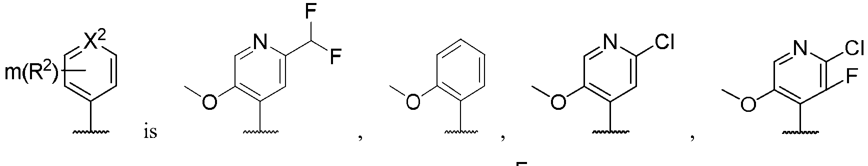 or 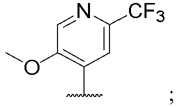 preferably,

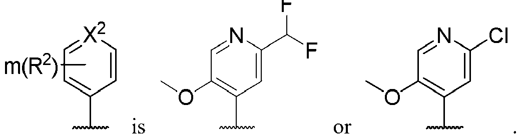

8. The compound of Formula I, the pharmaceutically acceptable salt thereof or the isotopic compound thereof according to claim 1, wherein said compound of Formula I is any one of the following compounds: 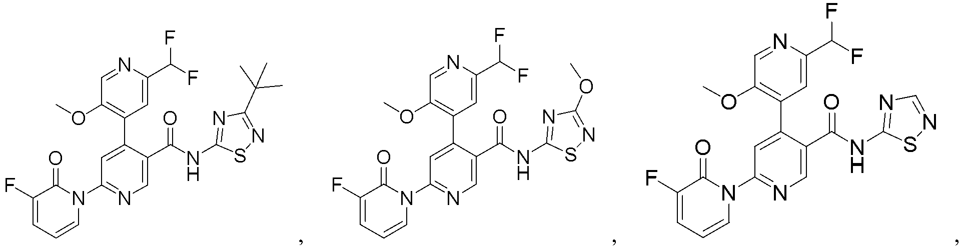 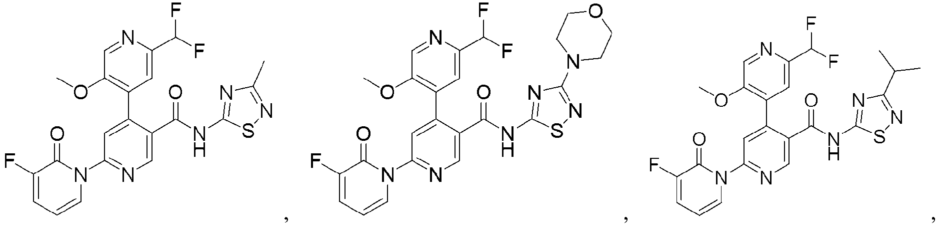 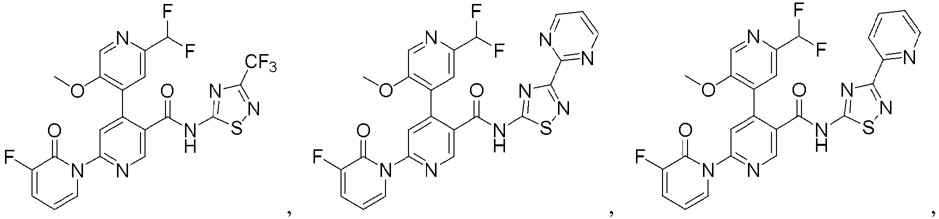 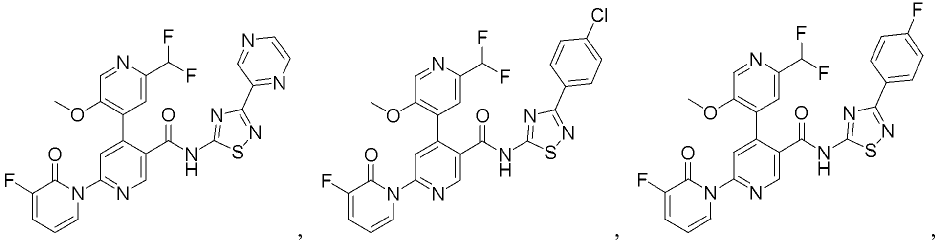 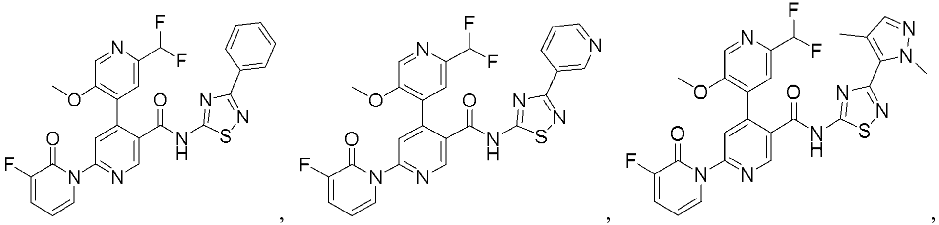 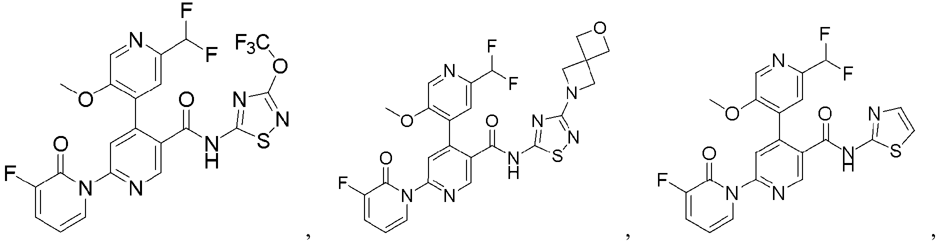 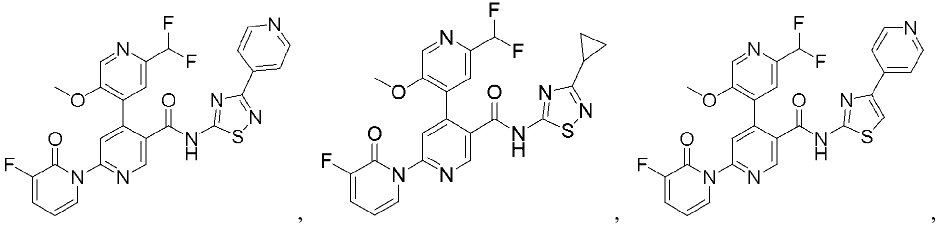 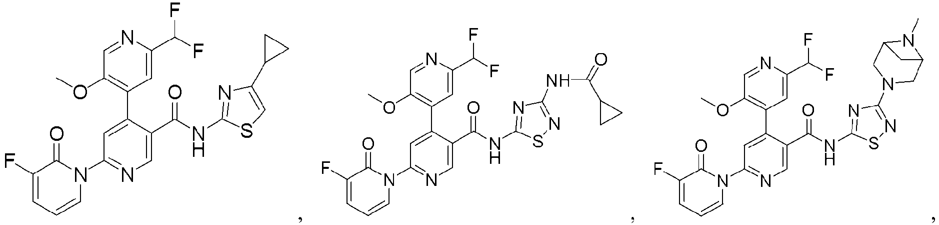 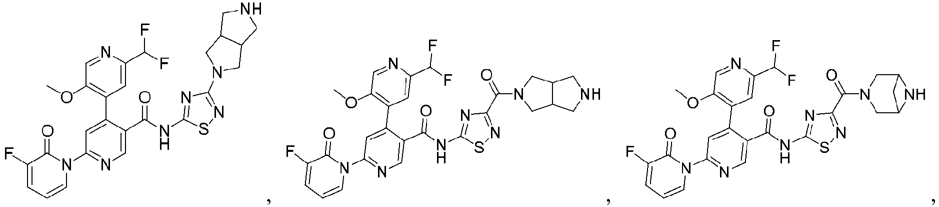 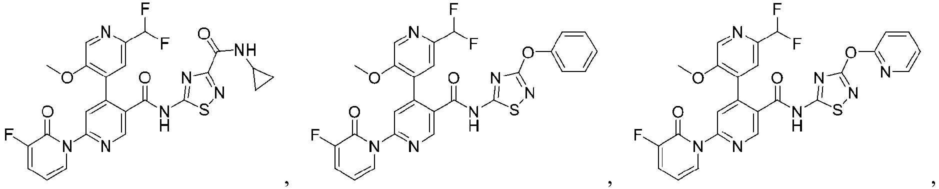 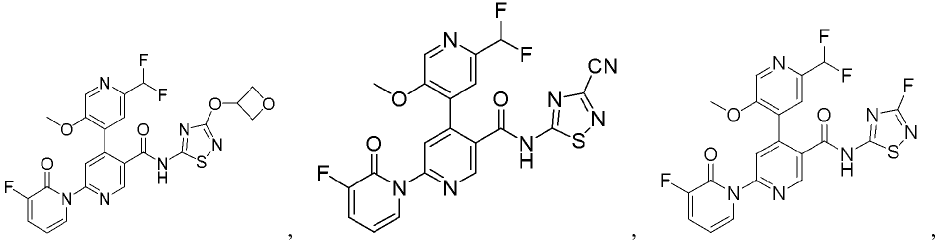 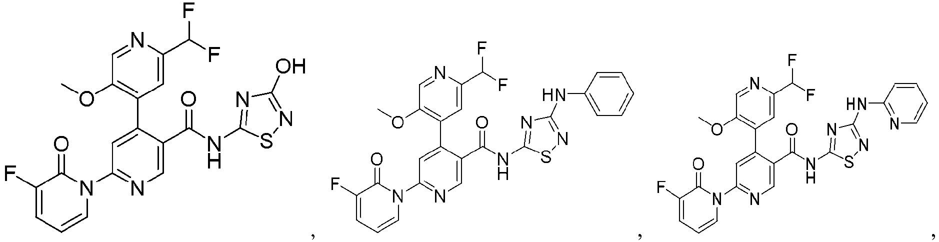 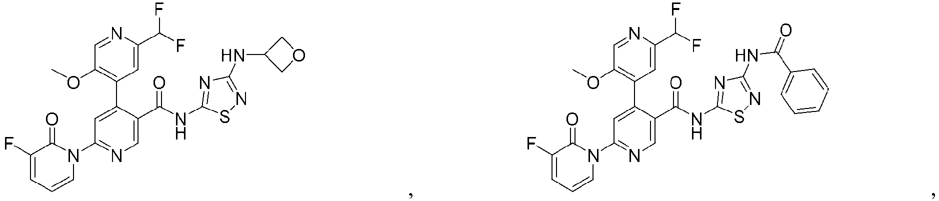 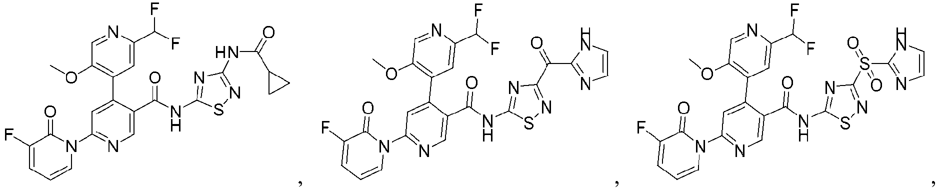 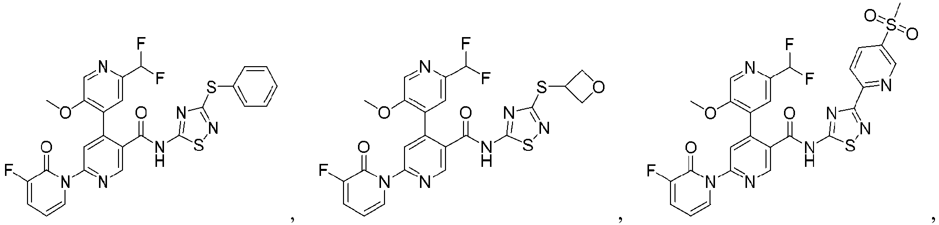 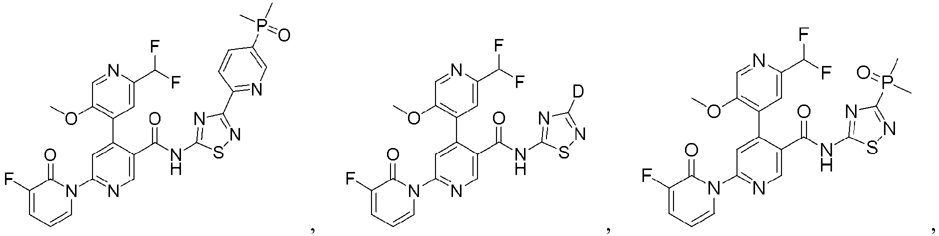 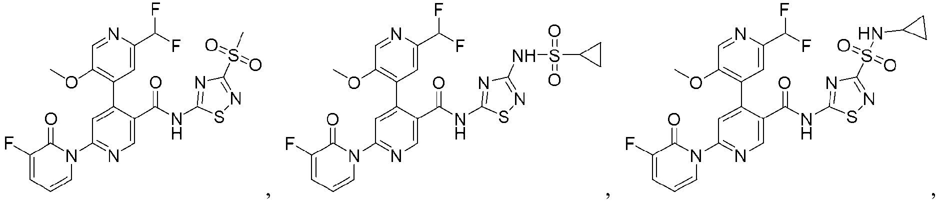 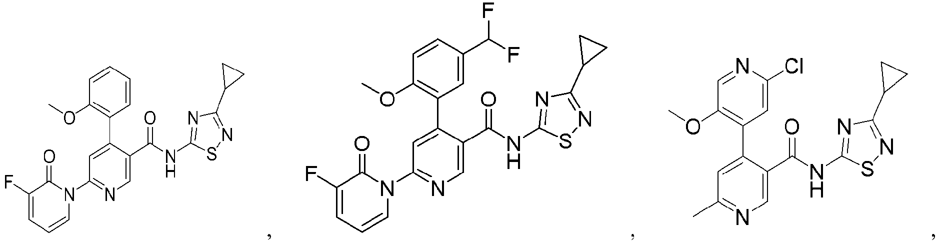 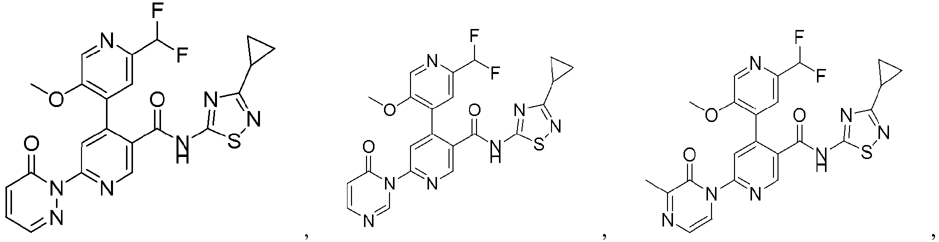 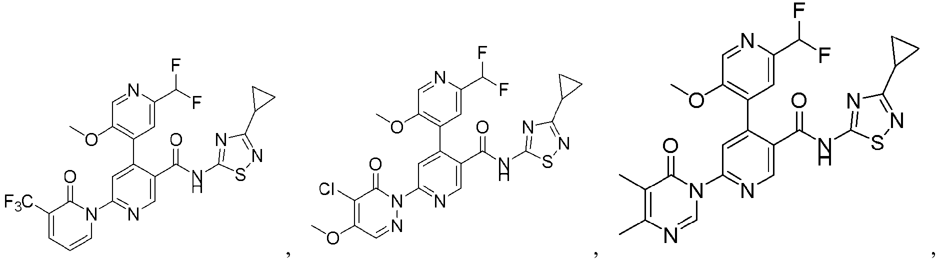 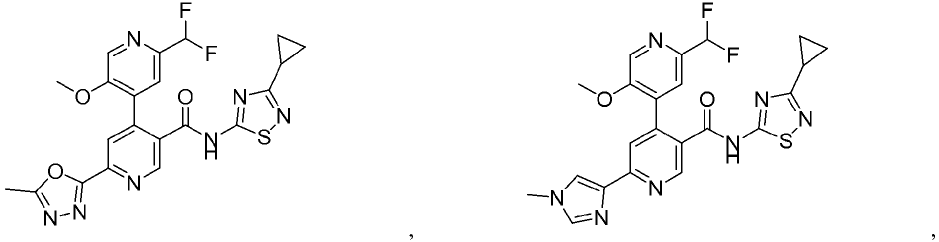 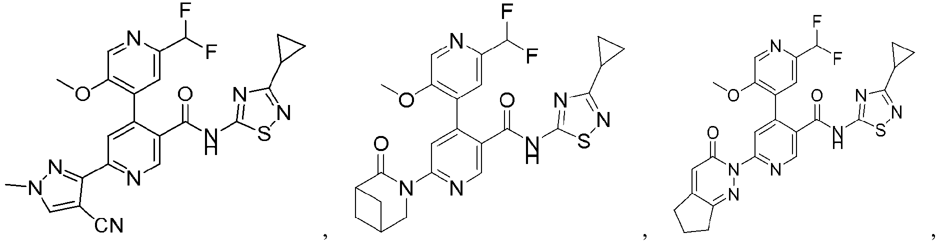 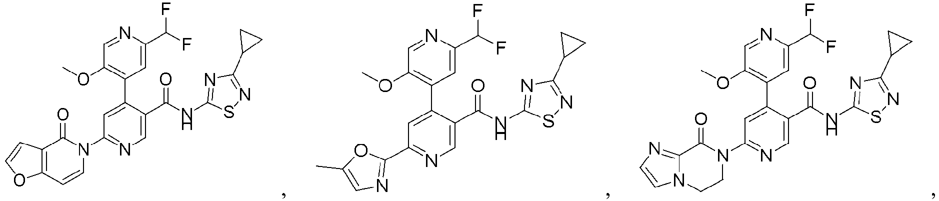 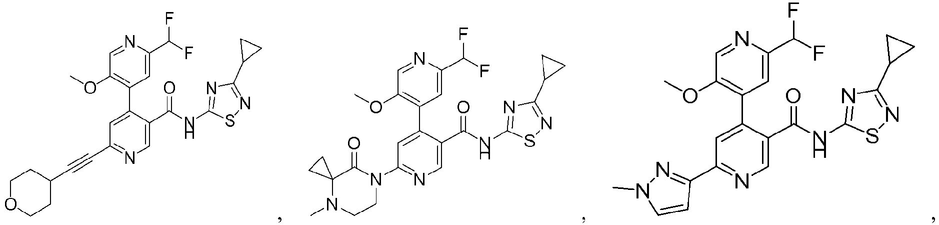 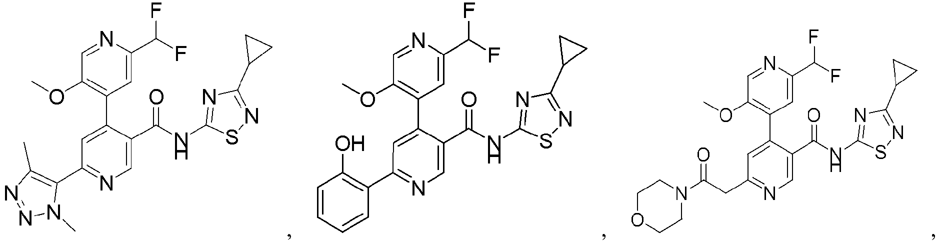 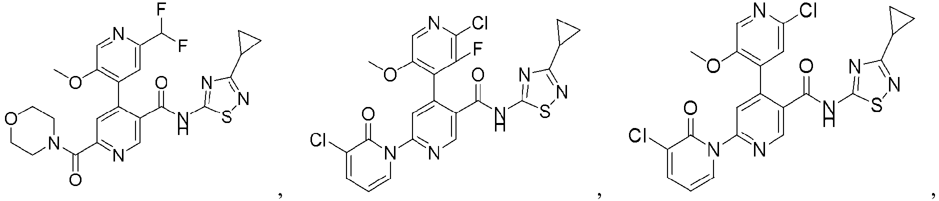 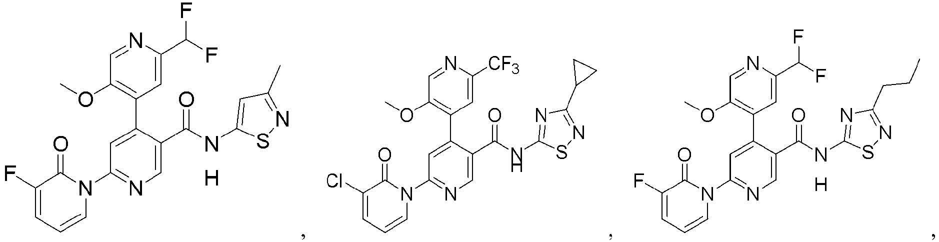 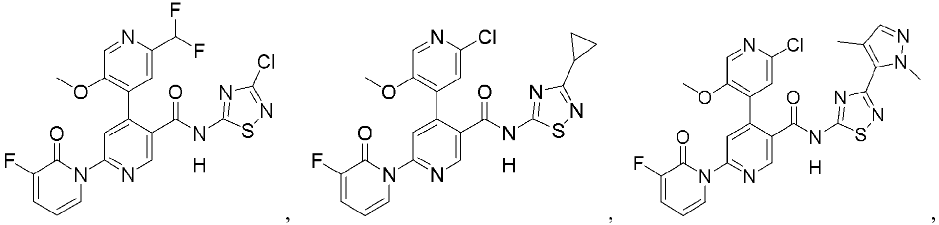 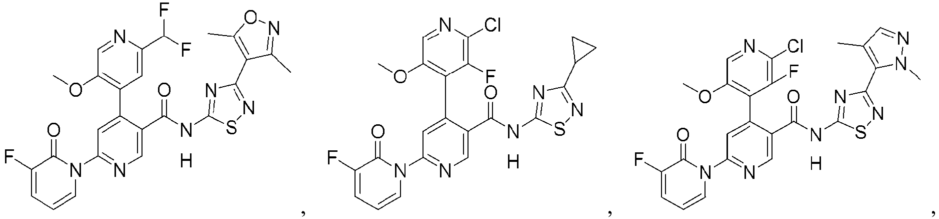 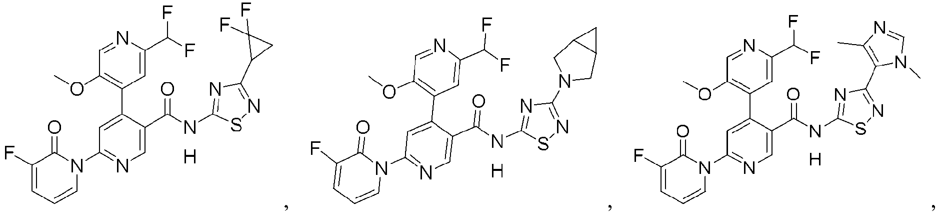 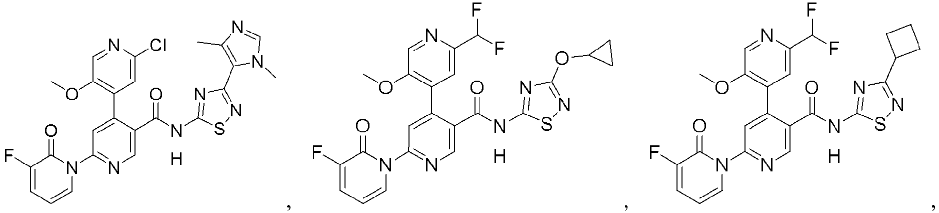 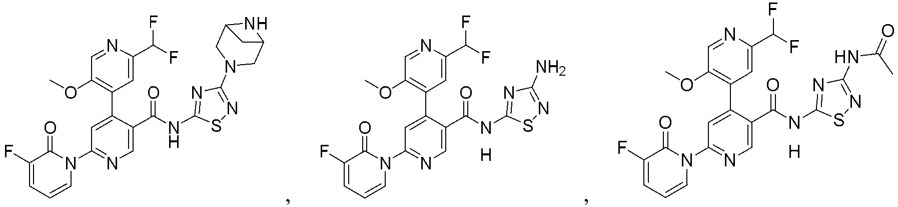 and

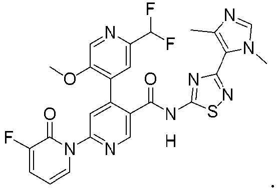

9. A pharmaceutical composition, comprising:
(1) a substance A, which is the compound of Formula I, the pharmaceutically acceptable salt thereof or the isotopic compound thereof according to any one of claims 1 to 8; and
(2) a pharmaceutical excipient.

10. Use of a substance A or the pharmaceutical composition according to claim 9 in preparation of a polymerase theta inhibitor, wherein said substance A is the compound of formula I, the pharmaceutically acceptable salt thereof or the isotopic compound thereof according to any one of claims 1 to 8.

11. Use of a substance A or the pharmaceutical composition according to claim 9 in preparation of a medicament, wherein said substance A is the compound of Formula I, the pharmaceutically acceptable salt thereof or the isotopic compound thereof according to any one of claims 1 to 8; and said medicament is used for treatment and/or prevention of lung cancer, breast cancer, HR-deficient ovarian cancer, gastric cancer, prostate cancer, pancreatic cancer or colon cancer.

12. Use of a substance A or the pharmaceutical composition according to claim 9 in preparation of a medicament for treatment and/or prevention of polymerase theta-related diseases, wherein said substance A is the compound of Formula I, the pharmaceutically acceptable salt thereof or the isotopic compound thereof according to any one of claims 1 to 8; and said polymerase theta-related diseases comprise lung cancer, breast cancer, HR-deficient ovarian cancer, gastric cancer, prostate cancer, pancreatic cancer or colon cancer.

13. A compound of Formula II, or a salt thereof: 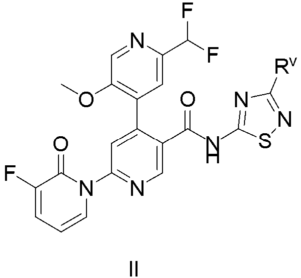 wherein R^{v} is halogen;
preferably, said compound of Formula II is

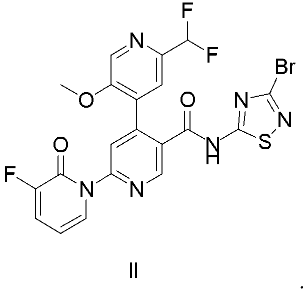

14. A method for treating a disease or a polymerase theta-related disease, comprising a step of: administering a substance A or the pharmaceutical composition according to claim 9 to a subject, wherein said substance A is the compound of Formula I, the pharmaceutically acceptable salt thereof or the isotopic compound thereof according to any one of claims 1 to 8; and said disease and said polymerase theta-related disease are independently lung cancer, breast cancer, HR-deficient ovarian cancer, gastric cancer, prostate cancer, pancreatic cancer or colon cancer.
